# EUROPEAN PATENT SPECIFICATION

(11) **EP 2 797 940 B1**
(45) Date of publication and mention of the grant of the patent: **24.02.2016**
(21) Application number: 12794880.0
(22) Date of filing: 03.12.2012
(51) Int. Cl.: C07F 15/00, H01L 51/00, A61K 33/24, A61P 17/00, A61Q 19/00

(54) **METAL COMPLEXES COMPRISING 1,2,3-TRIAZOLES**
METALLKOMPLEXE MIT 1,2,3-TRIAZOLEN
COMPLEXES MÉTALLIQUES COMPRENANT DES 1,2,3-TRIAZOLES

(30) Priority: 27.12.2011 EP 11010217
(43) Date of publication of application: 05.11.2014
(73) Proprietor: Merck Patent GmbH, 64293 Darmstadt (DE)
(72) Inventor: ANÉMIAN, Rémi Manouk, Seoul 657-169 (KR); EBERLE, Thomas, 76829 Landau (DE); STOESSEL, Philipp, 60487 Frankfurt am Main (DE); KOENIG, Burkhard, 93138 Lappersdorf (DE)
(86) International application number: PCT/EP2012/004979
(87) International publication number: WO 2013/097920

(56) References cited:
- WO-A2-2011/059791

## Description

The structure of organic electroluminescent devices (OLEDs) in which organic semiconductors are-employed as functional materials is described, for example, in US 4539507, US 5151629, EP 0676461 and WO 98/27136. The emitting materials being employed here are increasingly organometallic complexes which exhibit phosphorescence instead of fluorescence (M. A. Baldo et al., Appl. Phys. Lett. 1999, 75, 4-6). For quantum-mechanical reasons, an up to four-fold increase in energy and power efficiency is possible using organometallic compounds as phosphorescence emitters. In general, however, there is still a need for improvement in OLEDs which exhibit triplet emission, in particular with respect to efficiency, operating voltage and lifetime. This applies, in particular, to OLEDs which emit in the relatively short-wave range, i.e. green and blue. Furthermore, many phosphorescent emitters do not have adequate solubility for processing from solution, so there is also a further need for improvement here.

In accordance with the prior art, the triplet emitters employed in phosphorescent OLEDs are, in particular, iridium and platinum complexes, which are usually employed as cyclometallated complexes. The ligands here are frequently derivatives of phenylpyridine. However, the solubility of such complexes is frequently low, which makes processing from solution more difficult or prevents it completely.

The prior art discloses iridium complexes which are substituted by an optionally substituted aryl or heteroaryl group on the phenyl ring of the phenylpyridine ligand in the para-position to the coordination to the metal (WO 2004/026886 A2). This gives rise to improved solubility of the complexes. However, there is still a further need for improvement here with respect to the solubility and the efficiency and lifetime of the complexes.

WO 2011/059791 A2 discloses phosphorescent metal complexes comprising iridium. The ligand of the metal complex may be substituted with 1,2,3-triazole. The triazole is bound to the ligand via one of its carbon atoms.

Surprisingly, it has been found that certain metal chelate complexes described in greater detail below have improved solubility and furthermore result in improvements in the organic electroluminescent device, in particular with respect to the efficiency and lifetime. The present invention therefore relates to these metal complexes and to organic electroluminescent devices which comprise these complexes.

The invention relates to a compound of the Formula (1),

M(L)ₙ(L')ₘ Formula (1)

where the compound of the general Formula (1) contains a moiety M(L)ₙ of the Formula (2): 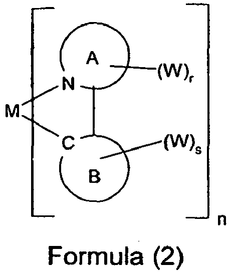 where M is bonded to any desired bidentate ligand L via a nitrogen atom N and a carbon atom C, and
wherein A and B can be any with one or more R¹ substituted or unsubstituted aromatic or heterearomatic ring or any with one or more R¹ substituted or unsubstituted aromatic or heteroaromatic polycyclic ring system;
wherein the symbols and indices are defined as follows:
- M: is a metal selected from the group consisting of iridium, rhodium, platinum and palladium, preferably iridium, platinum and palladium, particularly preferably iridium and platinum and very particularly preferably iridium;
- L': is, identically or differently on each occurrence, any desired co-ligand;
- W: is equal to the Formula (3) 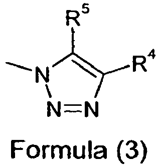
- r: is 0, 1, 2 or 3;
- s: is 0, 1, 2 or 3;
wherein the sum of r and s is at least 1, preferably 1, 2 or 3, particularly preferably 1 or 2 and very particularly preferably 1;
- n: is 1, 2 or 3 for M equal to iridium or rhodium and is 1 or 2 for M equal to platinum or palladium;
- m: is 0, 1, 2, 3 or 4;
- R¹,R⁴ and R⁵: are identically or differently from each other on each occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diaryl-amino group, diheteroarylamino group or arylheteroarylamino group having 10 to 40 aromatic ring atoms, which may be substituted by one or more radicals R², or a combination of two or more of these groups; two or more radicals R¹, R² or R⁵ here may also form a mono- or polycyclic, aliphatic, aromatic and/or benzo-fused ring system with one another;
- R²: is, identically or differently on each occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R³, where one or more non-adjacent CH₂ groups may be replaced by R³C=CR³, C=C, Si(R³)₂, Ge(R³)₂, Sn(R³)₂, C=O, C=S, C=Se, C=NR³, P(=O)(R³),SO, SO₂, NR³, O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 60 aromatic ring atoms, which may be substituted by one or more radicals R³, or a diarylamino group, diheteroarylamino group or arylheteroarylamino group having 10 to 40 aromatic ring atoms, which may be substituted by one or more radicals R³, or a combination of two or more of these groups; two or more adjacent radicals R² here may form a mono- or polycyclic, aliphatic or aromatic ring system with one another;
- R³: is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents R³ here may also form a mono- or polycyclic, aliphatic or aromatic ring system with one another;
the indices n and m here are selected so that the coordination number on the metal corresponds to 6 for M equal to iridium or rhodium and corresponds to 4 for M equal to platinum or palladium;
a plurality of ligands L here may also be linked to one another or L may be linked to L' via any desired bridge Z and thus form a tridentate, tetradentate, pentadentate or hexadentate ligand system.

An aryl group in the sense of this invention contains 6 to 40 C atoms; a heteroaryl group in the sense of this invention contains 2 to 40 C atoms and at least one heteroatom, with the proviso that the sum of C atoms and heteroatoms is at least 5. The heteroatoms are preferably selected from N, O and/or S. An aryl group or heteroaryl group here is taken to mean either a simple aromatic ring, i.e. benzene, or a simple heteroaromatic ring, for example pyridine, pyrimidine, thiophene, etc., or a condensed aryl or heteroaryl group, for example naphthalene, anthracene, phenanthrene, quinoline, isoquinoline, etc.

An aromatic ring system in the sense of this invention contains 6 to 60 C atoms in the ring system. A heteroaromatic ring system in the sense of this invention contains 2 to 60 C atoms and at least one heteroatom in the ring system, with the proviso that the sum of C atoms and heteroatoms is at least 5. The heteroatoms are preferably selected from N, O and/or S. For the purposes of this invention, an aromatic or heteroaromatic ring system is intended to be taken to mean a system which does not necessarily contain only aryl or heteroaryl groups, but instead in which, in addition, a plurality of aryl or heteroaryl groups may be interrupted by a non-aromatic unit (preferably less than 10% of the atoms other than H), such as, for example, an sp³-hybridised C, N or O atom or a carbonyl group. Thus, for example, systems such as 9,9'-spirobifluorene, 9,9-diarylfluorene, triarylamine, diaryl ether, stilbene, etc., are also intended to be taken to mean aromatic ring systems for the purposes of this invention, and likewise systems in which two or more aryl groups are interrupted, for example, by a linear or cyclic alkyl group or by a silyl group.

A cyclic alkyl, alkoxy or thioalkoxy group in the sense of this invention is taken to mean a monocyclic, bicyclic or polycyclic group.

For the purposes of the present invention, a C₁₋ to C₄₀-alkyl group, in which, in addition, individual H atoms or CH₂ groups may be substituted by the above-mentioned groups, is taken to mean, for example, the radicals methyl, ethyl, n-propyl, i-propyl, n-butyl, i-butyl, s-butyl, t-butyl, 2-methylbutyl, n-pentyl, s-pentyl, tert-pentyl, 2-pentyl, cyclopentyl, n-hexyl, s-hexyl, tert-hexyl, 2-hexyl, 3-hexyl, cyclohexyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2.2.2]octyl, 2-bicyclo[2.2.2]octyl, 2-(2,6-dimethyl)octyl, 3-(3,7-dimethyl)octyl, trifluoromethyl, pentafluoroethyl or 2,2,2-trifluoroethyl. An alkenyl group is taken to mean, for example, ethenyl, propenyl, butenyl, pentenyl, cyclopentenyl, hexenyl, cyclohexenyl, heptenyl, cycloheptenyl, octenyl, cyclooctenyl or cyclooctadienyl. An alkynyl group is taken to mean, for example, ethynyl, propynyl, butynyl, pentynyl, hexynyl, heptynyl or octynyl. A C₁- to C₄₀-alkoxy group is taken to mean, for example, methoxy, trifluoromethoxy, ethoxy, n-propoxy, i-propoxy, n-butoxy, i-butoxy, s-butoxy, t-butoxy or 2-methylbutoxy. An aromatic or heteroaromatic ring system having 5 - 60 aromatic ring atoms, which may also in each case be substituted by the radicals R mentioned above and which may be linked to the aromatic or heteroaromatic ring system via any desired positions, is taken to mean, for example, groups derived from benzene, naphthalene, anthracene, benzanthracene, phenanthrene, benzophenanthrene, pyrene, chrysene, perylene, fluoranthene, benzofluoranthene, naphthacene, pentacene, benzopyrene, biphenyl, biphenylene, terphenyl, terphenylene, fluorene, spirobifluorene, dihydrophenanthrene, dihydropyrene, tetrahydropyrene, cis- or trans-indenofluorene, cis- or trans-monobenzo-indenofluorene, cis- or trans-dibenzoindenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

The compounds of the Formula (1) may be electrically charged or uncharged. In a preferred embodiment, the compounds of the Formula (1) are electrically neutral. This is achieved in a simple manner in that the charges of the ligands L and L' are selected so that they compensate for the charge of the complexed metal atom M.

Preference is furthermore given to compounds of the Formula (1), characterised in that the sum of the valence electrons around the metal atom is 16 for platinum and palladium and 18 for iridium and rhodium. This preference is due to the particular stability of these metal complexes.

If M stands for platinum or palladium, the index n stands for 1 or 2. If the index n = 1, one bidentate or two monodentate ligands L', preferably one bidentate ligand L', are also coordinated to the metal M. Correspondingly, the index m = 1 for one bidentate ligand L' and the index m = 2 for two monodentate ligands L'. If the index n = 2, the index m = 0.

If M stands for iridium or rhodium, the index n stands for 1, 2 or 3, preferably for 2 or 3 and particularly preferably for 3. If the index n = 1, four monodentate or two bidentate or one bidentate and two monodentate or one tridentate and one monodentate or one tetradentate ligand L', preferably two bidentate ligands L', are also coordinated to the metal. Correspondingly, the index m is, depending on the ligand L', equal to 1, 2, 3 or 4. If the index n = 2, one bidentate or two monodentate ligands L', preferably one bidentate ligand L', are also coordinated to the metal. Correspondingly, the index m is, depending on the ligand L', equal to 1 or 2. If the index n = 3, the index m = 0.

In a preferred embodiment of the present invention, the compound of Formula (1) contains a moiety M(L)ₙ of Formula (4)

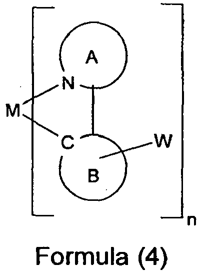

In a preferred embodiment of the present invention, the compound of Formula (1) contains a moiety M(L)ₙ of Formula (5a) or (5b), preferably (5b). 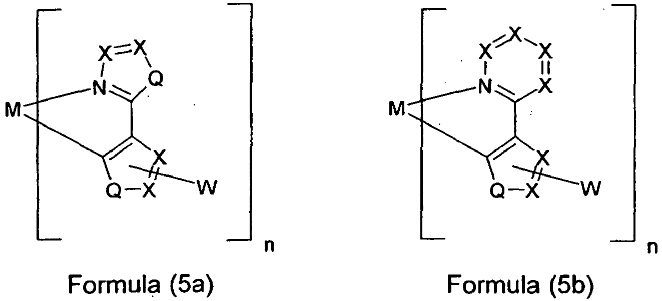 wherein the symbols and indices are defined as above and wherein
- X: is, identically or differently on each occurrence, CR¹ or N;
- Q: is, identically or differently on each occurrence, R¹C=CR¹, R¹C=N, O, S, Se or NR¹, preferably R¹C=CR¹, S or NR¹; particularly preferably R¹C=CR¹ or S and very particularly preferably R¹C=CR¹;
and wherein R¹ is defined as above.

In a further preferred embodiment of the present invention, the compound of Formula (1) contains a moiety M(L)ₙ of Formula (6)

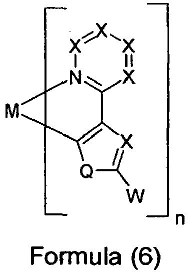

In yet another preferred embodiment of the present invention, the compound of Formula (1) contains a moiety M(L)ₙ of Formula (7)

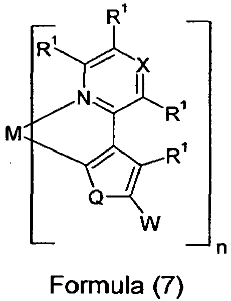

Preference is given to a compound of Formula (1) which contains a moiety M(L)ₙ of Formula (8) 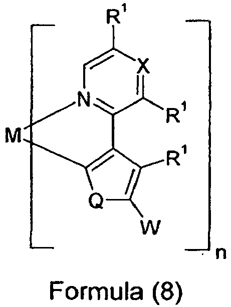 wherein Q is preferably R¹C=CR¹, S or NR¹; particularly preferably R¹C=CR¹ or S and wherein R¹ is defined as above and wherein M is preferably iridium, platinum and palladium, particularly preferably iridium and platinum and very particularly preferably iridium.

Further preference is given to a compound of Formula (1) which contains a moiety M(L)ₙ one of the following Formulae (9) to (10). 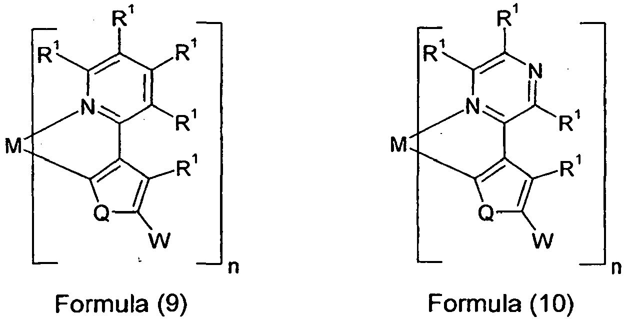 wherein Q is preferably R¹C=CR¹, S or NR¹; particularly preferably R¹C=CR¹ or S and very particularly preferably R¹C=CR¹ and wherein R¹ is defined as above and wherein M is preferably iridium, platinum and palladium, particularly preferably iridium and platinum and very particularly preferably iridium. One particular preferred embodiment according to the present invention is a compound of Formula (1) wherein the moiety of M(L)ₙ is selected from a compound of Formula (9).

The present invention also relates to a compound of Formula (1) which contains a moiety M(L)ₙ one of the following Formulae (11) to (19), preferably Formulae (11) to (14). 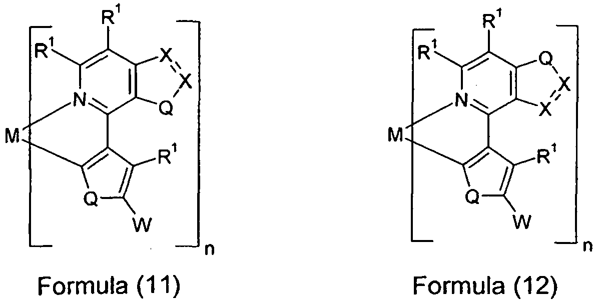 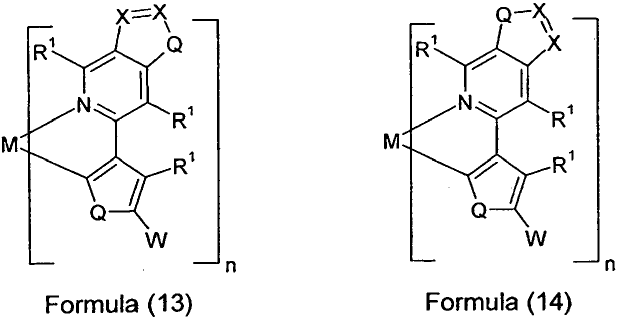 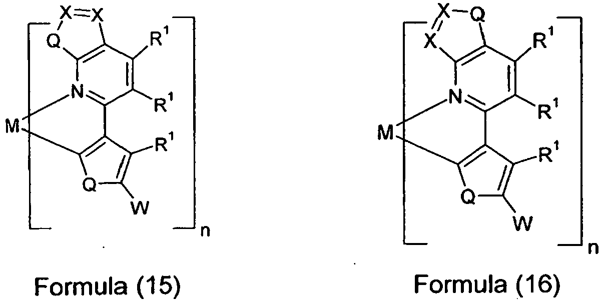 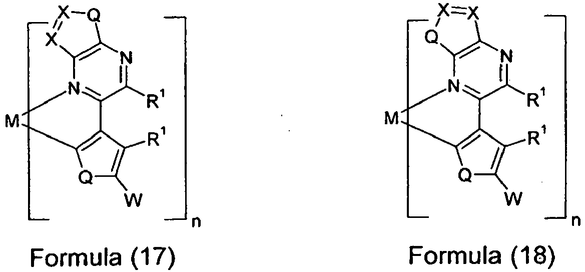 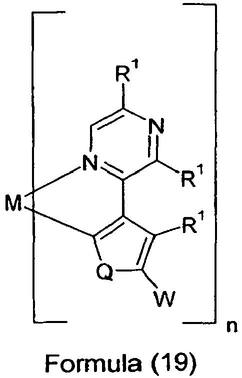 wherein X is defined as above and wherein Q is preferably R¹C=CR¹, S or NR¹; particularly preferably R¹C=CR¹ or S and wherein R¹ is defined as above and wherein M is preferably iridium, platinum and palladium, particularly preferably iridium and platinum and very particularly preferably iridium.

Preferably the present invention relates to a compound of Formula (1) which contains a moiety M(L)ₙ one of the following Formulae (11) to (19), preferably Formulae (11) to (14) wherein X is CR¹ and wherein Q is preferably R¹C=CR¹, S or NR¹; particularly preferably R¹C=CR¹ or S and very particularly preferably R¹C=CR¹ and wherein R¹ is defined as above and wherein M is preferably iridium, platinum and palladium, particularly preferably iridium and platinum and very particularly preferably iridium.

Particularly preferably the present invention relates to a compound of Formula (1) which contains a moiety M(L)ₙ one of the following Formulae (20) to (26), preferably Formulae (20) to (23) and (26), particularly preferably Formulae (29 to (23) 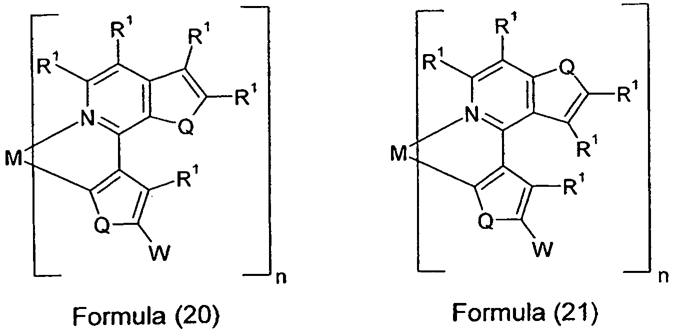 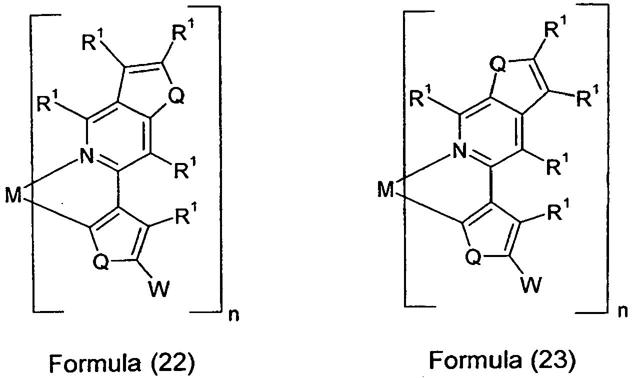 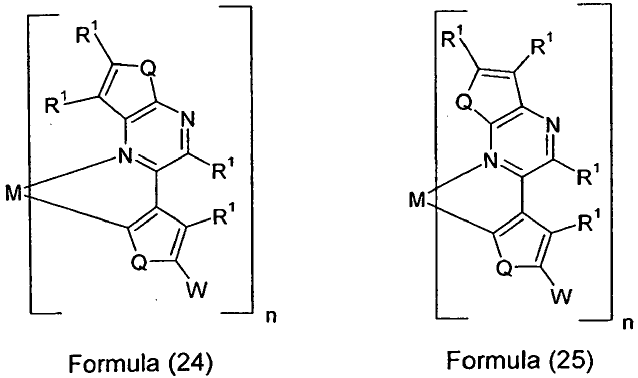 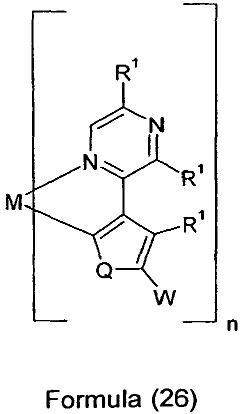 wherein Q is preferably R¹C=CR¹, S or NR¹; particularly preferably R¹C=CR¹ or S and very particularly preferably R¹C=CR¹ and wherein R¹ is defined as above and wherein M is preferably iridium, platinum and palladium, particularly preferably iridium and platinum and very particularly preferably iridium.

R¹ in moieties of Formulae (1) to (26) is, independent of each other, preferably selected from H, D, F, Cl, Br, I, N(R²)₂, CN, NO₂, P(=O)(R²)₂, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by R²C=CR², C=C, C=O, C=S, C=Se, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of two or more of these groups; two or more radicals R¹ here may also form a mono- or polycyclic, aliphatic, aromatic and/or benzo-fused ring system with one another, wherein R² is defined as above.

Very particularly preferably the present invention relates to a compound of Formula (1) which contains a moiety M(L)ₙ one of the following Formulae (27) to (31), preferably Formula (27), (29) and (30). 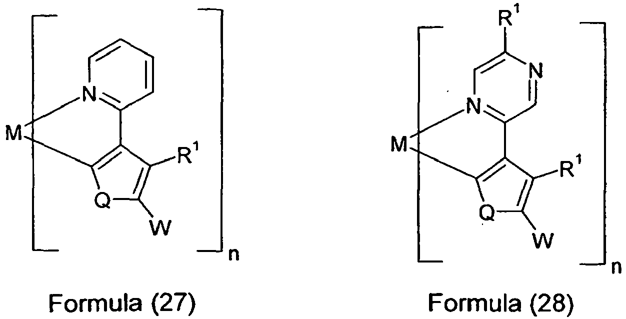 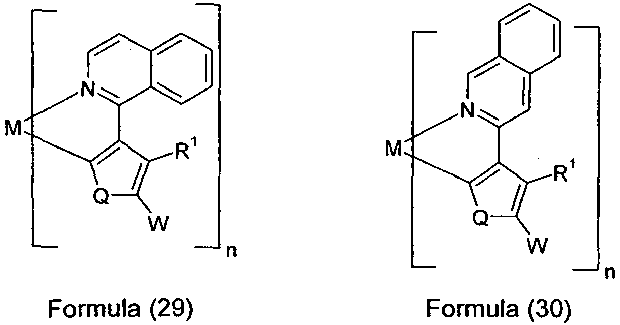 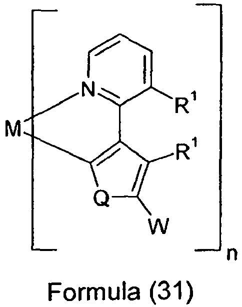 wherein Q is preferably R¹C=CR¹, S or NR¹; particularly preferably R¹C=CR¹ or S and very particularly preferably R¹C=CR¹ and wherein R¹ is defined as above and wherein M is preferably iridium, platinum and palladium, particularly preferably iridium and platinum and very particularly preferably iridium.

Preferably R⁵ in Formula (3) is H, D, F, Cl, Br, I, N(R²)₂, CN, NO₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R² or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of two or more of these groups; two or more radicals R⁵ here may also form a mono- or polycyclic, aliphatic, aromatic and/or benzo-fused ring system with one another, wherein R² is defined as above.

Particularly preferably R⁵ in Formula (3) is H.

Preferably R⁴ in Formulae (1) to (31) are, identically or differently from each other on each occurrence, H, D, F, Cl, Br, I, N(R²)₂, CN, NO₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R² or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of two or more of these groups; two or more radicals R⁴ here may also form a mono- or polycyclic, aliphatic, aromatic and/or benzo-fused ring system with one another, wherein R² is defined as above.

Particularly preferably R⁴ in Formulae (1) to (31) is, identically or differently from each other on each occurrence, H, a straight-chain alkyl or alkoxy-group having 1 to 40 C-atoms or a straight-chain alkenyl or aklynyl-group having 2 to 40 C-atoms or a branched or cyclic alkyl-, alkenyl-, alkynyl- or alkoxy-group having 3 to 40 C-atoms, -NH₂, -SR³, -OR³ or an aromatic or heteroaromatic ring system having 5 to 60 aromatic ring atoms, which may in each case be substituted by one or more radicals R².

Very particularly preferably R⁴ in Formulae (1) to (31) is, identically or differently from each other on each occurrence, H, a straight-chain alkyl or alkoxy-group having 1 to 40 C-atoms or a straight-chain alkenyl or aklynyl-group having 2 to 40 C-atoms or a branched or cyclic alkyl-, alkenyl-, alkynyl- or alkoxy-group having 3 to 40 C-atoms, -NH₂, -SR³, -OR³ or group selected from the following Formulae (32) to (46), wherein Y is, identically or differently from each other on each occurrence, CR², N, P, or PR²2, preferably CR² or N, wherein R² is defined as above.

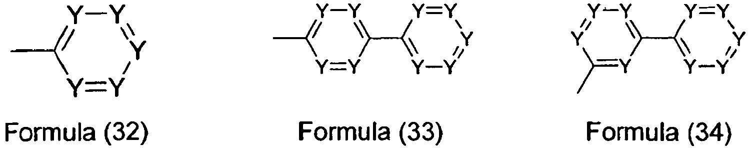

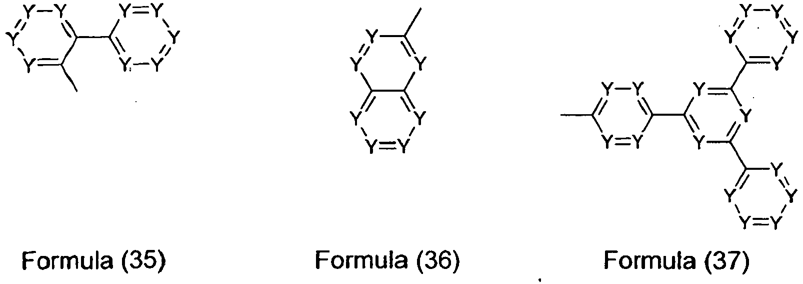

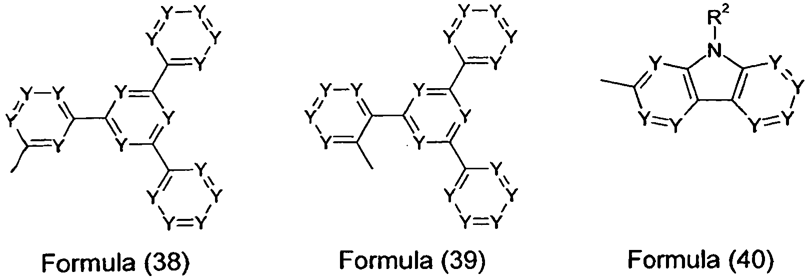

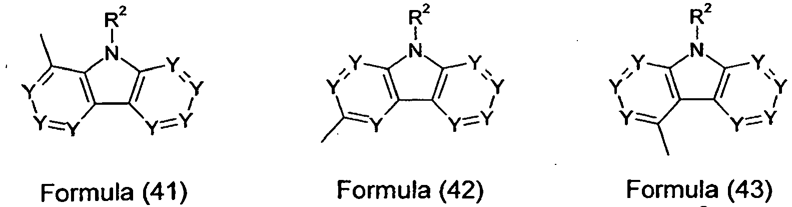

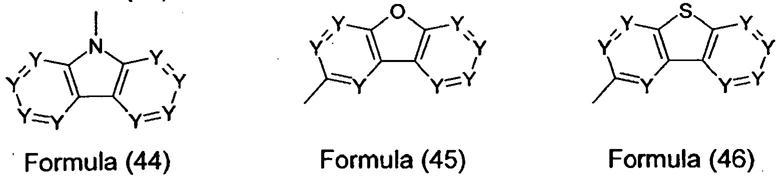

Even more preferably R⁴ in Formulae (1) to (31) is selected from Formulae (47) to (220) wherein the rest can be, identically or differently from each other on each occurrence, substituted with one or more -CN or R³, wherein R³ is defined as above.

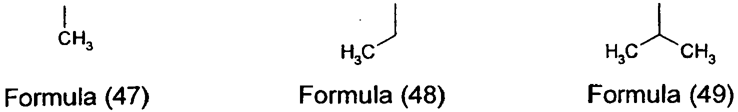

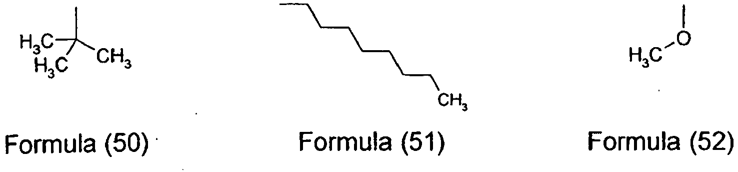

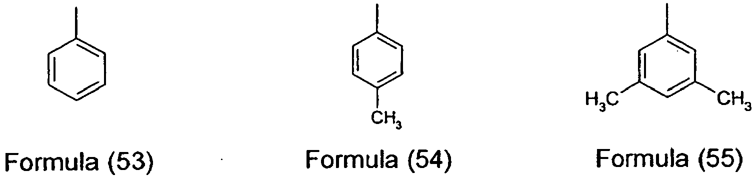

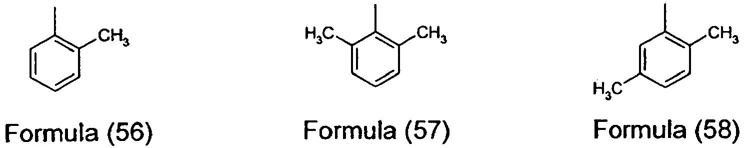

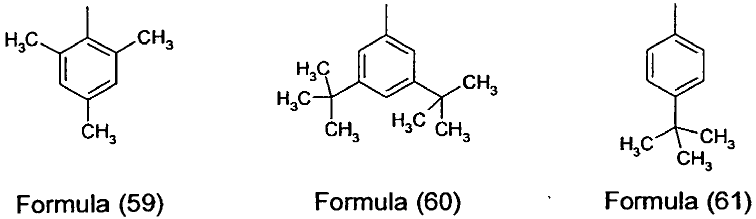

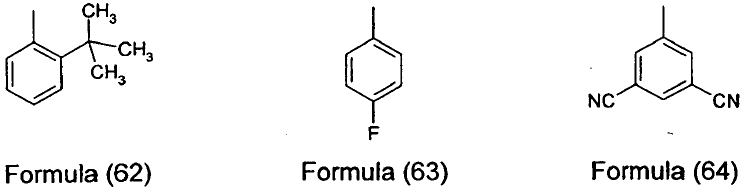

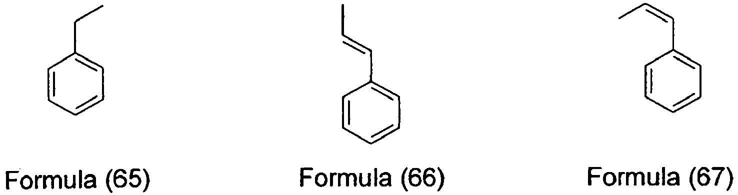

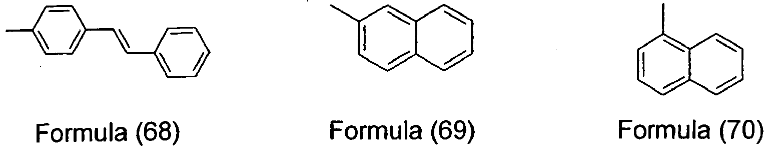

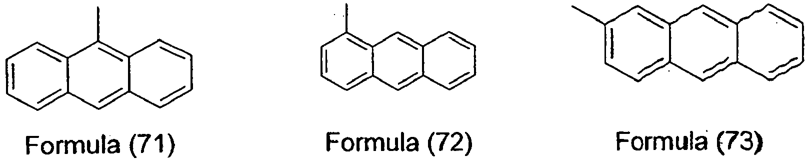

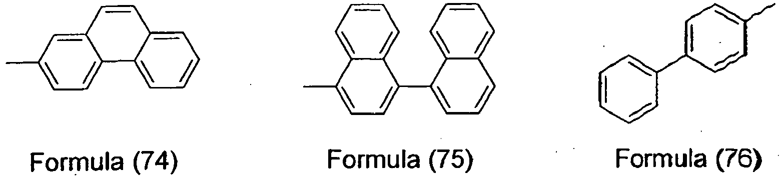

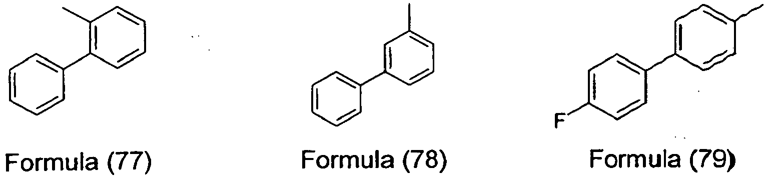

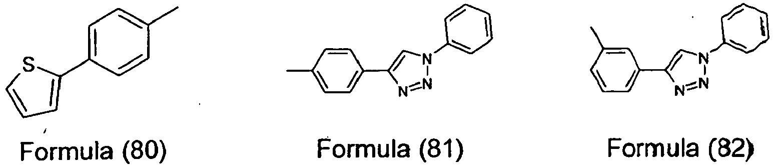

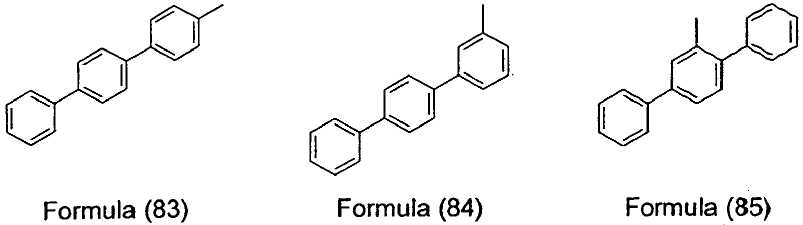

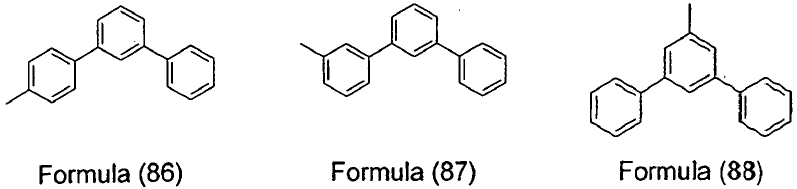

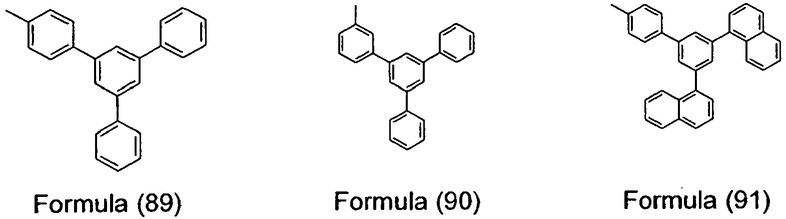

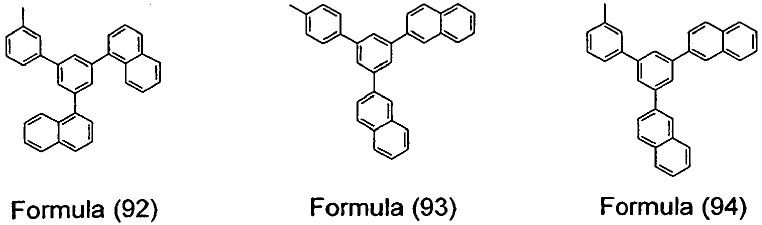

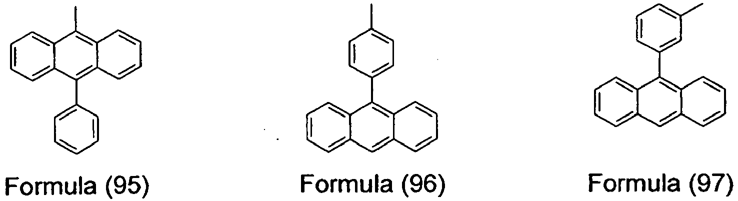

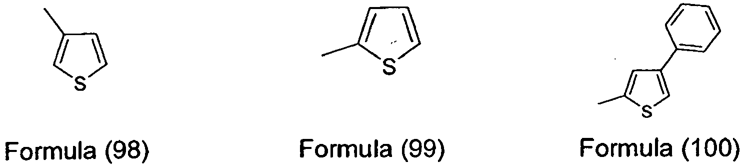

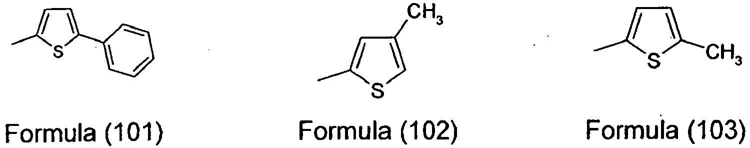

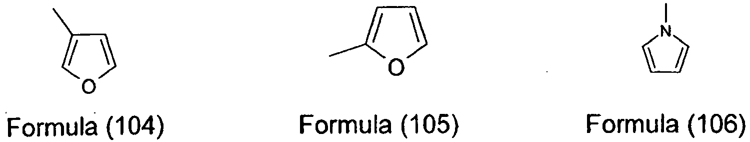

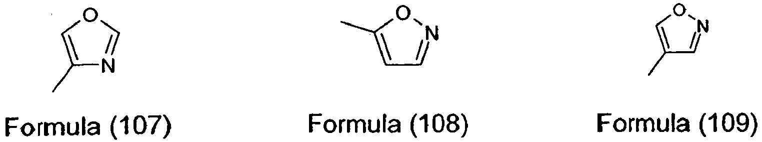

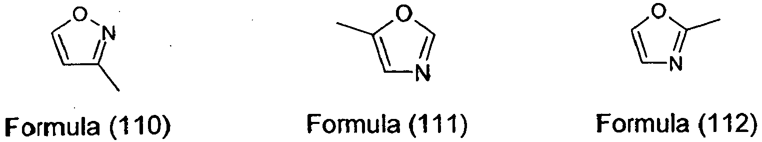

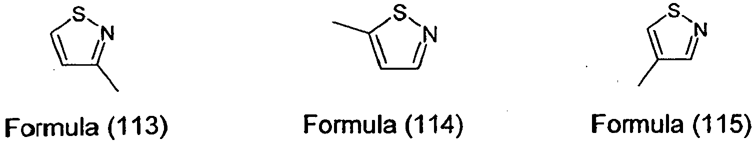

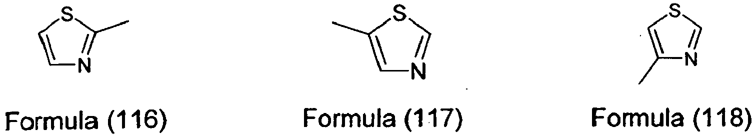

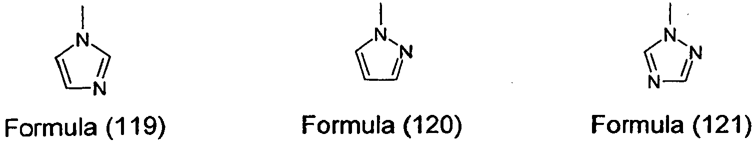

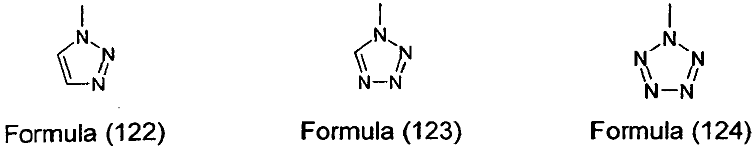

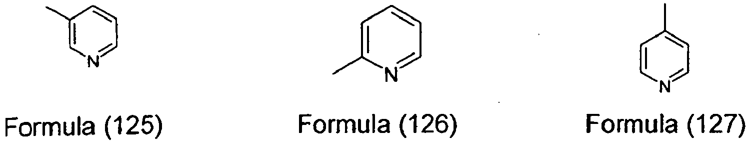

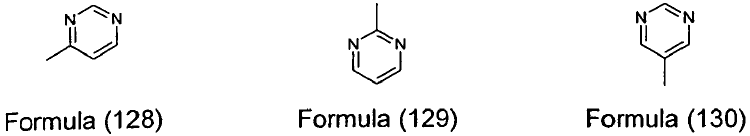

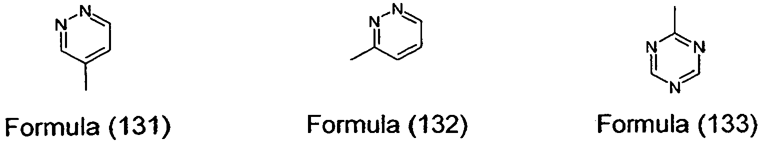

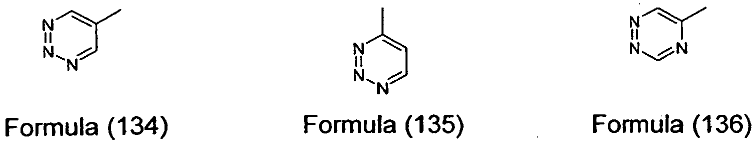

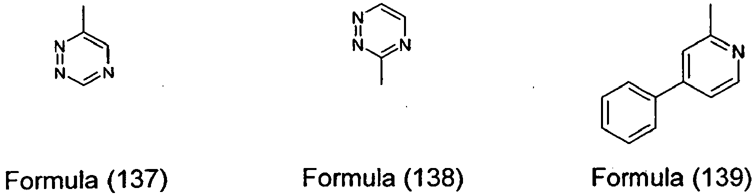

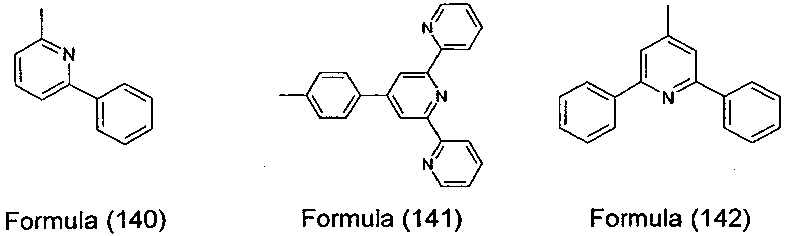

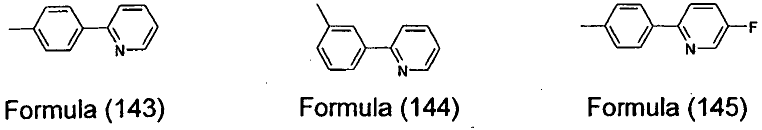

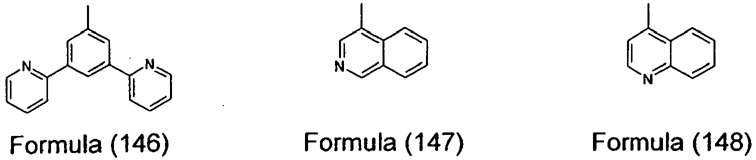

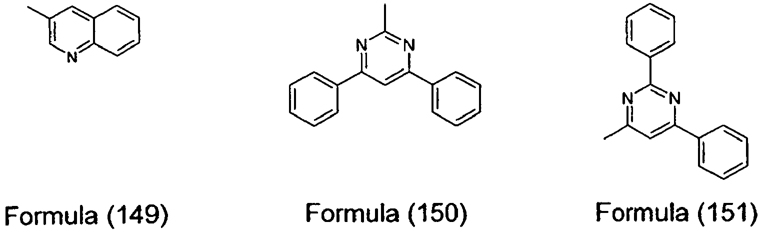

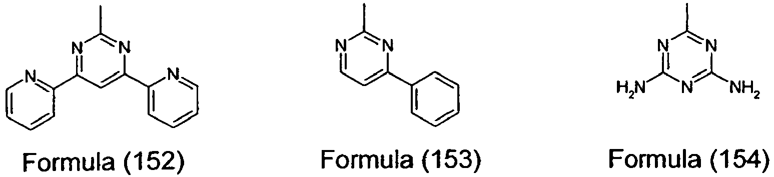

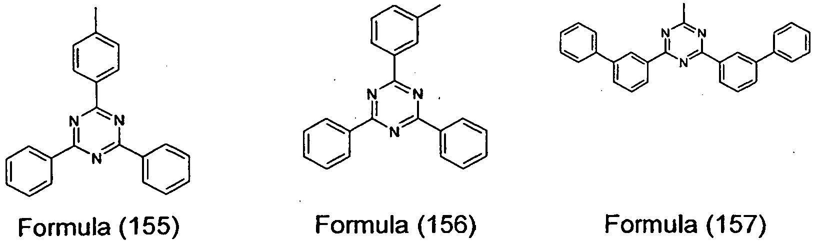

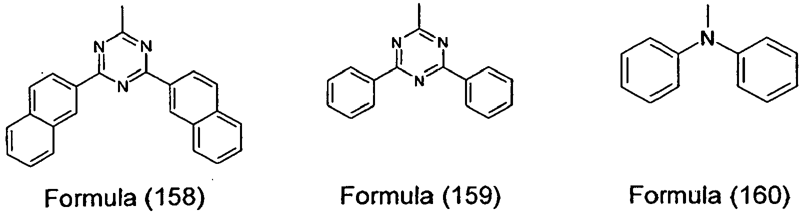

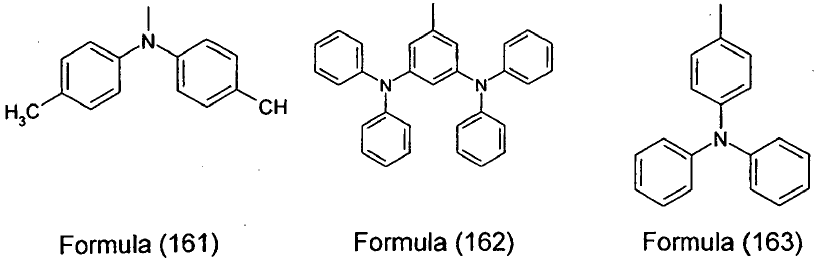

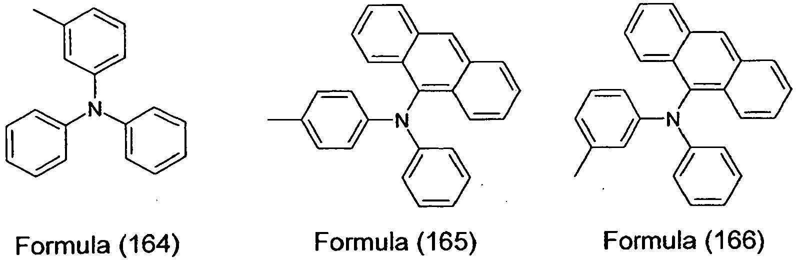

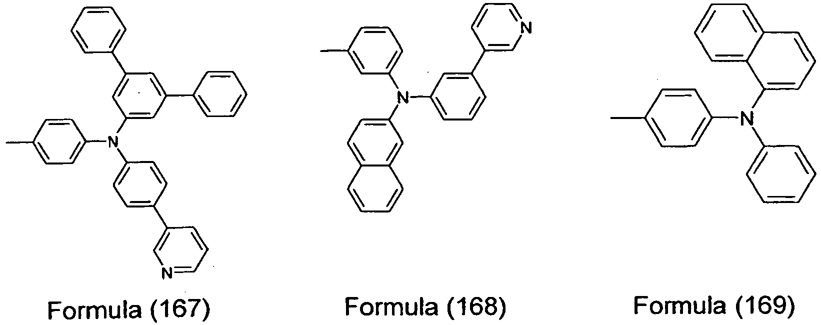

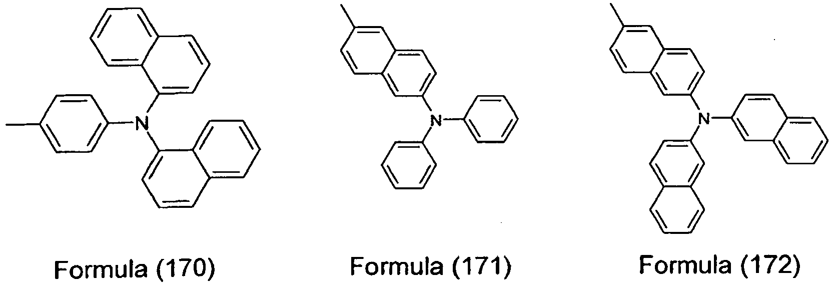

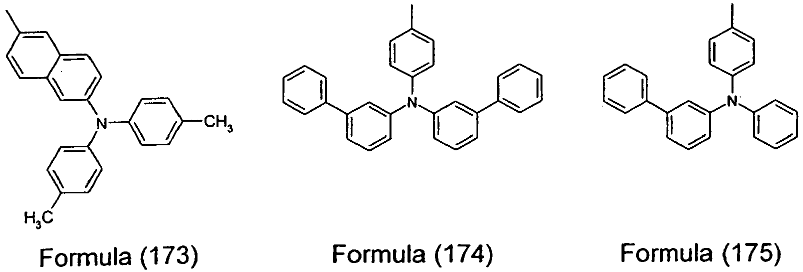

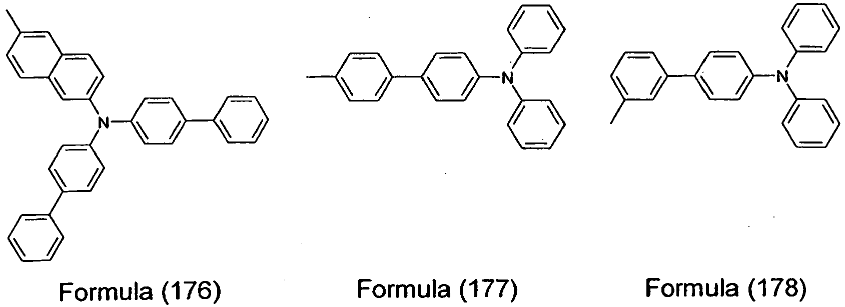

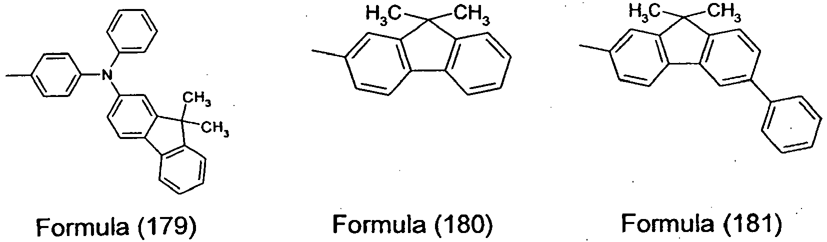

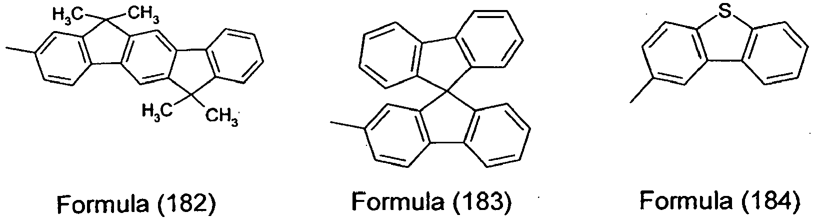

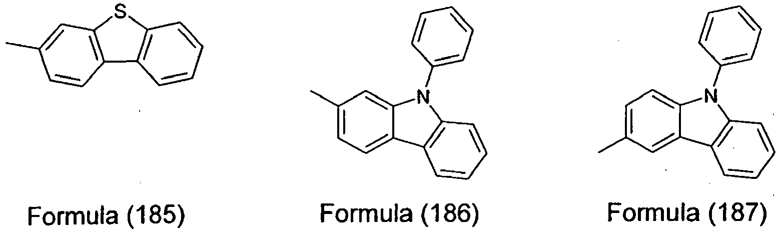

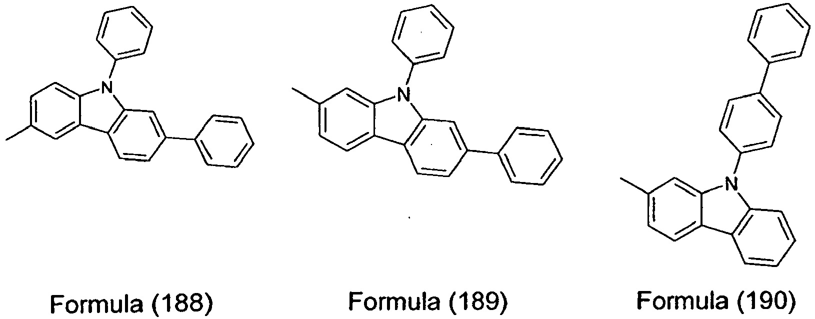

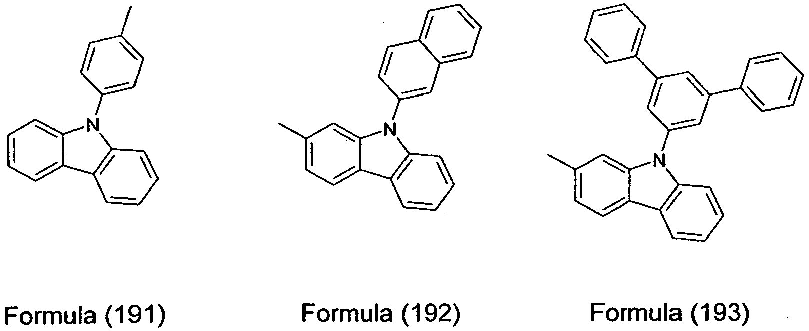

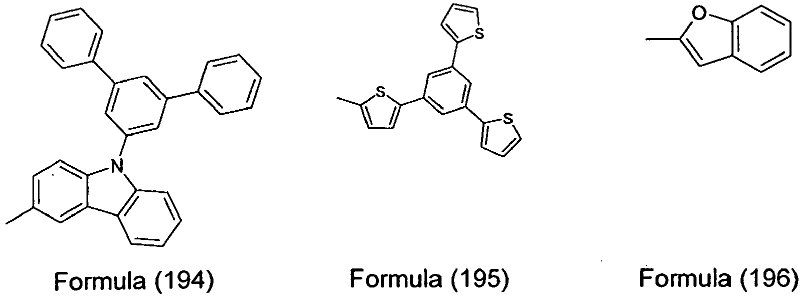

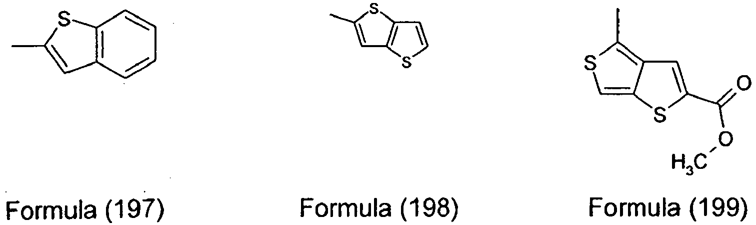

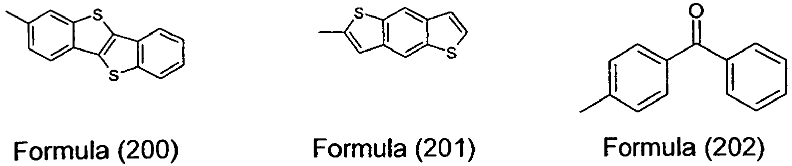

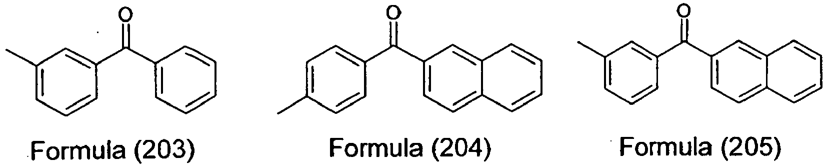

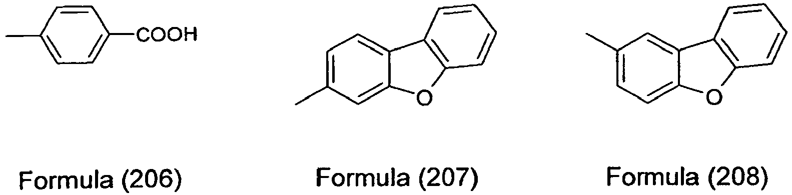

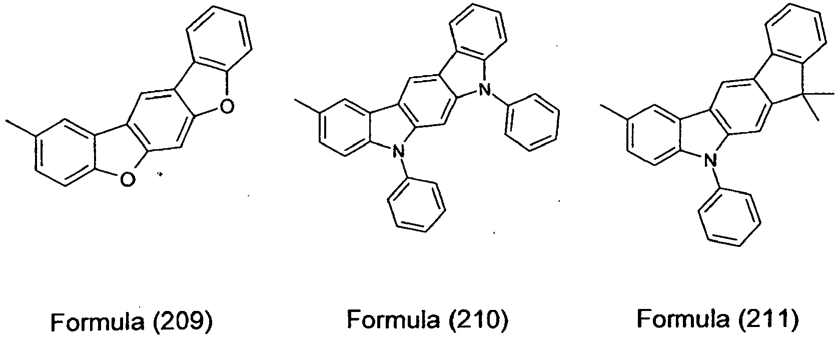

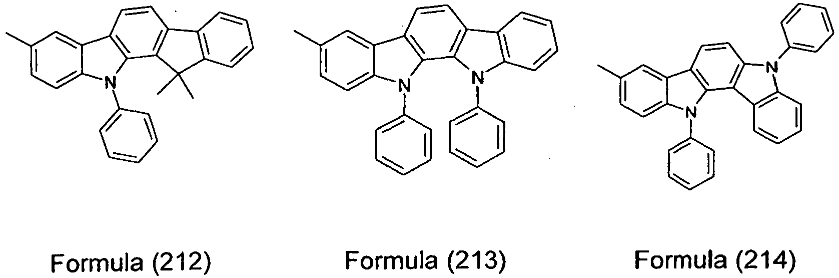

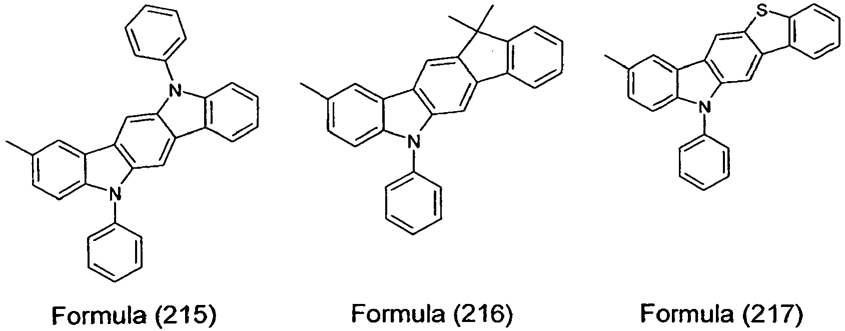

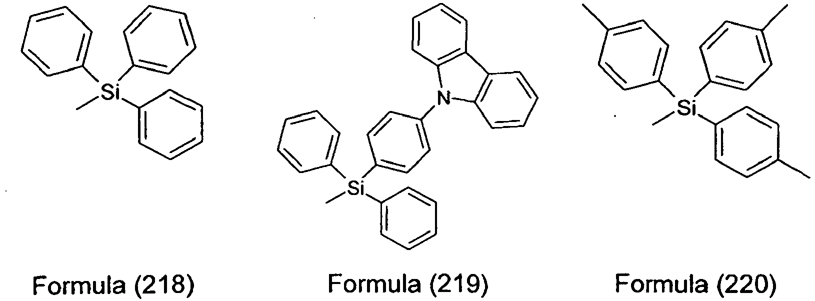

A bridging unit Z which links the ligand L to one or more further ligands L or L' may also be present on one of the radicals R¹. In a preferred embodiment of the invention, a bridging unit Z is present instead of one of the radicals R¹, meaning that the ligands have a tridentate or polydentate or polypodal character. Two bridging units Z of this type may also be present. This results in the formation of macrocyclic ligands or in the formation of cryptates.

Preferred structures having polydentate ligands are the metal complexes of the following formulae (221) to (222): 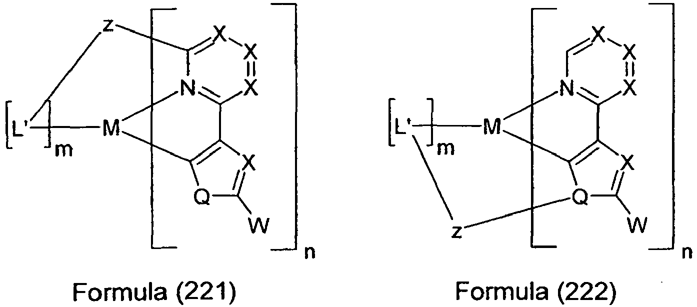 where Q is, identically or differently on each occurrence, R¹C=CR¹, R¹C=N, or NR¹, preferably R¹C=CR¹ or NR¹; particularly preferably R¹C=CR¹ and where Z is bonded to Q by replacing R¹ in Q and where the other symbols used have the meanings mentioned above, and Z preferably represents a bridging unit containing 1 to 80 atoms from the third, fourth, fifth and/or sixth main group (IUPAC group 13, 14, 15 or 16) or a 3- to 6-membered homo- or heterocycle which covalently bonds the part-ligands L to one another or L to L'. The bridging unit Z here may also have an asymmetric structure, i.e. the linking of Z to L or L' need not be identical.

The bridging unit Z may be neutral, singly, doubly or triply negatively charged or singly, doubly or triply positively charged. Z is preferably neutral or singly negatively charged or singly positively charged. The charge of Z here is preferably selected so that overall a neutral complex arises.

If Z is a trivalent group, i.e. bridges three ligands L to one another or two ligands L to L' or one ligand L to two ligands L', Z is preferably selected, identically or differently on each occurrence, from the group consisting of B, B(R²)⁻, B(C(R²)₂)₃, (R²)B(C(R²)₂)₃⁻, B(O)₃, (R²)B(O)₃⁻, B(C(R²)₂C(R²)₂)₃, (R²)B(C(R²)₂C(R²)₂)₃-, B(C(R²)₂O)₃, (R²)B(C(R²)₂O)3⁻ B(OC(R²)₂)₃, (R²)B(OC(R²)₂)₃⁻, C(R²), CO⁻, CN(R²)₂, (R²)C(C(R²)₂)₃, (R²)C(O)₃, (R²)C(C(R²)₂C(R²)2)3, (R²)C(C(R²)₂O)_{3,} (R²)C(OC(R²)₂)₃, (R²)C(Si(R²)₂)₃, (R²)C(Si(R²)₂C(R²)₂)₃, (R²)C(C(R²)₂Si(R²)₂)₃, (R²)C(Si(R²)₂Si(R²)₂)₃, Si(R²), (R2)Si(C(R²)₂)₃, (R²)Si(O)₃, (R²)Si(C(R²)₂C(R²)₂)₃, (R²)Si(OC(R²)₂)₃, (R²)Si(C(R²)₂O)₃, (R²)Si(Si(R²)₂)₃, (R²)Si(Si(R²)₂C(R²)₂)₃, (R²)Si(C(R²)₂Si(R²)₂)₃, (R²)Si(Si(R²)₂Si(R²)₂)₃, N, NO, N(R²)⁺, N(C(R²)₂)₃, (R²)N(C(R²)₂)₃⁺, N(C=O)₃, N(C(R²)₂C(R²)₂)₃, (R²)N(C(R²)C(R²)₂+, P, P(R²)⁺, PO, PS, PSe, PTe, P(O)₃, PO(O)₃, P(OC(R²)₂)₃, PO(OC(R²)₂)₃, P(C(R²)₂)₃, P(R²)(C(R²)₂)₃⁺, PO(C(R²)₂)_{3,} P(C(R²)₂C(R²)₂)₃, P(R²) (C(R²)₂C(R²)₂)₃⁺, PO(C(R²)₂C(R²)₂)_{3,} S⁺, S(C(R²)₂)₃⁺ S(C(R²)₂C(R²)₂)₃⁺, or a unit of the Formulas (223) to (226), 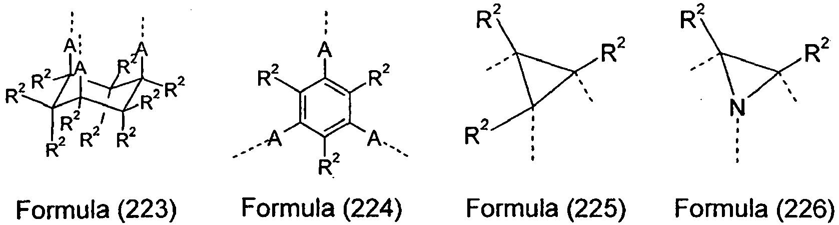 where the dashed bonds in each case indicate the bonding to the part-ligands L or L', and A is selected, identically or differently on each occurrence, from the group consisting of a single bond, O, S, S(=O), S(=O)₂, NR², PR², P(=O)R², P(=NR²), C(R²)₂, C(=O), C(=NR²), C(=C(R²)₂), Si(R²)₂ or BR². The other symbols used have the meanings mentioned above.

If Z is a divalent group, i.e. bridges two ligands L to one another or one ligand L to L', Z is preferably selected, identically or differently on each occurrence, from the group consisting of BR², B(R²)₂⁻, C(R²)₂, C(=O), Si(R²)₂, NR², PR², P(R²)₂⁺, P(=O)(R²), P(=S)(R²), AsR², As(=O)(R²), As(=S)(R²), O, S, Se, or a unit of the Formulae (227) to (235), 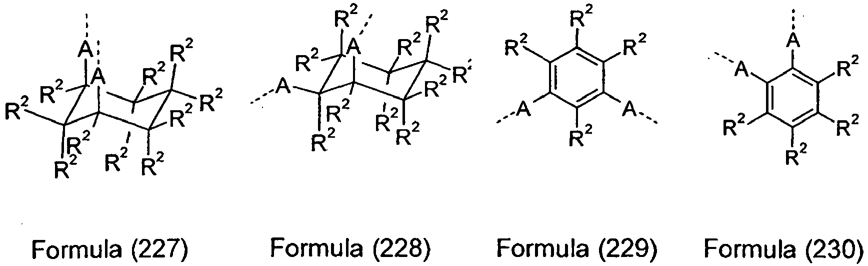 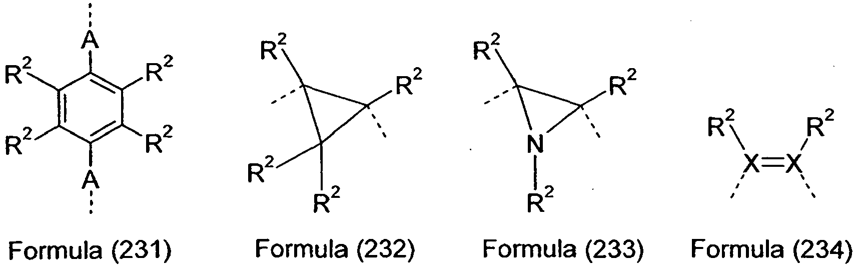 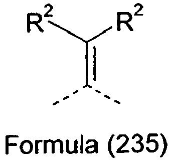 where the dashed bonds in each case indicate the bonding to the part-ligands L or L', and the further symbols used in each case have the meanings mentioned above.

Preferred ligands L' as occur in Formula (1) are described below. Correspondingly, the ligand groups L' can also be selected if they are bonded to L via a bridging unit Z.

The ligands L' are preferably neutral, monoanionic, dianionic or trianionic ligands, particularly preferably neutral or monoanionic ligands. They can be monodentate, bidentate, tridentate or tetradentate and are preferably bidentate, i.e. preferably have two coordination sites. As described above, the ligands L' may also be bonded to L via a bridging group Z.

Preferred neutral, monodentate ligands L' are selected from carbon monoxide, nitrogen monoxide, alkyl cyanides, such as, for example, acetonitrile, aryl cyanides, such as, for example, benzonitrile, alkyl isocyanides, such as, for example, methyl isonitrile, aryl isocyanides, such as, for example, benzoisonitrile, amines, such as, for example, trimethylamine, triethylamine, morpholine, phosphines, in particular halophosphines, trialkylphosphines, triarylphosphines or alkylarylphosphines, such as, for example, trifluorophosphine, trimethylphosphine, tricyclohexylphosphine, tri-*tert*-butylphosphine, triphenylphosphine, tris(pentafluorophenyl)phosphine, phosphites, such as, for example, trimethyl phosphite, triethyl phosphite, arsines, such as, for example, trifluoroarsine, trimethylarsine, tricyclohexylarsine, tri-*tert-*butylarsine, triphenylarsine, tris(pentafluorophenyl)arsine, stibines, such as, for example, trifluorostibine, trimethylstibine, tricyclohexylstibine, tri-*tert*-butylstibine, triphenylstibine, tris(pentafluorophenyl)stibine, nitrogen-containing heterocycles, such as, for example, pyridine, pyridazine, pyrazine, pyrimidine, triazine, and carbenes, in particular Arduengo carbenes.

Preferred monoanionic, monodentate ligands L' are selected from hydride, deuteride, the halides F⁻, Cl⁻, Br⁻ and I⁻, alkylacetylides, such as, for example, methyl-C≡C⁻, tert-butyl-C≡C⁻, arylacetylides, such as, for example, phenyl-C≡C⁻, cyanide, cyanate, isocyanate, thiocyanate, isothiocyanate, aliphatic or aromatic alcoholates, such as, for example, methanolate, ethanolate, propanolate, isopropanolate, *tert*-butylate, phenolate, aliphatic or aromatic thioalcoholates, such as, for example, methanethiolate, ethanethiolate, propanethiolate, isopropanethiolate, *tert*-butanethiolate, thiophenolate, amides, such as, for example, dimethylamide, diethylamide, diisopropylamide, morpholide, carboxylates, such as, for example, acetate, trifluoroacetate, propionate, benzoate, aryl groups, such as, for example, phenyl, naphthyl, and anionic, nitrogen-containing heterocycles, such as pyrrolide, imidazolide, pyrazolide. The alkyl groups in these groups are preferably C₁-C₂₀-alkyl groups, particularly preferably C₁-C₁₀-alkyl groups, very particularly preferably C₁-C₄-alkyl groups. An aryl group is also taken to mean heteroaryl groups. These groups are as defined above.

Preferred di- or trianionic ligands are O²⁻, S²⁻, carbides, which result in coordination in the form R-C≡M, and nitrenes, which result in coordination in the form R-N=M, where R generally stands for a substituent, or N³⁻.

Preferred neutral or mono- or dianionic, bidentate or polydentate ligands L' are selected from diamines, such as, for example, ethylenediamine, N,N,N',N'-tetramethylethylenediamine, propylenediamine, N,N,N',N'-tetramethylpropylenediamine, cis- or trans-diaminocyclohexane, cis- or trans-N,N,N',N'-tetramethyldiaminocyclohexane, imines, such as, for example, 2-[1-(phenylimino)ethyl]pyridine, 2-[1-(2-methylphenylimino)ethyl]pyridine, 2-[1-(2,6-diisopropylphenylimino)ethyl]pyridine, 2-[1-(methylimino)ethyl]-pyridine, 2-[1-(ethylimino)ethyl]pyridine, 2-[1-(isopropylimino)ethyl]pyridine, 2-[1-(*tert*-butylimino)ethyl]pyridine, diimines, such as, for example, 1,2-bis-(methylimino)ethane, 1,2-bis(ethylimino)ethane, 1,2-bis(isopropylimino)-ethane, 1,2-bis(*tert-*butylimino)ethane, 2,3-bis(methylimino)butane, 2,3-bis-(ethylimino)butane, 2,3-bis(isopropylimino)butane, 2,3-bis(*tert-*butylimino)-butane, 1,2-bis(phenylimino)ethane, 1,2-bis(2-methylphenylimino)ethane, 1,2-bis(2,6-diisopropylphenylimino)ethane, 1,2-bis(2,6-di-*tert*-butylphenylimino)ethane, 2,3-bis(phenylimino)butane, 2,3-bis(2-methylphenylimino)-butane, 2,3-bis(2,6-diisopropylphenylimino)butane, 2,3-bis(2,6-di-*tert*-butyl-phenylimino)butane, heterocycles containing two nitrogen atoms, such as, for example, 2,2'-bipyridine, o-phenanthroline, diphosphines, such as, for example, bisdiphenylphosphinomethane, bisdiphenylphosphinoethane, bis-(diphenylphosphino)propane, bis(diphenylphosphino)butane, bis(dimethylphosphino)methane, bis(dimethylphosphino)ethane, bis(dimethylphosphino)propane, bis(diethylphosphino)methane, bis(diethylphosphino)-ethane, bis(diethylphosphino)propane, bis(di-*tert-*butylphosphino)methane, bis(di-*tert-*butylphosphino)ethane, bis(*tert-*butylphosphino)propane, 1,3-diketonates derived from 1,3-diketones, such as, for example, acetylacetone, benzoylacetone, 1,5-diphenylacetylacetone, dibenzoylmethane, bis(1,1,1-trifluoroacetyl)methane, 3-ketonates derived from 3-ketoesters, such as, for example, ethyl acetoacetate, carboxylates derived from aminocarboxylic acids, such as, for example, pyridine-2-carboxylic acid, quinoline-2-carboxylic acid, glycine, N,N-dimethylglycine, alanine, N,N-dimethylaminoalanine, salicyliminates derived from salicylimines, such as, for example, methylsalicylimine, ethylsalicylimine, phenylsalicylimine, dialcoholates derived from dialcohols, such as, for example, ethylene glycol, 1,3-propylene glycol, and dithiolates derived from dithiols, such as, for example, 1,2-ethylenedithiol, 1,3-propylenedithiol.

Preferred tridentate ligands are borates of nitrogen-containing heterocycles, such as, for example, tetrakis(1-imidazolyl) borate and tetrakis(1-pyrazolyl) borate.

Particular preference is furthermore given to bidentate, monoanionic ligands L' which form, with the metal, a cyclometallated five-membered or six-membered ring having at least one metal-carbon bond, in particular a cyctometallated five-membered ring. These are, in particular, ligands as generally used in the area of phosphorescent metal complexes for organic electroluminescent devices, i.e. ligands of the phenylpyridine, naphthylpyridine, phenylquinoline, phenylisoquinoline, etc., type, each of which may be substituted by one or more radicals R¹ to R⁷. A multiplicity of ligands of this type is known to the person skilled in the art in the area of phosphorescent electroluminescent devices, and he will be able to select further ligands of this type, without inventive step, as ligand L' for compounds of the Formula (1). In general, the combination of two groups, as represented by the following Formulae (236) to (263), is particularly suitable for this purpose, where one group is bonded via a neutral nitrogen atom or a carbene atom and the other group is bonded via a negatively charged carbon atom or a negatively charged nitrogen atom. The ligand L' can then be formed from the groups of the Formulae (236) to (263) by these groups bonding to one another, in each case at the position denoted by #. The position at which the groups coordinate to the metal is denoted by *. These groups may also be bonded to the ligand L via one or two bridging units Z.

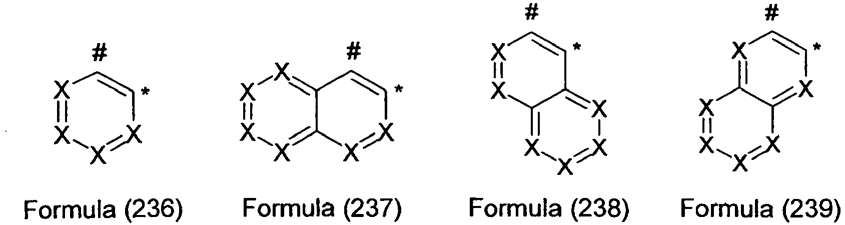

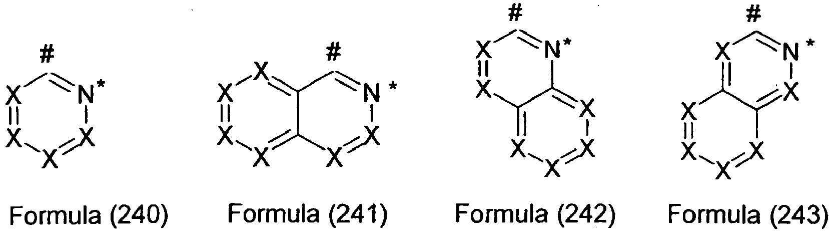

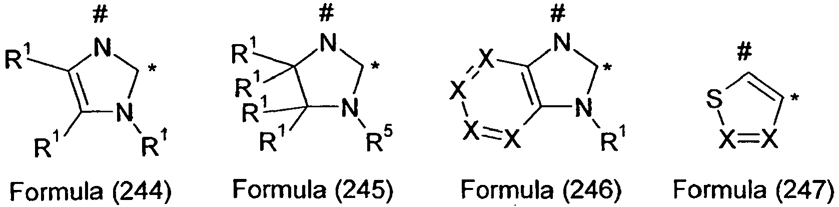

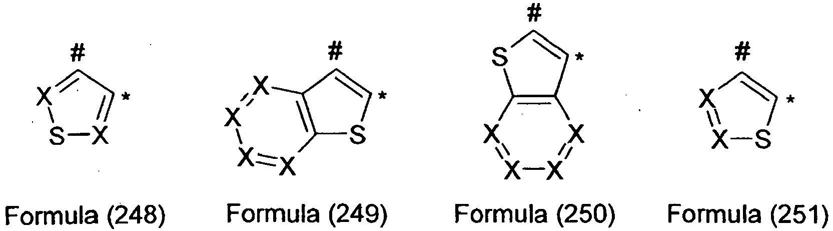

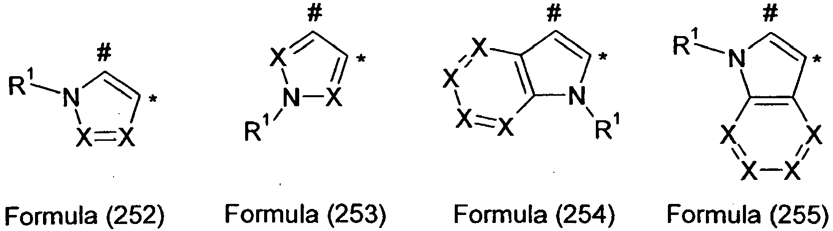

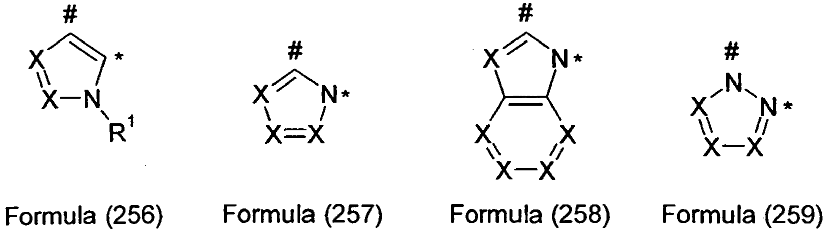

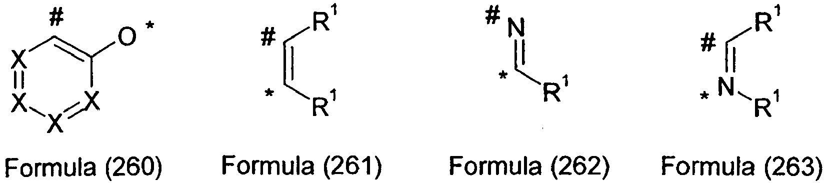

The symbols used here have the same meaning as described above, and preferably a maximum of three symbols X in each group stand for N, particularly preferably a maximum of two symbols X in each group stand for N, very particularly preferably a maximum of one symbol X in each group stands for N. Especially preferably, all symbols X stand, identically or differently on each occurrence, for CR¹.

In a particularly preferred embodiment of the present invention, two fragments of a ligand L' of the Formulae (236) to (263) are combined with one another via position # in such a way that at least one of the fragments contains a heteroatom at position *.

In a very particularly preferred embodiment of the present invention, the ligand L' is composed of precisely one fragment with no heteroatom from the list of the Formulae (236) to (263) and precisely one fragment with a heteroatom, preferably a nitrogen atom, from the list of the fragments having the Formulae (236) to (263).

Likewise preferred ligands L' are η⁵-cyclopentadienyl, η⁵-pentamethylcyclo-pentadienyl, η⁶-benzene and η⁷-cycloheptatrienyl, each of which may be substituted by one or more radicals R¹.

Likewise preferred ligands L' are 1,3,5-cis-cyclohexane derivatives, in particular of the Formula (264), 1,1,1-tri(methylene)methane derivatives, in particular of the formula (265), and 1,1,1-trisubstituted methanes, in particular of the formulae (266) and (267), 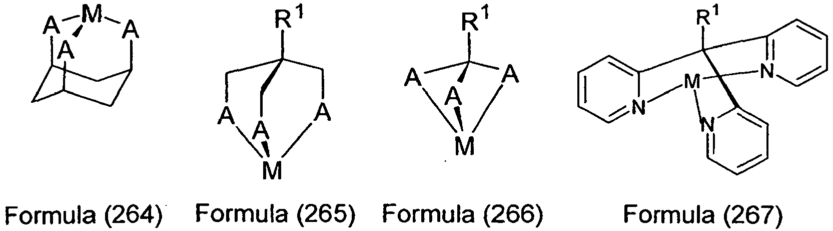 where, in each of the formulae, the coordination to the metal M is depicted, R¹ has the meaning mentioned above, and A stands, identically or differently on each occurrence, for O⁻, S⁻, COO⁻, P(R¹)₂ or N(R¹)₂.

The present invention furthermore relates to a process for the preparation of the metal-complex compounds of the Formula (1) by reaction of the corresponding free ligands bearing at least one azide group with metal alkoxides of the Formula (268), with metal ketoketonates of the Formula (269) or with metal halides of the Formula (270),

M(OR¹)ₙ Formula (268)

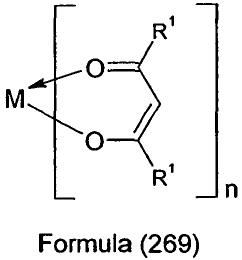

MHalₙ Formula (270)

where the symbols M, n and R¹ have the meanings indicated above, and Hal = F, Cl, Br or I.

It is likewise possible to use metal compounds, in particular iridium compounds, which carry both alcoholate and/or halide and/or hydroxyl radicals as well as ketoketonate radicals. These compounds may also be charged. Corresponding iridium compounds which are particularly suitable as starting materials are disclosed in WO 04/085449. [IrCl₂(acac)₂]⁻, for example Na[IrCl₂(aca_{c})₂], is particularly suitable.

The complexes are preferably synthesised as described in WO 02/060910 and in WO 2004/085449. Heteroleptic complexes can also be synthesised, for example, in accordance with WO 2005/042548. The synthesis can also be activated, for example, thermally, photochemically and/or by microwave radiation.

The product A' obtained by the reaction described above then reacts with B' to obtain C' as indicated by Schema (1).

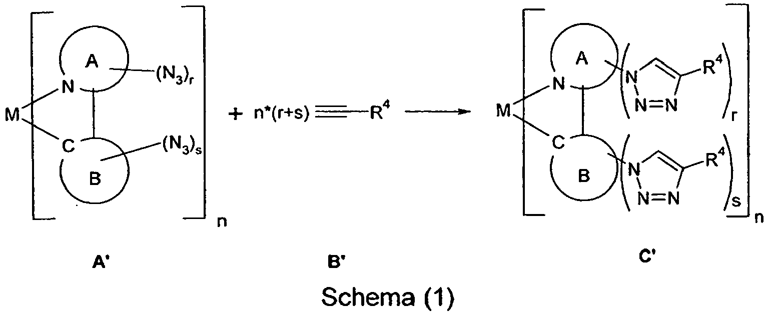

Details for this type of click chemistry are provided by V. V. Rostovtsev, L. G. Green, V. V. Fokin, and K. B. Sharpless in Angew. Chem. Int. Ed., 2002, 41, 2596-2599. One of the main advantages of this reaction is high yield that can be achieved.

Thus, the present invention also relates to the preparation of a compound according to Formula (1) pursuant Schema (1).

These processes enable the compounds of the Formula (1) according to the invention to be obtained in high purity, preferably greater than 99% (determined by means of ¹H-NMR and/or HPLC).

The synthetic methods explained here enable the preparation of, inter alia, the compounds of the Formulae (271) to (432) according to the invention depicted below.

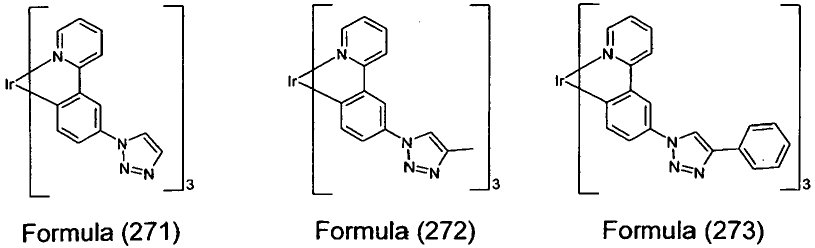

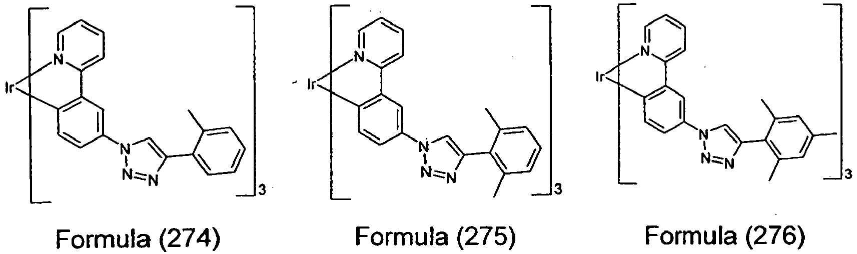

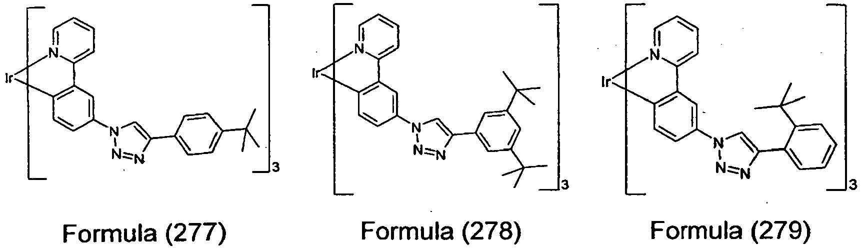

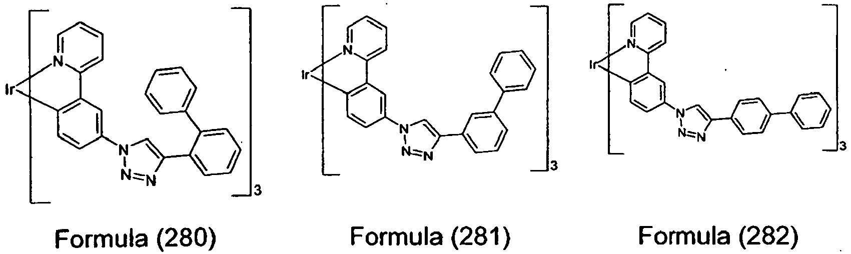

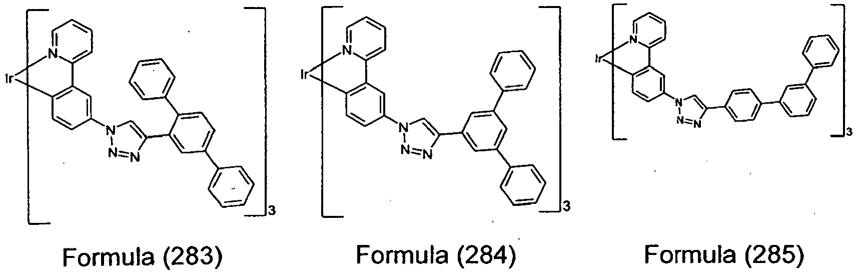

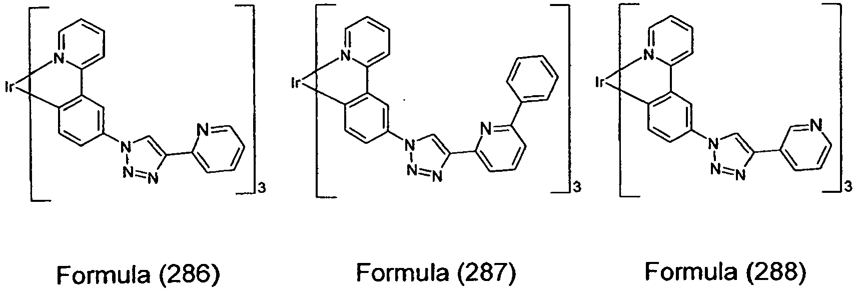

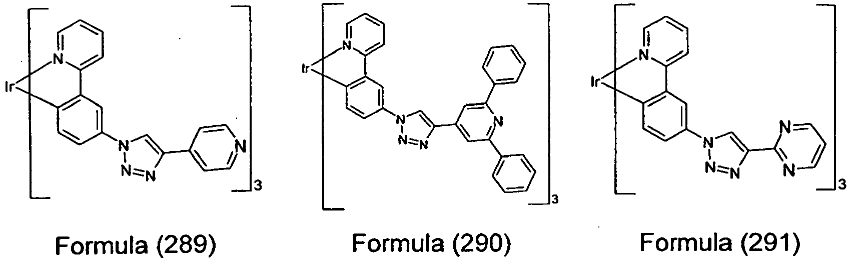

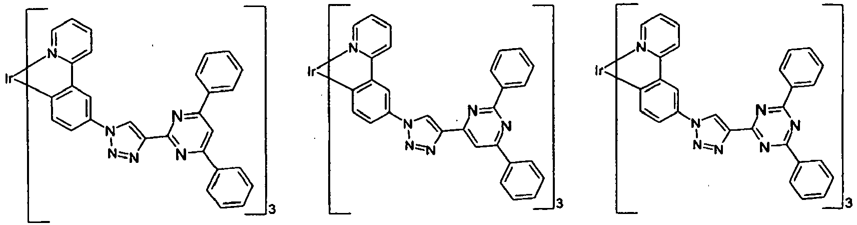

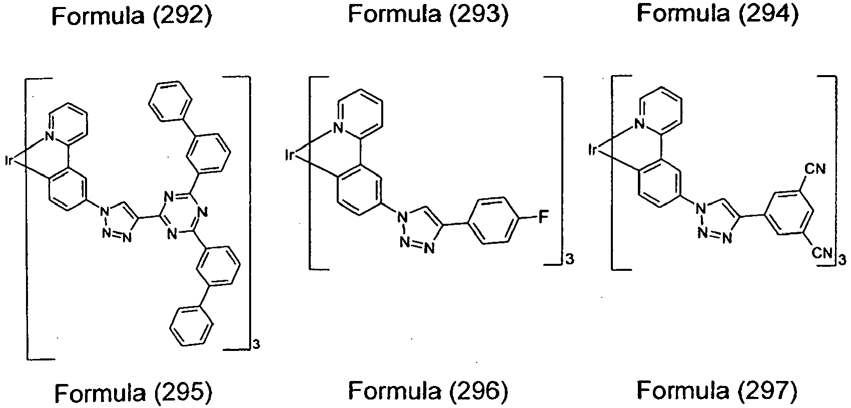

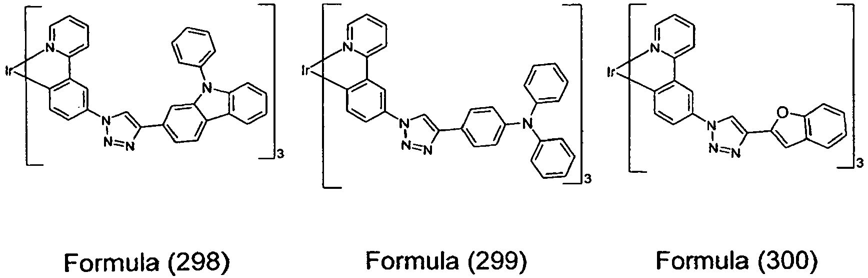

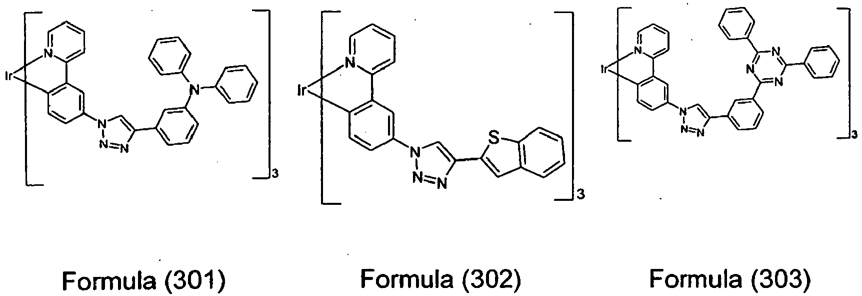

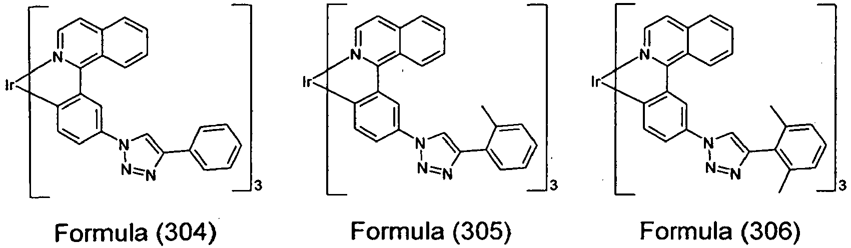

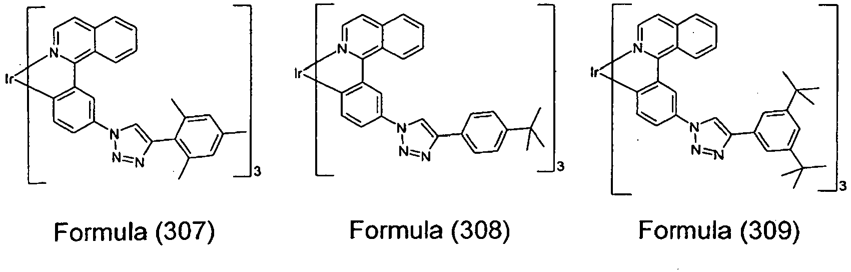

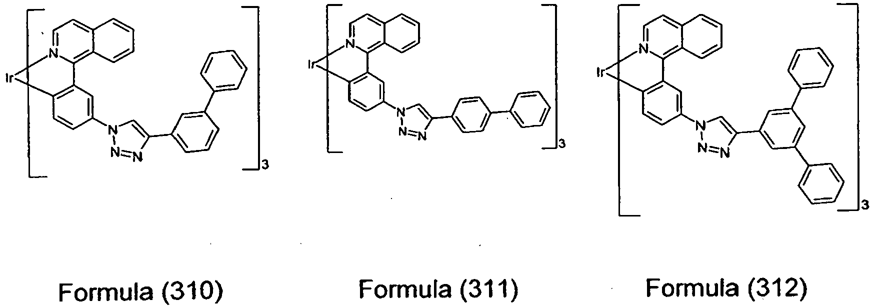

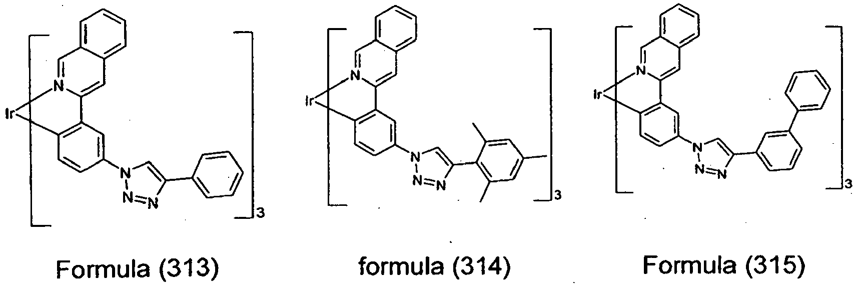

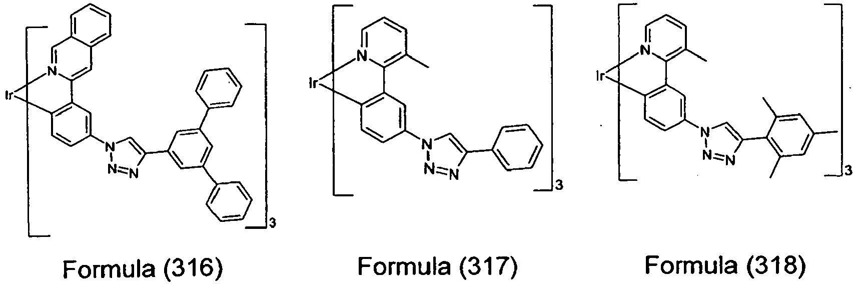

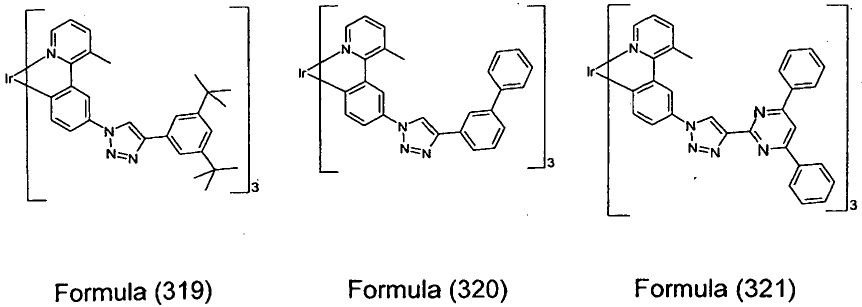

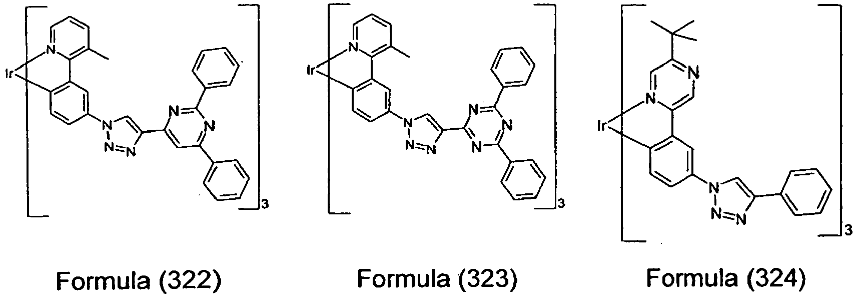

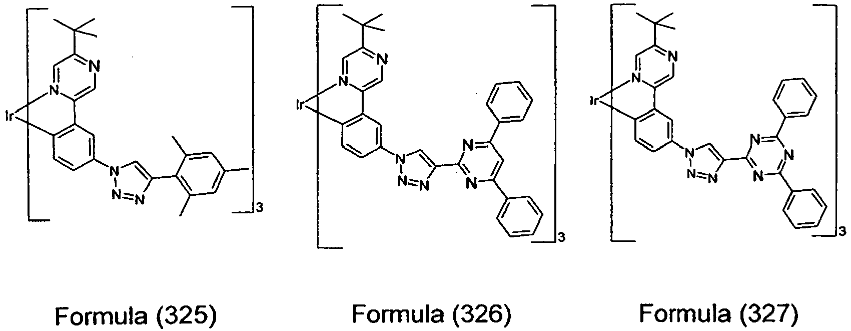

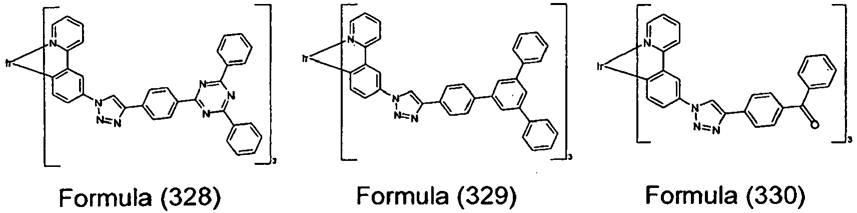

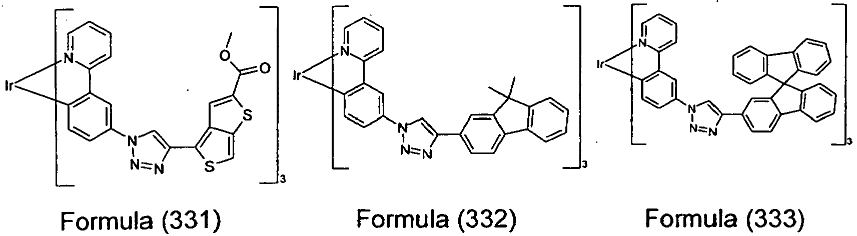

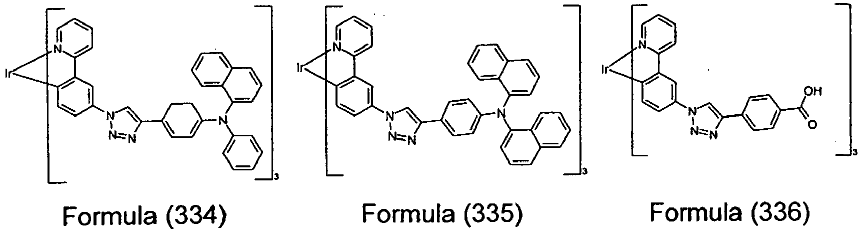

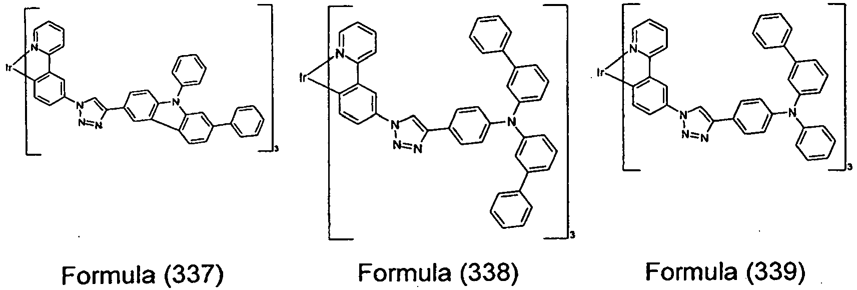

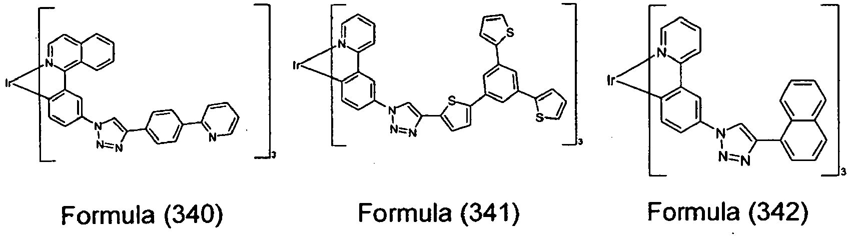

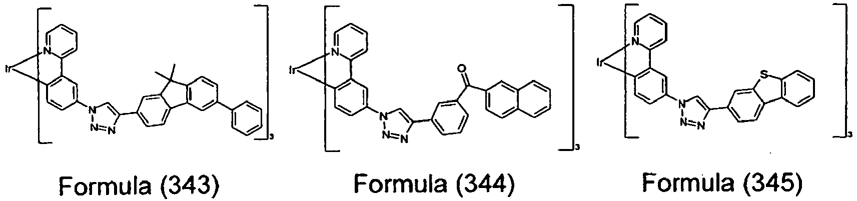

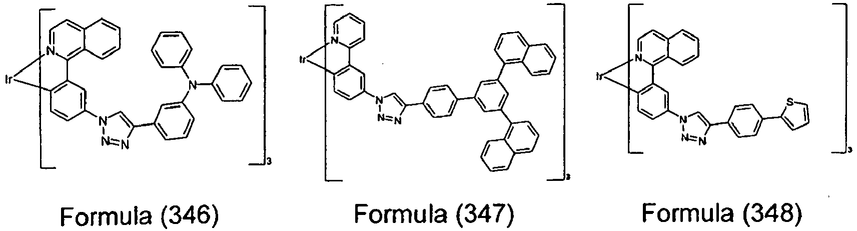

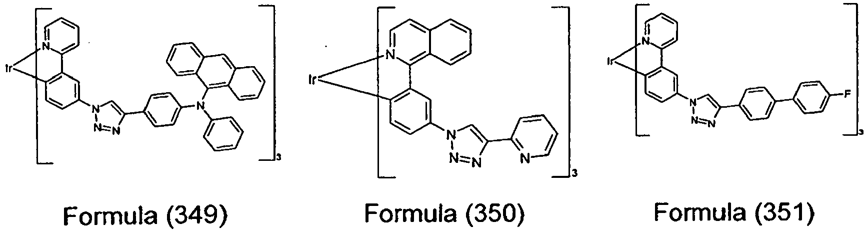

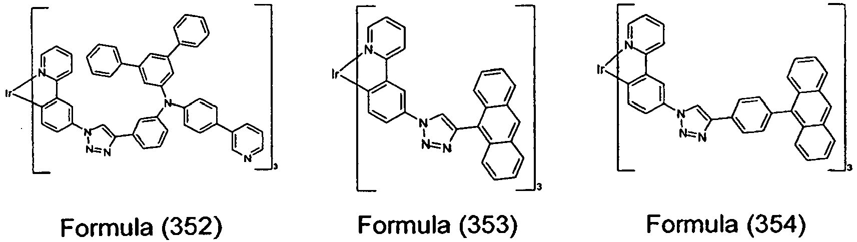

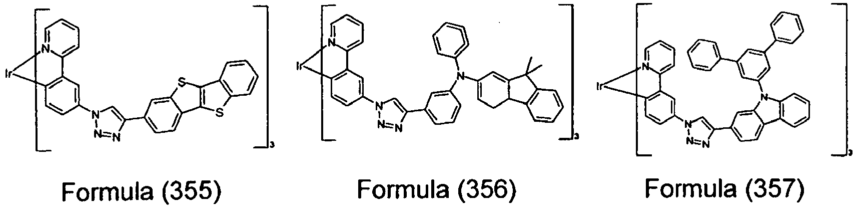

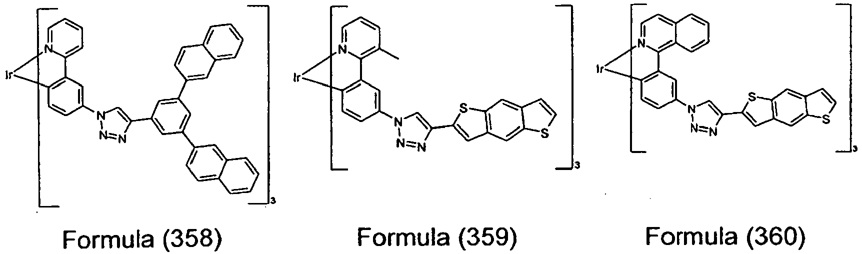

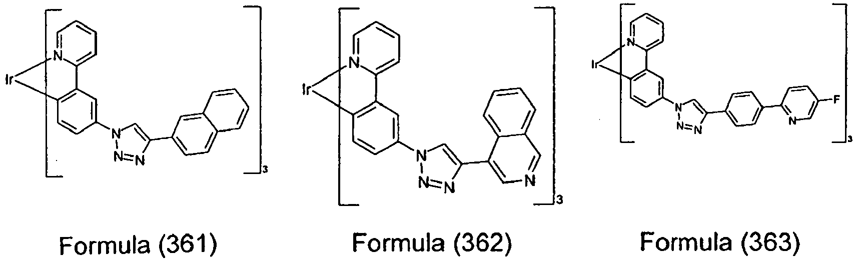

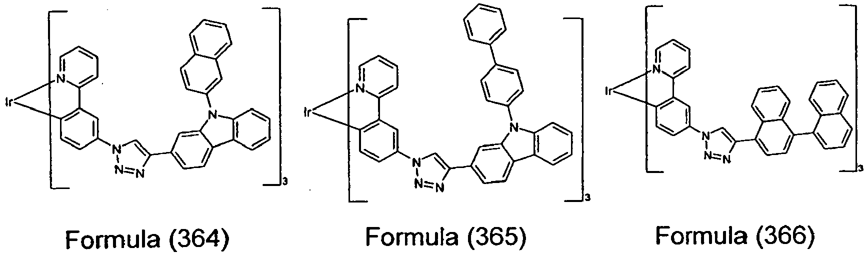

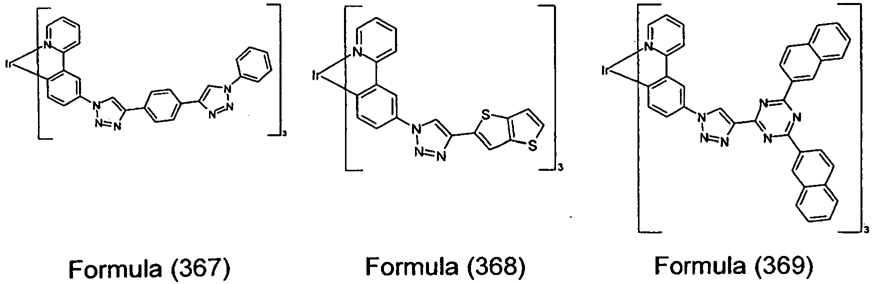

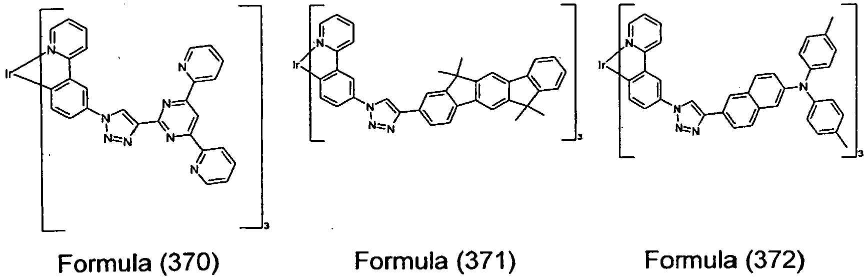

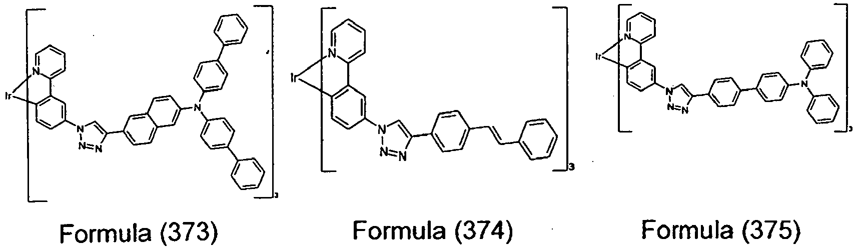

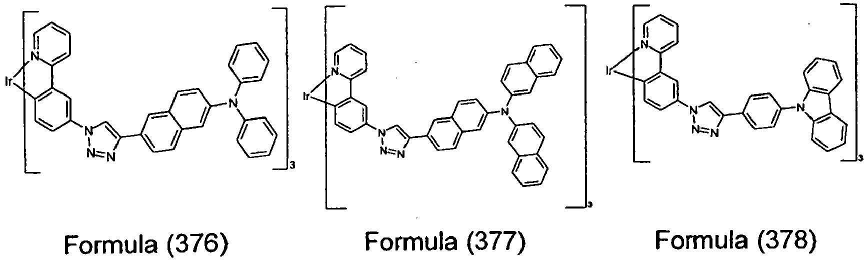

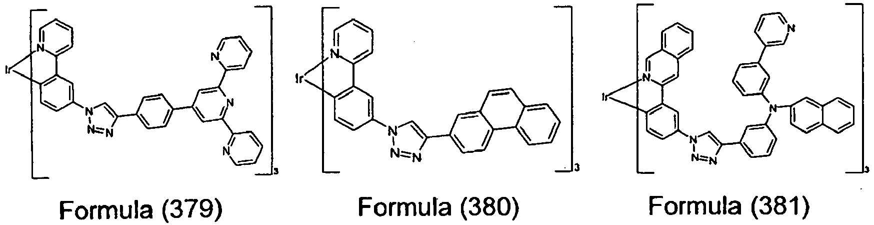

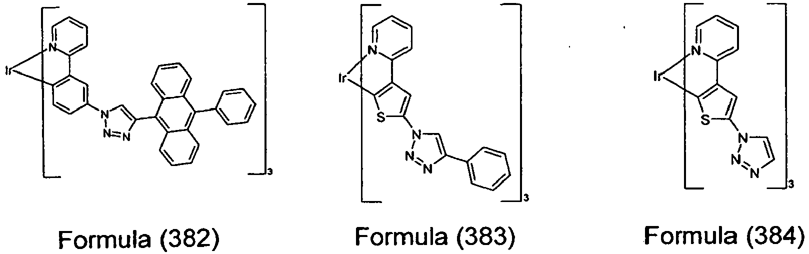

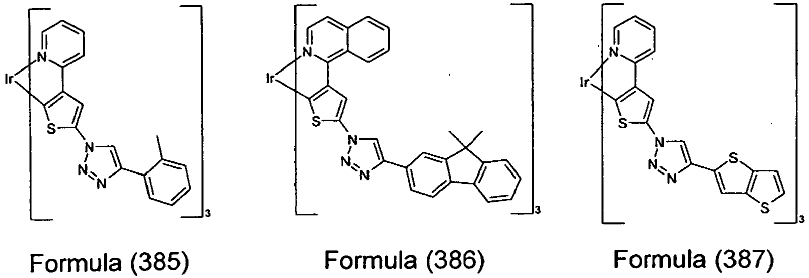

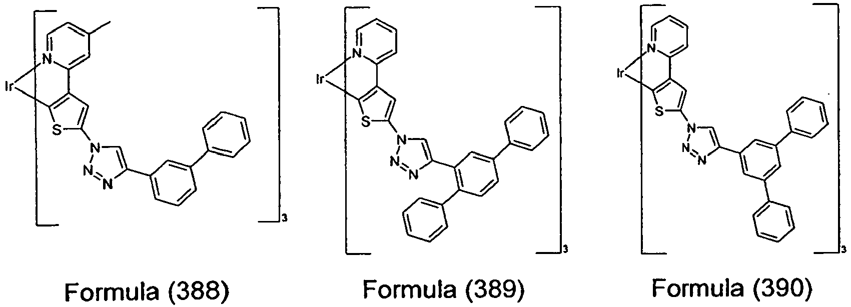

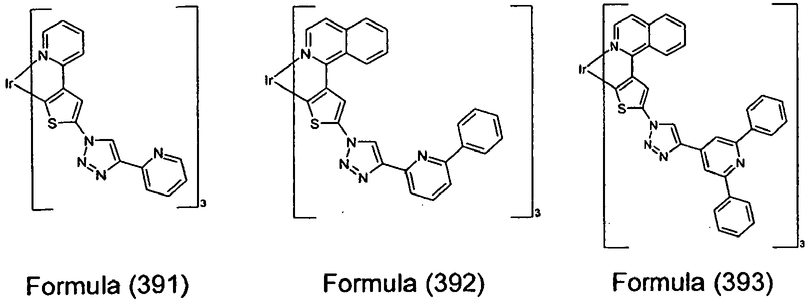

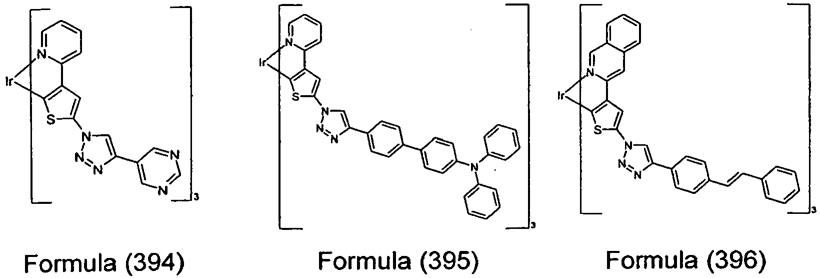

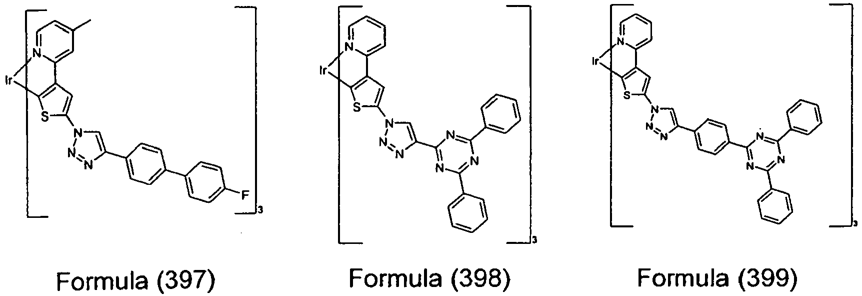

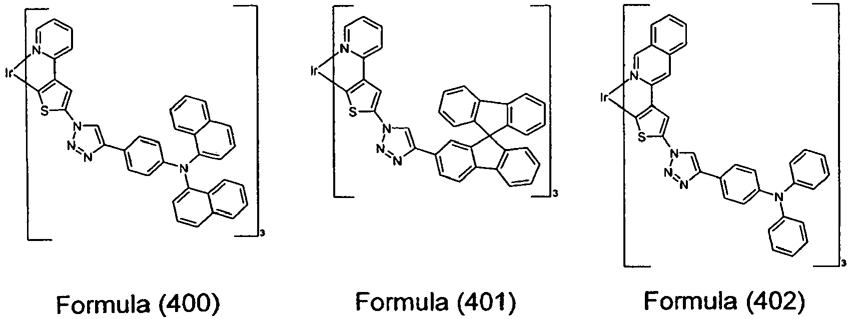

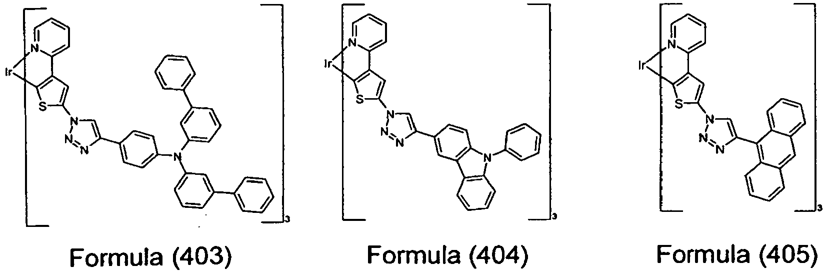

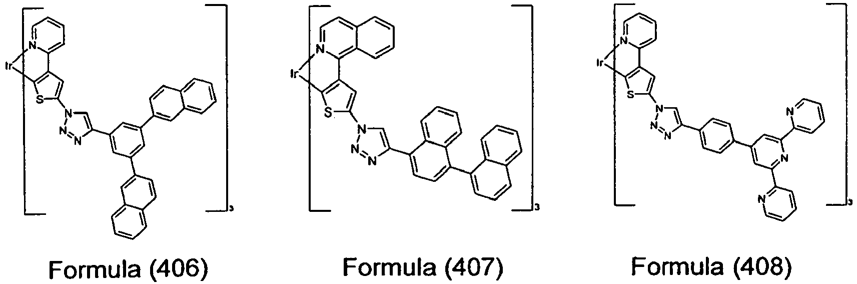

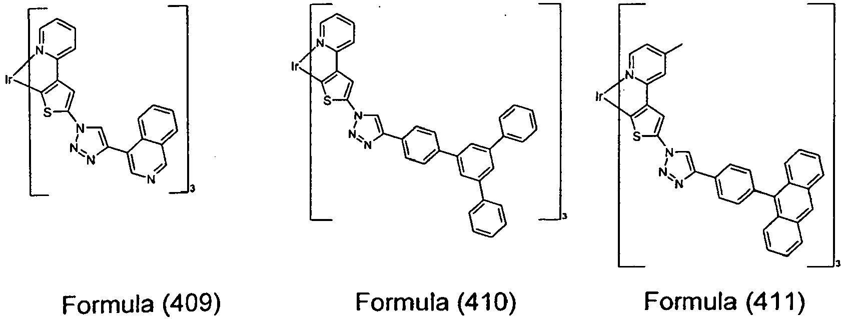

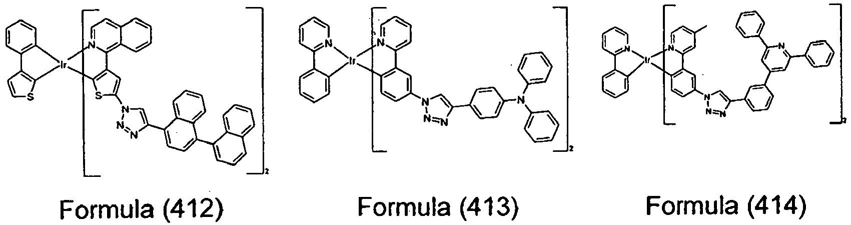

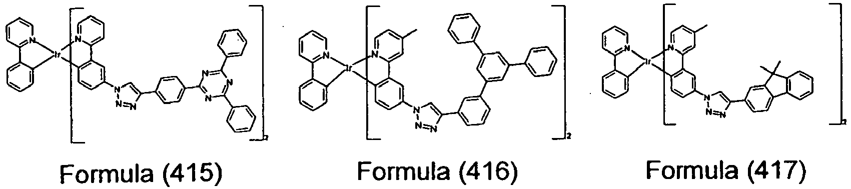

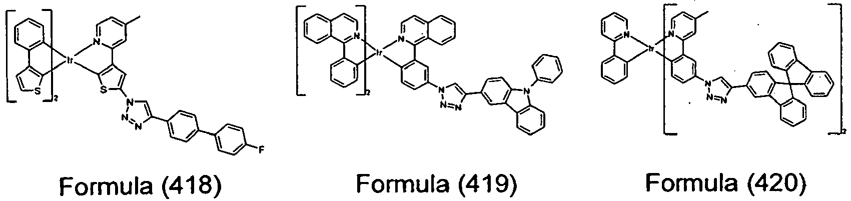

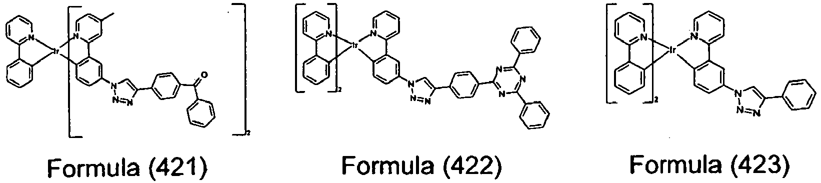

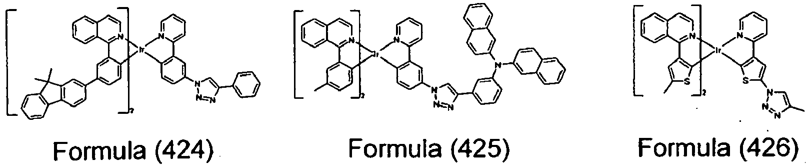

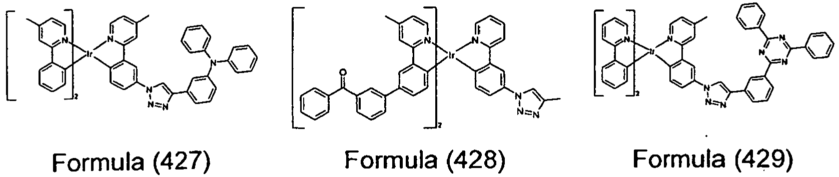

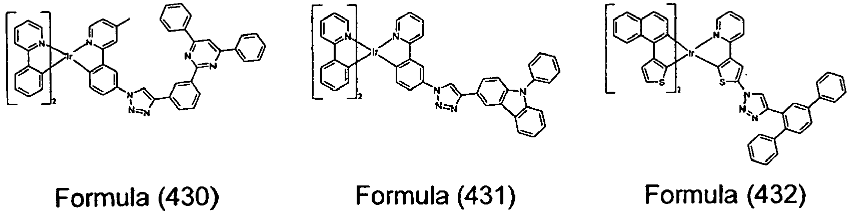

The complexes of the Formula (1) described above and the preferred embodiments mentioned above can be used as active component in the electronic device. An electronic device is taken to mean a device which comprises an anode, a cathode and at least one layer, where this layer comprises at least one organic or organometallic compound. The electronic device according to the invention thus comprises an anode, a cathode and at least one layer which comprises at least one compound of the Formula (1) indicated above. Preferred electronic devices here are selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) or organic laser diodes (O-lasers), comprising, in at least one layer, at least one compound of the Formula (1) indicated above. Particular preference is given to organic electroluminescent devices. Active components are generally the organic or inorganic materials, which are introduced between the anode and cathode, for example charge-injection, charge-transport or charge-blocking materials, but in particular emission materials and matrix materials. The compounds according to the invention exhibit particularly good properties as emission material in organic electroluminescent devices. Organic electroluminescent devices are therefore a preferred embodiment of the invention.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, charge-generation layers and/or organic or inorganic p/n junctions. It is likewise possible for interlayers, which have, for example, an exciton-blocking function and/or control the charge balance in the electroluminescent device, to be introduced between two emitting layers. However, it should be pointed out that each of these layers does not necessarily have to be present. The organic electroluminescent device may comprise one emitting layer or a plurality of emitting layers. If a plurality of emission layers are present, these preferably have in total a plurality of emission maxima between 300 nm and 800 nm, resulting overall in white emission, i.e. various emitting compounds which are able to fluoresce or phosphoresce are used in the emitting layers. Particular preference is given to three-layer systems, where the three layers exhibit blue, green and orange or red emission (for the basic structure see, for example, WO 2005/011013), or systems which comprise more than three emitting layers.

In a preferred embodiment of the invention, the organic electroluminescent device comprises the compound of the Formula (1) or the preferred embodiments mentioned above as emitting compound in one or more emitting layers.

If the compound of the Formula (1) is employed as emitting compound in an emitting layer, it is preferably employed in combination with one or more matrix materials. The mixture of the compound of the Formula (1) and the matrix material comprises between 1 and 99% by weight, preferably between 2 and 50% by weight, particularly preferably between 3 and 40% by weight, in particular between 5 and 30% by weight, of the compound of the formula (1), based on the entire mixture comprising emitter and matrix material. Correspondingly, the mixture comprises between 99 and 1% by weight, preferably between 98 and 50% by weight, particularly preferably between 97 and 60% by weight, in particular between 95 and 70% by weight, of the matrix material, based on the entire mixture comprising emitter and matrix material.

Suitable matrix materials for the compounds according to the invention are ketones, phosphine oxides, sulfoxides and sulfones, for example in accordance with WO 2004/013080, WO 2004/093207, WO 2006/005627 or the application DE 102008033943, triarylamines, carbazole derivatives, for example CBP (N,N-biscarbazolylbiphenyl) or the carbazole derivatives disclosed in WO 2005/039246, US 2005/0069729, JP 2004/288381, EP 1205527 or WO 2008/086851, indolocarbazole derivatives, for example in accordance with WO 2007/063754 or WO 2008/056746, indenocarbazole derivatives, for example in accordance with the applications DE 102009023155 and DE 102009031021, azacarbazoles, for example in accordance with EP 1617710, EP 1617711, EP 1731584, JP 2005/347160, bipolar matrix materials, for example in accordance with WO 2007/137725, silanes, for example in accordance with WO 2005/111172, azaboroles or boronic esters, for example in accordance with WO 2006/117052, triazine derivatives, for example in accordance with the application DE 102008036982, WO 2007/063754 or WO 2008/056746, zinc complexes, for example in accordance with EP 652273 or in accordance with WO 2009/062578, diaza- or tetraazasilole derivatives, for example in accordance with the application DE 102008056688, or diazaphosphole derivatives, for example in accordance with application DE 102009022858.

It may also be preferred to employ a plurality of different matrix materials as a mixture, in particular at least one electron-conducting matrix material and at least one hole-conducting matrix material. A preferred combination is, for example, the use of an aromatic ketone or a triazine with a triarylamine derivative or a carbazole derivative as mixed matrix for the metal complex according to the invention. Preference is likewise also given to mixtures of a hole- or electron-transporting material with a material which is involved in neither hole transport nor electron transport, as disclosed, for example, in DE 102009014513.

In a further preferred embodiment of the present invention, the compounds according to the invention can be employed in mixtures with one or more further emitters. Very particular preference is given here to a mixture of the compounds according to the invention with one or more fluorescent emitters. Preference is furthermore given to a mixture with one or more phosphorescent emitters. Fluorescent emitters emit principally from excited singlet states, whereas phosphorescent emitters emit light principally from higher spin states (for example triplet and quintet). For the purposes of this invention, the complexes of organic transition metals are taken to be phosphorescent emitters. The further emitters are preferably organic compounds.

In a particularly preferred embodiment of the present invention, the compounds according to the invention are mixed with 3 further emitters, in a particularly preferred embodiment with 2 further emitters and in an especially very preferred embodiment with one further emitter.

In a further preferred embodiment of the present invention, the emitter mixtures comprise 3, particularly preferably 2 and very particularly preferably one compound according to the invention.

In a particularly preferred embodiment of the present invention, the emitter mixtures comprise precisely one of the compounds according to the invention and precisely one further emitter.

It is furthermore preferred for the purposes of the present invention for the absorption spectra of at least one emitter and the emission spectrum of at least one other emitter of the mixture to overlap, simplifying energy transfer (double doping) between the emitters. The energy transfer here can take place by various mechanisms. Non-definitive examples of this are Förster or Dexter energy transfer.

The emitter mixtures described preferably comprise at least two emitters which both emit red light. Preference is furthermore given to emitter mixtures comprising at least two emitters which both emit green light. Preference is furthermore given to emitter mixtures comprising at least one emitter which emits red light and at least one emitter which emits green light.

The compound according to the present invention can, as outlined above, be mixed with further matrix materials. Besides matrix materials the compounds according to the present invention can also be mixed with any other organic functional material that is typically employed in electronic devices. Thus, the present invention also relates to a composition comprising at least one compound according to Formula (1) and at least one organic functional material selected from hole transport material (HTM), hole injection material (HIM), electron transport material (ETM), electron injection material (EIM), hole blocking material (HBM), exciton blocking material (ExBM), host or matrix material, fluorescent emitter, phosphorescent emitter, preferably matrix materials.

The cathode preferably comprises metals having a low work function, metal alloys or multilayered structures comprising various metals, such as, for example, alkaline-earth metals, alkali metals, main-group metals or lanthanoids (for example Ca, Ba, Mg, Al, In, Mg, Yb, Sm, etc.). Also suitable are alloys of an alkali or alkaline-earth metal and silver, for example an alloy of magnesium and silver. In the case of multilayered structures, further metals which have a relatively high work function, such as, for example, Ag, may also be used in addition to the said metals, in which case combinations of the metals, such as, for example, Ca/Ag or Ba/Ag, are generally used. It may also be preferred to introduce a thin interlayer of a material having a high dielectric constant between a metallic cathode and the organic semiconductor. Suitable for this purpose are, for example, alkali metal or alkaline-earth metal fluorides, but also the corresponding oxides or carbonates (for example LiF, Li₂O, BaF₂, MgO, NaF, CsF, CS₂CO₃, etc.). The layer thickness of this layer is preferably between 0.5 and 5 nm.

The anode preferably comprises materials having a high work function. The anode preferably has a work function of greater than 4.5 eV vs. vacuum. Suitable for this purpose are on the one hand metals having a high redox potential, such as, for example, Ag, Pt or Au. On the other hand, metal/metal oxide electrodes (for example Al/Ni/NiOₓ, Al/PtOₓ) may also be preferred. For some applications, at least one of the electrodes must be transparent in order to enable either irradiation of the organic material (O-SCs) or the coupling-out of light (OLEDs/PLEDs, O-lasers). A preferred structure uses a transparent anode. Preferred anode materials here are conductive mixed metal oxides. Particular preference is given to indium tin oxide (ITO) or indium zinc oxide (IZO). Preference is furthermore given to conductive doped organic materials, in particular conductive doped polymers.

In general, all materials as used for the layers in accordance with the prior art can be used in the further layers, and the person skilled in the art will be able to combine each of these materials with the materials according to the invention in an electronic device without inventive step.

The device is correspondingly (depending on the application) structured, provided with contacts and finally hermetically sealed, since the lifetime of devices of this type is drastically shortened in the presence of water and/or air.

Preference is furthermore given to an organic electroluminescent device, characterised in that one or more layers are applied by means of a sublimation process, in which the materials are vapour-deposited in vacuum sublimation units at an initial pressure of usually less than 10⁻⁵ mbar, preferably less than 10⁻⁶ mbar. It is also possible for the initial pressure to be even lower, for example less than 10⁻⁷ mbar.

Preference is likewise given to an organic electroluminescent device, characterised in that one or more layers are applied by means of the OVPD (organic vapour phase deposition) process or with the aid of carrier-gas sublimation, in which the materials are applied at a pressure between 10⁻⁵ mbar and 1 bar. A special case of this process is the OVJP (organic vapour jet printing) process, in which the materials are applied directly through a nozzle and thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, screen printing, flexographic printing or offset printing, but particularly preferably LITI (light induced thermal imaging, thermal transfer printing) or ink-jet printing. Since the compounds of the Formula (1) according to the invention have very good solubility in organic solvents, they are particularly suitable for processing from solution.

The organic electroluminescent device can also be produced as a hybrid system by applying one or more layers from solution and applying one or more other layers by vapour deposition. Thus, for example, it is possible to apply an emitting layer comprising a compound of the Formula (1) and a matrix material from solution and to apply a hole-blocking layer and/or an electron-transport layer on top by vacuum vapour deposition.

These processes are generally known to the person skilled in the art and can be applied by him without problems to organic electroluminescent devices comprising compounds of the Formula (1) or the preferred embodiments mentioned above.

For processing from solution, solutions or formulations of the compounds of the Formula (1) are necessary. It may also be preferred to use mixtures of two or more solvents. Suitable and preferred solvents are, for example, toluene, anisole, o-, m- or p-xylene, methyl benzoate, dimethylanisole, mesitylene, tetralin, veratrol, THF, methyl-THF, THP, chlorobenzene, dioxane, or mixtures of these solvents.

The present invention therefore furthermore relates to a solution or formulation comprising at least one compound of the Formula (1) and one or more solvents, in particular organic solvents. The way in which solutions of this type can be prepared is known to the person skilled in the art and is described, for example, in WO 02/072714, WO 03/019694 and the literature cited therein.

The electronic devices according to the invention, in particular organic electroluminescent devices, are distinguished by the following surprising advantages over the prior art:
1. The compounds of the Formula (1) have very good solubility in a multiplicity of common organic solvents and are therefore very highly suitable for processing from solution. In particular, the compounds according to the invention have higher solubility than the similar compounds described in the prior art.
2. Organic electroluminescent devices comprising compounds of the Formula (1) as emitting materials have an excellent lifetime. In particular, the lifetime is better than in the case of similar compounds in accordance with the prior art.
3. Organic electroluminescent devices comprising compounds of the Formula (1) as emitting materials have excellent efficiency. In particular, the efficiency is better than in the case of similar compounds in accordance with the prior art.
4. Organic electroluminescent devices comprising compounds of the Formula (1) as emitting materials required lower operating voltages as compared to similar compounds of the prior art.
5. The compounds according to the present invention can be obtained in high yield.
6. The compounds according to the present invention can be purified by easy purification processes.
7. The compounds according to the present invention show better film forming properties as compared to similar compounds of the prior art. This makes them more suitable for production, in particular for mass production, of electronic or opto-electronic devices comprising them.

These above-mentioned advantages are not accompanied by an impairment in the other electronic properties.

The compounds according to the invention are capable of emitting light under certain prerequisites. These compounds are thus very versatile. Some of the principal areas of application here are display or illumination technologies. It is furthermore particularly advantageous to employ the compounds and devices comprising these compounds in the area of phototherapy.

The present invention therefore furthermore relates to the use of the compounds according to the invention and devices comprising the compounds for the treatment, prophylaxis and diagnosis of diseases. The present invention still furthermore relates to the use of the compounds according to the invention and devices comprising the compounds for the treatment and prophylaxis of cosmetic conditions.

The present invention furthermore relates to the use of the compounds according to the invention for the production of devices for the therapy, prophylaxis and/or diagnosis of therapeutic diseases and/or for cosmetic applications.

Phototherapy or light therapy is used in many medical and/or cosmetic areas. The compounds according to the invention and the devices comprising these compounds can therefore be employed for the therapy and/or prophylaxis and/or diagnosis of all diseases and/or in cosmetic applications for which the person skilled in the art considers the use of phototherapy. Besides irradiation, the term phototherapy also includes photodynamic therapy (PDT) and disinfection and sterilisation in general. Phototherapy or light therapy can be used for the treatment of not only humans or animals, but also any other type of living or non-living materials. These include, for example, fungi, bacteria, microbes, viruses, eukaryotes, prokaryonts, foods, drinks, water and drinking water.

The term phototherapy also includes any type of combination of light therapy and other types of therapy, such as, for example, treatment with active compounds. Many light therapies have the aim of irradiating or treating exterior parts of an object, such as the skin of humans and animals, wounds, mucous membranes, the eye, hair, nails, the nail bed, gums and the tongue. The treatment or irradiation according to the invention can in addition also be carried out inside an object in order, for example, to treat internal organs (heart, lung, etc.) or blood vessels or the breast.

The therapeutic and/or cosmetic areas of application according to the invention are preferably selected from the group of skin diseases and skin-associated diseases or changes or conditions, such as, for example, psoriasis, skin ageing, skin wrinkling, skin rejuvenation, enlarged skin pores, cellulite, oily/greasy skin, folliculitis, actinic keratosis, precancerous actinic keratosis, skin lesions, sun-damaged and sun-stressed skin, crows' feet, skin ulcers, acne, acne rosacea, scars caused by acne, acne bacteria, photomodulation of greasy/oily sebaceous glands and their surrounding tissue, jaundice, jaundice of the newborn, vitiligo, skin cancer, skin tumours, Crigler-Najjar, dermatitis, atopic dermatitis, diabetic skin ulcers and desensitisation of the skin.

Particular preference is given for the purposes of the invention to the treatment and/or prophylaxis of psoriasis, acne, cellulite, skin wrinkling, skin ageing, jaundice and vitiligo.

Further areas of application according to the invention for the compositions and/or devices comprising the compositions according to the invention are selected from the group of inflammatory diseases, rheumatoid arthritis, pain therapy, treatment of wounds, neurological diseases and conditions, oedema, Paget's disease, primary and metastasising tumours, connective-tissue diseases or changes, changes in the collagen, fibroblasts and cell level originating from fibroblasts in tissues of mammals, irradiation of the retina, neovascular and hypertrophic diseases, allergic reactions, irradiation of the respiratory tract, sweating, ocular neovascular diseases, viral infections, particularly infections caused by herpes simplex or HPV (human papillomaviruses) for the treatment of warts and genital warts.

Particular preference is given for the purposes of the invention to the treatment and/or prophylaxis of rheumatoid arthritis, viral infections and pain.

Further areas of application according to the invention for the compounds and/or devices comprising the compounds according to the invention are selected from winter depression, sleeping sickness, irradiation for improving the mood, the reduction in pain, particularly muscular pain caused by, for example, tension or joint pain, elimination of the stiffness of joints and the whitening of the teeth (bleaching).

Further areas of application according to the invention for the compounds and/or devices comprising the compounds according to the invention are selected from the group of disinfections. The compounds according to the invention and/or the devices according to the invention can be used for the treatment of any type of objects (non-living materials) or subjects (living materials such as, for example, humans and animals) for the purposes of disinfection. This includes, for example, the disinfection of wounds, the reduction in bacteria, the disinfection of surgical instruments or other articles, the disinfection of foods, of liquids, in particular water, drinking water and other drinks, the disinfection of mucous membranes and gums and teeth. Disinfection here is taken to mean the reduction in the living microbiological causative agents of undesired effects, such as bacteria and germs.

For the purposes of the phototherapy mentioned above, devices comprising the compounds according to the invention preferably emit light having a wavelength between 250 and 1250 nm, particularly preferably between 300 and 1000 nm and particularly preferably between 400 and 850 nm.

In a particularly preferred embodiment of the present invention, the compounds according to the invention are employed in an organic light-emitting diode (OLED) or an organic light-emitting electrochemical cell (OLEC) for the purposes of phototherapy. Both the OLED and the OLEC can have a planar or fibre-like structure having any desired cross section (for example round, oval, polygonal, square) with a single- or multilayered structure. These OLECs and/or OLEDs can be installed in other devices which comprise further mechanical, adhesive and/or electronic elements (for example battery and/or control unit for adjustment of the irradiation times, intensities and wavelengths). These devices comprising the OLECs and/or OLEDs according to the invention are preferably selected from the group comprising plasters, pads, tapes, bandages, cuffs, blankets, caps, sleeping bags, textiles and stents.

The use of the said devices for the said therapeutic and/or cosmetic purpose is particularly advantageous compared with the prior art, since homogeneous irradiation of lower irradiation intensity is possible at virtually any site and at any time of day with the aid of the devices according to the invention using the OLEDs and/or OLECs. The irradiation can be carried out as an inpatient, as an outpatient and/or by the patient themselves, i.e. without initiation by medical or cosmetic specialists. Thus, for example, plasters can be worn under clothing, so that irradiation is also possible during working hours, in leisure time or during sleep. Complex inpatient/ outpatient treatments can in many cases be avoided or their frequency reduced. The devices according to the invention may be intended for reuse or be disposable articles, which can be disposed of after use once, twice or three times.

Further advantages over the prior art are, for example, lower evolution of heat and emotional aspects. Thus, newborn being treated owing to jaundice typically have to be irradiated blindfolded in an incubator without physical contact with the parents, which represents an emotional stress situation for parents and newborn. With the aid of a blanket according to the invention comprising the OLEDs and/or OLECs according to the invention, the emotional stress can be reduced significantly. In addition, better temperature control of the child is possible due to reduced heat production of the devices according to the invention compared with conventional irradiation equipment.

It should be pointed out that variations of the embodiments described in the present invention fall within the scope of this invention. Each feature disclosed in the present invention can, unless explicitly excluded, be replaced by alternative features which serve the same, an equivalent or a similar purpose. Thus, each feature disclosed in the present invention should, unless stated otherwise, be regarded as an example of a generic series or as an equivalent or similar feature.

All features of the present invention can be combined with one another in any way, unless certain features and/or steps are mutually exclusive. This applies, in particular, to preferred features of the present invention. Equally, features of non-essential combinations can be used separately (and not in combination).

It should furthermore be pointed out that many of the features, and in particular those of the preferred embodiments of the present invention, should be regarded as inventive themselves and not merely as part of the embodiments of the present invention. Independent protection may be granted for these features in addition or as an alternative to each invention claimed at present.

The teaching regarding technical action disclosed with the present invention can be abstracted and combined with other examples.

The invention is explained in greater detail by the following examples without wishing it to be restricted thereby.

### Working Examples

The following syntheses are, unless indicated otherwise, carried out under a protective-gas atmosphere in dried solvents. Compound (**I**) can be prepared in accordance with Inorg. Chem 2011, 50, 806. Compound (**III**) can be prepared in accordance with Eur. J. Inorg. Chem 2005, 3, 447. Compound **(V)** can be purchased by VWR. Compound (**VII**) can be prepared in accordance with Eur. J. Org. Chem 2011, 143. Compound (**IX**) can be prepared in accordance with J. Phys. Chem 2010, 114, 3924. Compound (**XI**) can be prepared in accordance with Polymer 2006, 47, 6551. Compound (**XIII**) can be prepared in accordance with Organometallics 2005, 24, 3966 and compound (**XV**) can be prepared in accordance with J. Chem. SOC 1988, 7, 1251. Compound **(XXIII),** (**XXV**) and **(XXVII)** can be prepared in accordance to the general procedure used for the preparation of compound (**II**). Compound (**XXI**) can be prepared in accordance with J. Med. Chem 2010, 53, 616. Compounds **(XVI)** and **(XVIII)** are commercially available.

### Example 1:

### Preparation of compound (IV)

Synthetic procedure for the preparation of compound **(IV):**

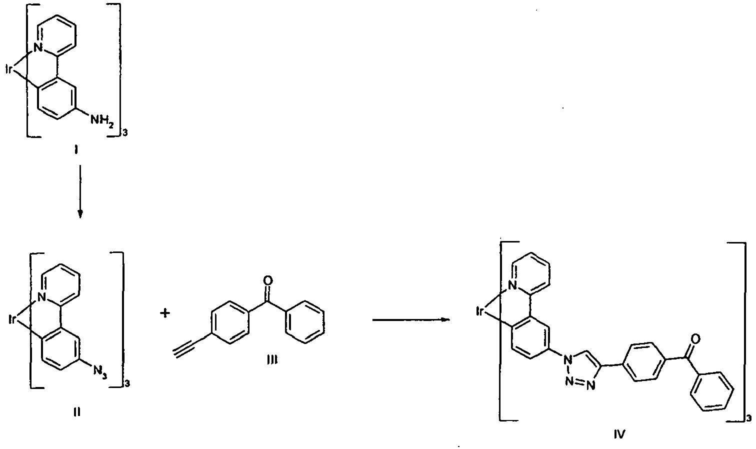

### a) Synthesis of compound (II)

To a solution of 100.0 g (142.2 mmol) of compound (I) in 200 ml of acetonitrile cooled to 0°C is added 16.5 g (160.5 mmol) of tert-butyl nitrite (t-BuONO) followed by 15.0 g (128.0 mmol) of azidotrimethylsilane (TMSN₃) dropwise. The resulting solution is stirred at room temperature for 3 h and then concentrated under vacuum. The crude product is used as obtained without further purification. The yield is 93.7 g (100.8 mmol), corresponding to 71% of theory.

### b) Synthesis of compound (IV)

7.4 g (39.2 mmol) of Cul are added to a suspension of 11.0 g (14.1 mmol) of compound (**II**) and 9.6 g (46.6 mmol) of compound (**III**) in 300 ml of a mixture of ethanol/water (7:3). The mixture is stirred at room temperature for 24 h. The resulting precipitate is filtered off, dissolved in dichloromethane (DCM), filtered over silica gel and washed three times with water. The organic phase is separated off, dried over magnesium sulfate, filtered and evaporated to dryness. The crude product is purified by recrystallization from a mixture of toluene/ethanol. The yield is 13.8 g (9.87 mmol), corresponding to 70% of theory.

### Example 2:

### Preparation of compound (VI)

Synthetic procedure for the preparation of compound (**VI**):

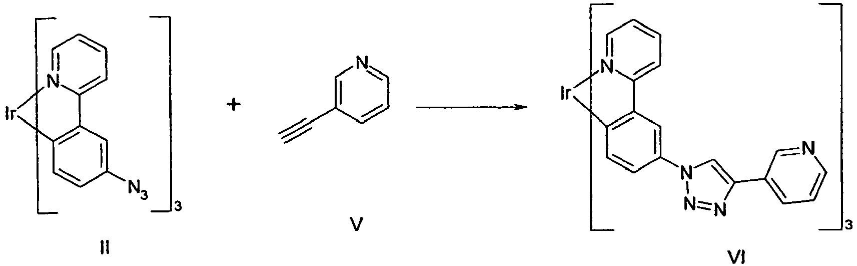

8.9 g (47.0 mmol) of Cul are added to a suspension of 12.0 g (16.9 mmol) of compound (**II**) and 5.8 g (55.9 mmol) of compound **(V)** in 350 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 12.0 g (11.0 mmol), corresponding to 65% of theory.

### Example 3:

### Preparation of compound (VIII)

Synthetic procedure for the preparation of compound (**VIII**):

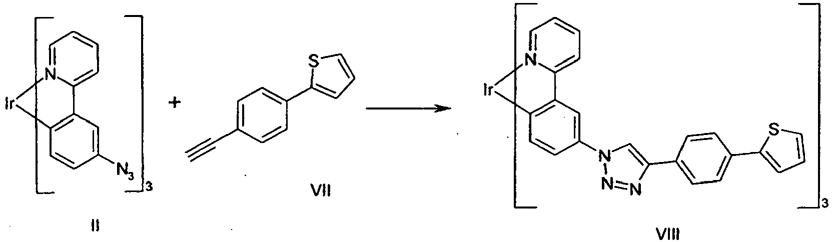

7.4 g (39.2 mmol) of Cul are added to a suspension of 11.0 g (14.1 mmol) of compound (**II**) and 8.6 g (46.6 mmol) of compound (**VII**) in 300 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 10.3 g (9.5 mmol), corresponding to 67% of theory.

### Example 4:

### Preparation of compound (X)

Synthetic procedure for the preparation of compound (**X**):

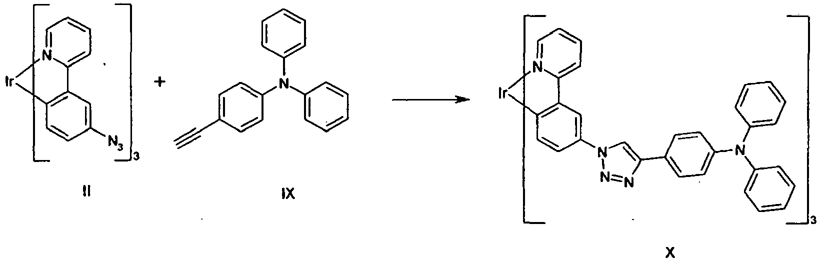

9.7 g (51.0 mmol) of Cul are added to a suspension of 16.6 g (21.2 mmol) of compound (**II**) and 16.2 g (60.5 mmol) of compound (**IX**) in 400 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 29.9 g (16.9 mmol), corresponding to 80% of theory.

### Example 5:

### Preparation of compound (XII)

Synthetic procedure for the preparation of compound (**XII**):

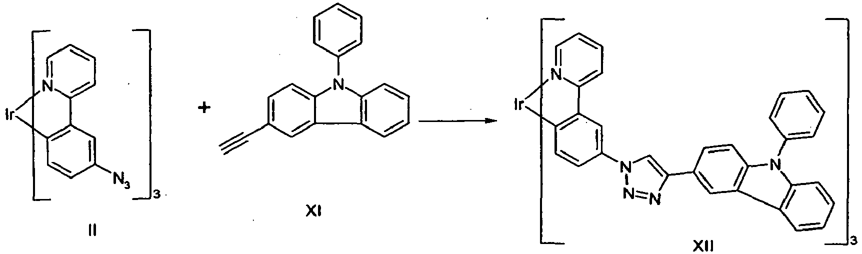

9.7 g (51.0 mmol) of Cul are added to a suspension of 14.3 g (18.3 mmol) of compound (**II**) and 16.2 g (60.5 mmol) of compound (**XI**) in 350 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 19.7 g (12.4 mmol), corresponding to 68% of theory.

### Example 6:

### Preparation of compound (XIV)

Synthetic procedure for the preparation of compound (**XIV**):

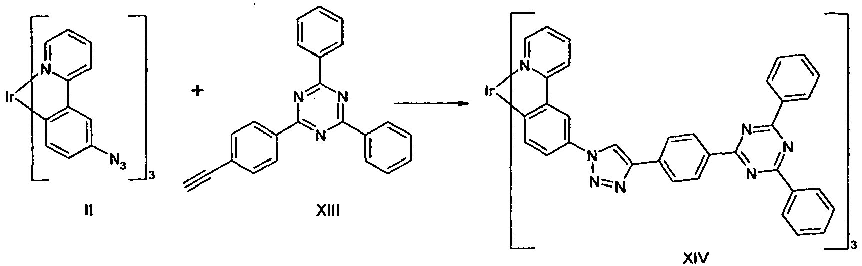

11.2 g (58.8 mmol) of Cul are added to a suspension of 16.6 g (21.2 mmol) of compound (**II**) and 23.3 g (69.8 mmol) of compound (**XIII**) in 400 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 26.9 g (15.1 mmol), corresponding to 71% of theory.

### Example 7:

### Preparation of compound (XVI)

Synthetic procedure for the preparation of compound (**XVI**):

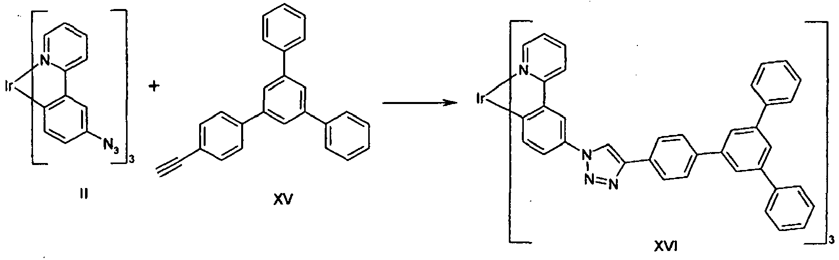

7.4 g (39.2 mmol) of Cul are added to a suspension of 11.0 g (14.1 mmol) of compound (**II**) and 15.5 g (46.6 mmol) of compound (**XV**) in 300 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 18.8 g (10.6 mmol), corresponding to 75% of theory.

### Example 8:

### Preparation of compound (XVIII)

Synthetic procedure for the preparation of compound (**XVIII**):

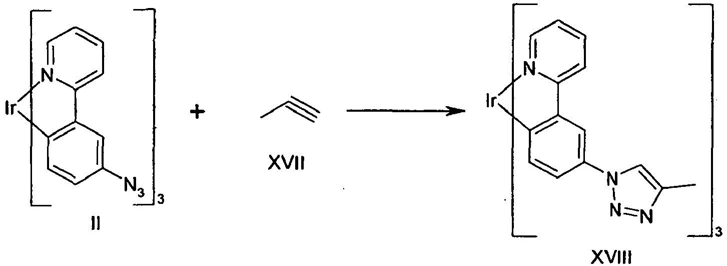

7.4 g (39.1 mmol) of Cul are added to a suspension of 11.0 g (14.1 mmol) of compound (**II**) and 1.9 g (46.6 mmol) of compound (**XVII**) in 300 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 8.5 g (9.5 mmol), corresponding to 67% of theory

### Example 9:

### Preparation of compound (XX)

Synthetic procedure for the preparation of compound (**XX**):

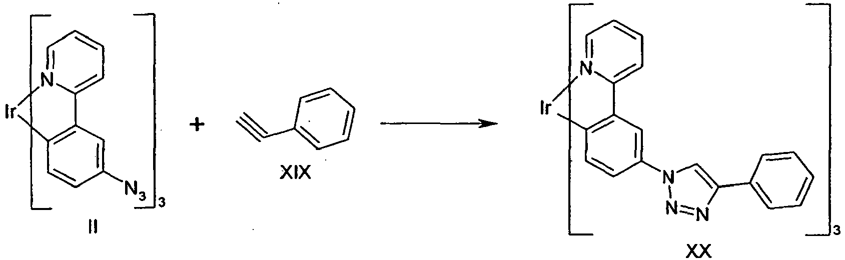

7.4 g (39.2 mmol) of Cul are added to a suspension of 11.0 g (14.1 mmol) of compound (**II**) and 4.7 g (46.6 mmol) of compound (**XIX**) in 300 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 11.1 g (10.0 mmol), corresponding to 89% of theory

### Example 10:

### Preparation of compound (XXII)

Synthetic procedure for the preparation of compound (**XXII**):

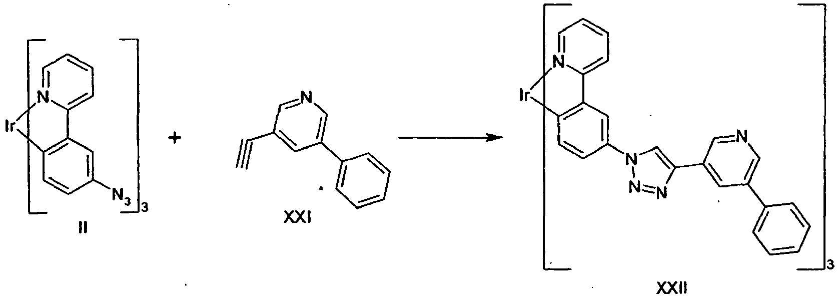

7.4 g (39.2 mmol) of CuI are added to a suspension of 11.0 g (14.1 mmol) of compound (**II**) and 8.3 g (46.6 mmol) of compound (**XXI**) in 300 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 12.8 g (9.7 mmol), corresponding to 69% of theory.

### Example 11:

### Preparation of compound (XXIV)

Synthetic procedure for the preparation of compound (**XXIV**):

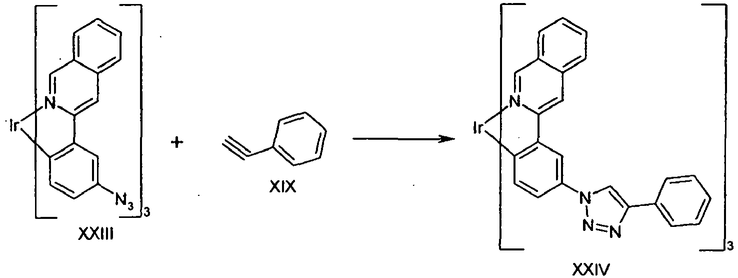

5.7 g (29.8 mmol) of Cul are added to a suspension of 10.0 g (10.7 mmol) of compound **(XXIII)** and 3.6 g (35.4 mmol) of compound (**XIX**) in 300 ml of a mixture of ethanol/water (7:3). The mixture is stirred at room temperature for 24 h. The resulting precipitate is filtered off, dissolved in dcm, filtered over silica gel and washed three times with water. The organic phase is separated off, dried over magnesium sulphate, filtered and evaporated to dryness. The crude product is purified by recrystallization from a mixture of toluene/ethanol. The yield is 10.6 g (8.6 mmol), corresponding to 70% of theory.

### Example 12:

### Preparation of compound (XXVI)

Synthetic procedure for the preparation of compound (**XXVI**):

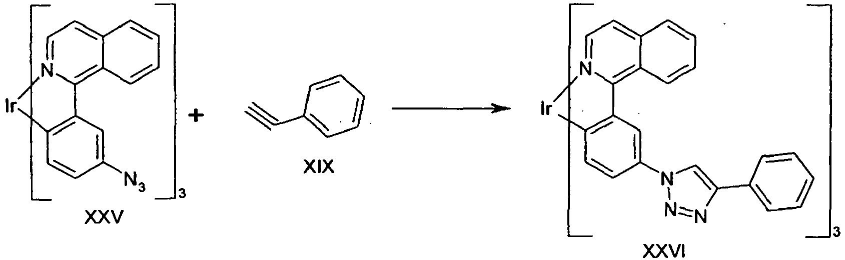

5.7 g (29.8 mmol) of CuI are added to a suspension of 10.0 g (10.7 mmol) of compound (**XXV**) and 3.6 g (35.4 mmol) of compound (**XIX**) in 300 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 8.7 g (7.0 mmol), corresponding to 84% of theory.

### Example 13:

### Preparation of compound (XXVIII)

Synthetic procedure for the preparation of compound (**XXVIII**):

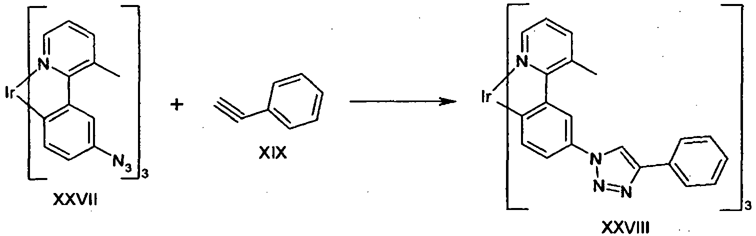

6.5 g (33.7 mmol) of Cul are added to a suspension of 10.0 g (12.1 mmol) of compound (**XXVII**) and 4.0 g (40.1 mmol) of compound (**XIX**) in 300 ml of a mixture of ethanol/water (7:3). The mixture is stirred at room temperature for 24 h. The resulting precipitate is filtered off, dissolved in DCM, filtered over silica gel and washed three times with water. The organic phase is separated off, dried over magnesium sulphate, filtered and evaporated to dryness. The crude product is purified by recrystallization from a mixture of toluene/ethanol. The yield is 8.7 g (7.6 mmol), corresponding to 63% of theory.

### Examples 14 to 27:

### Production and characterisation of organic electroluminescent devices

The structures of compounds TE-1 to TE-13 according to the invention, TMM-1 (synthesised in accordance with DE 102008036982 - WO 2010/015306) and TMM-2 (synthesised in accordance with DE 102008017591.9 - WO 2009/124628) are depicted below for clarity.

### Structures of the emitters related to this invention

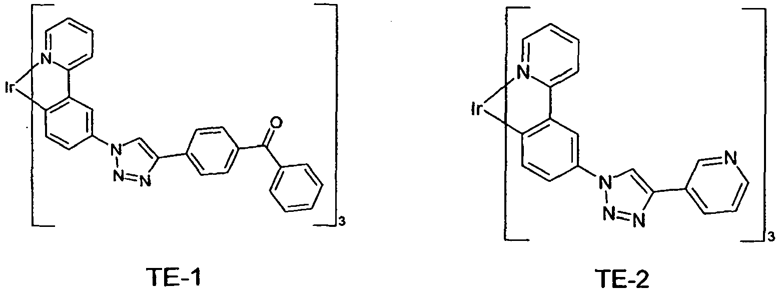

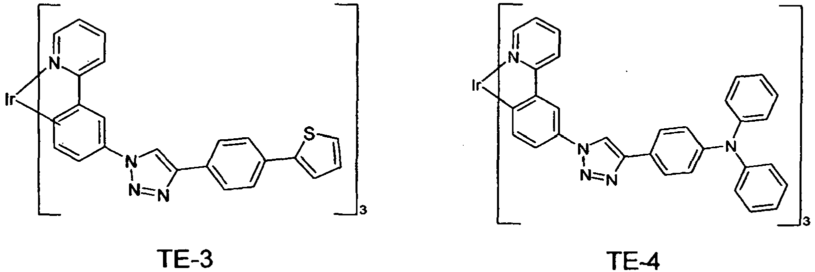

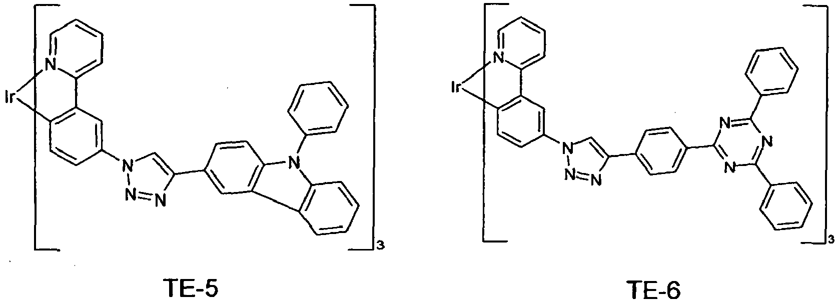

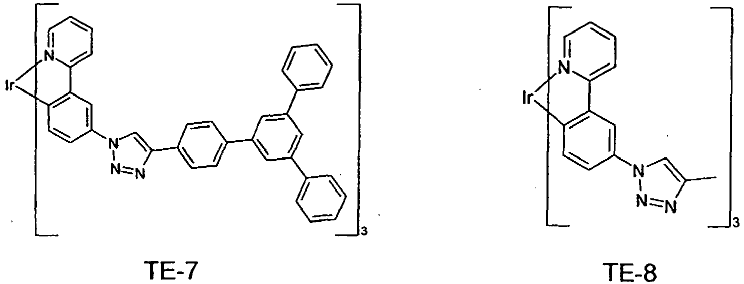

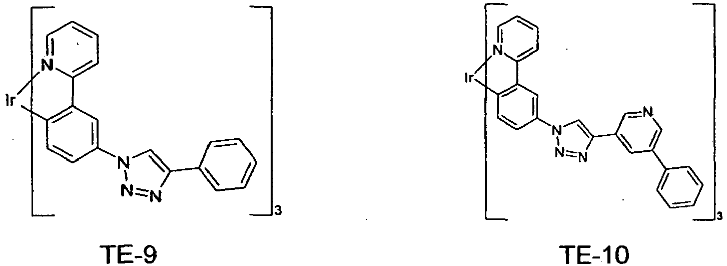

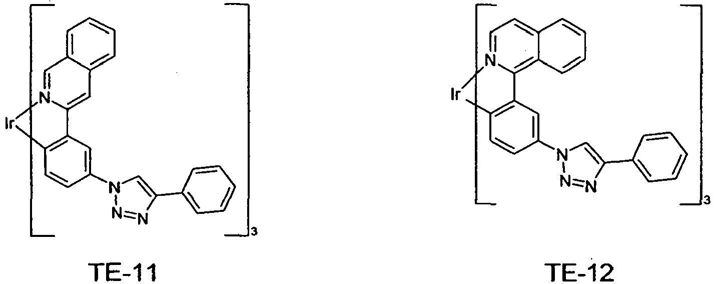

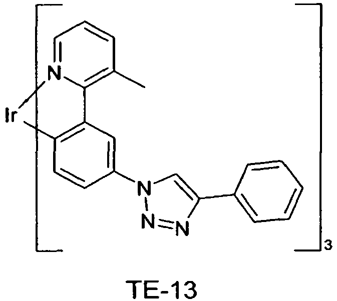

### Structures of the emitters used in the comparatives examples

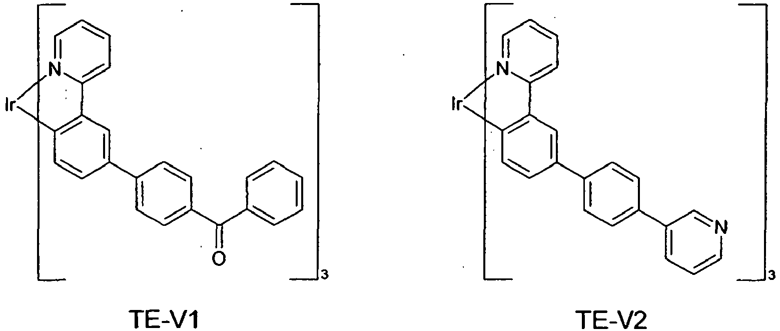

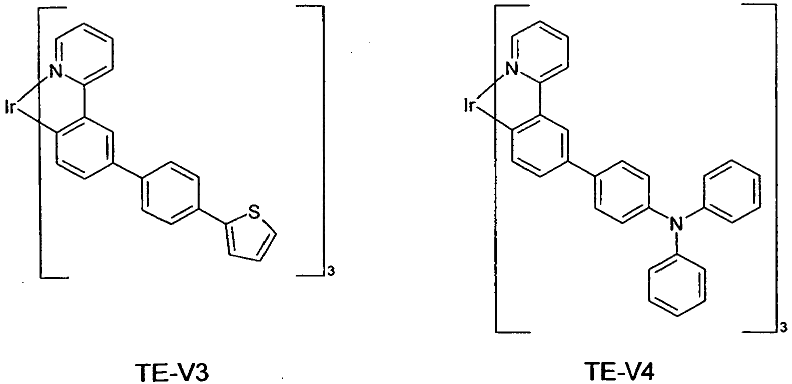

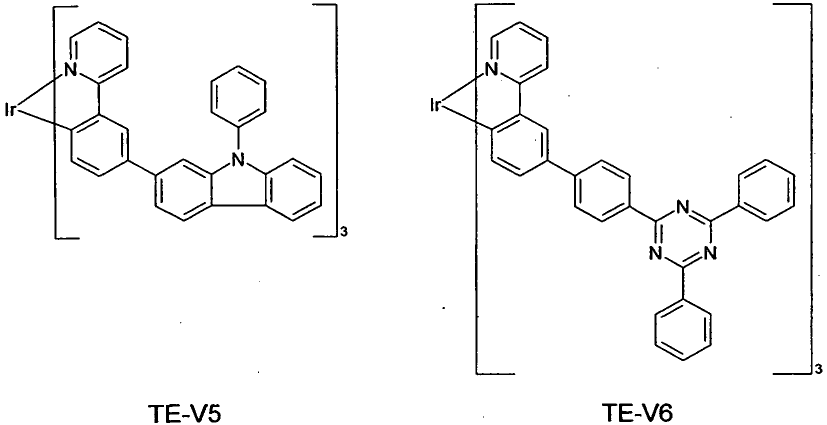

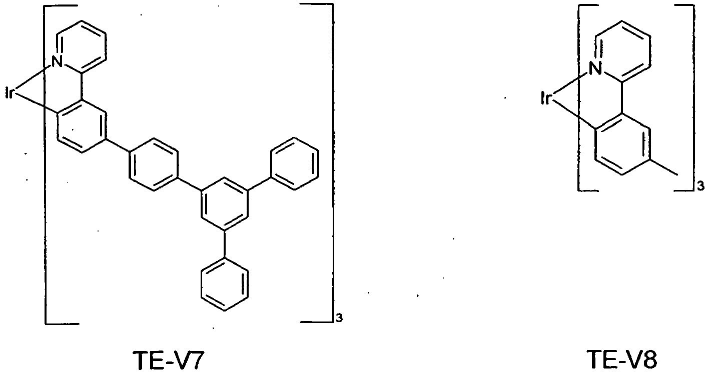

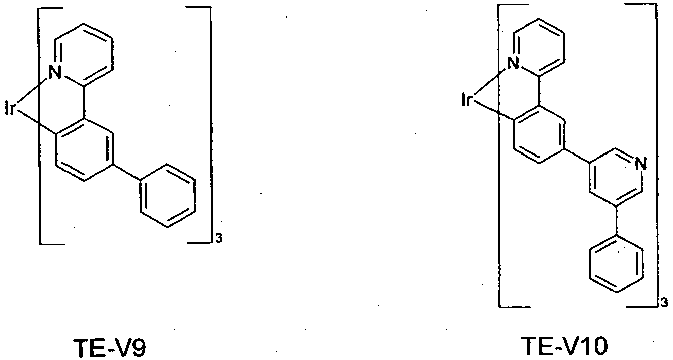

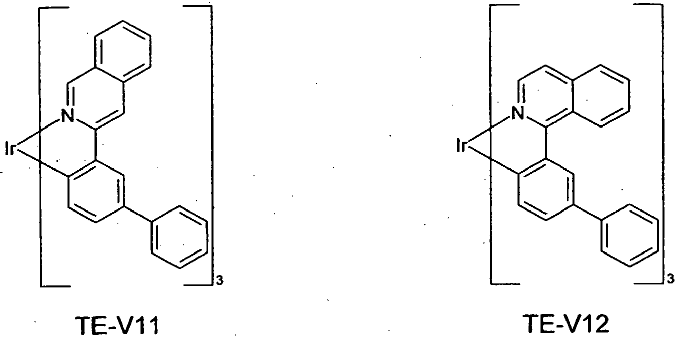

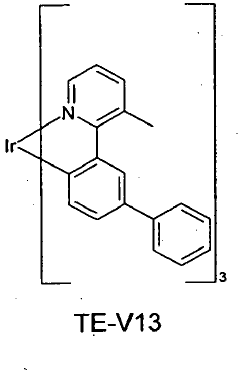

### Structures of the matrices

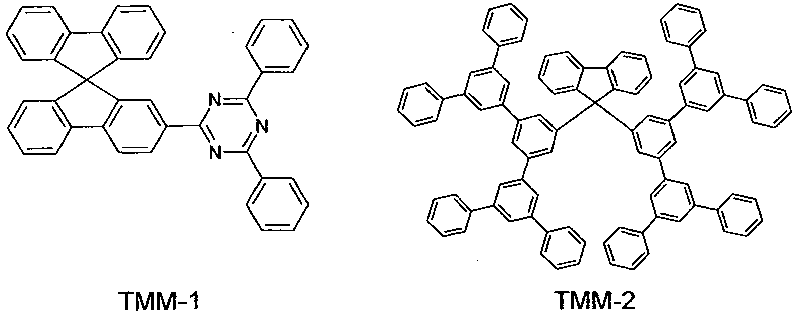

Materials according to the invention can be used from solution, where they result in simple devices having good properties. The production of such components is based on the production of polymeric light-emitting diodes (PLEDs), which has already been described a number of times in the literature (for example in WO 04/037887). In the present case, compounds TE-1 to TE-13 according to the invention are dissolved in toluene. The typical solids content of such solutions is between 16 and 25 g/l if, as here, the typical layer thickness of 80 nm for a device is to be achieved by means of spin coating. Structured ITO substrates and the material for the so-called buffer layer (PEDOT, actually PEDOT:PSS) are commercially available (ITO from Technoprint and others, PEDOT:PSS as Clevios Baytron P aqueous dispersion from H.C. Starck). The interlayer used serves for hole injection; in this case, HIL-012 from Merck was used. The emission layer is applied by spin coating in an inert-gas atmosphere, in the present case argon, and dried by heating at 160°C for 10 min. Finally, a cathode comprising barium and aluminium is applied by vacuum vapour deposition. A hole-blocking layer and/or an electron-transport layer can also be applied between the emitting layer and the cathode by vapour deposition, and the interlayer may also be replaced by one or more layers which merely have to satisfy the condition of not being detached again by the subsequent processing step of deposition of the emitting layer from solution. The devices are characterised by standard methods, and the OLED examples given have not yet been optimised. Table 1 summarises the data obtained. In the case of the processed devices, it is evident here that the materials according to the invention have superior efficiency and/or lifetime to those available previously. The OLED here exhibits the following layer structure: I) cathode (Ba/Al: 3 nm/150 nm), II) emitting layer (80 nm; 47.5% by weight of TMM-1 + 47.5% by weight of TMM-2 + 5% by weight of TE for TE-11 and TE-12 or 80 nm; 40% by weight of TMM-1 + 40% by weight of TMM-2 + 20% by weight of TE for TE-1 to TE-10 and TE-13), III) interlayer (20 nm), IV) buffer layer (80 nm; PEDOT) and V) anode.

**Table 1: Results with materials processed from solution in the device configuration indicated**

| Ex. | EML 80 nm | Max. eff. [cd/A] | Voltage [V] at 100 cd/m² | CIE (x, y) | Lifetime [h], initial luminance 1000 cd/m² |
|---|---|---|---|---|---|
| 14 | TMM-1 :TMM-2 : TE-1 | 33.0 | 5.3 | 0.33/0.63 | 30000 |
| | TMM-1 :TMM-2 : TE-V1 | 28.0 | 6.0 | 0.33/0.63 | 22000 |
| 15 | TMM-1 :TMM-2 : TE-2 | 27.5 | 5.1. | 0.32/0.63 | 29500 |
| | TMM-1 :TMM-2 : TE-V2 | 24.5 | 5.8 | 0.33/0.63 | 20500 |
| 16 | TMM-1 :TMM-2 : TE-3 | 29.0 | 5.0 | 0.32/0.64 | 32000 |
| | TMM-1 :TMM-2 : TE-V3 | 27.5 | 5.8 | 0.33/0.63 | 24500 |
| 17 | TMM-1 :TMM-2 : TE-4 | 28.5 | 5.1 | 0.33/0.62 | 31000 |
| | TMM-1 :TMM-2 : TE-V4 | 23.0 | 5.8 | 0.33/0.63 | 24000 |
| 18 | TMM-1 :TMM-2 : TE-5 | 31.0 | 5.0 | 0.34/0.63 | 26000 |
| | TMM-1 :TMM-2 : TE-V5 | 26.5 | 5.5 | 0.33/0.63 | 18300 |
| 19 | TMM-1 :TMM-2 : TE-6 | 31.5 | 4.9 | 0.32/0.63 | 29000 |
| | TMM-1 :TMM-2 : TE-V6 | 26.0 | 5.6 | 0.33/0.63 | 23000 |
| 20 | TMM-1 :TMM-2 : TE-7 | 32.0 | 4.9 | 0.33/0.64 | 32000 |
| | TMM-1 :TMM-2 : TE-V7 | 28.0 | 5.6 | 0.33/0.63 | 24000 |
| 21 | TMM-1 :TMM-2 : TE-8 | 27.5 | 5.1 | 0.34/0.62 | 28000 |
| | TMM-1 :TMM-2 : TE-V8 | 24.0 | 5.6 | 0.33/0.63 | 22000 |
| 22 | TMM-1 :TMM-2 : TE-9 | 29.0 | 4.9 | 0.33/0.63 | 31500 |
| | TMM-1 :TMM-2 : TE-V9 | 24.0 | 5.5 | 0.33/0.63 | 25500 |
| 23 | TMM-1 :TMM-2 : TE-10 | 30.5 | 5.0 | 0.32/0.63 | 32000 |
| | TMM-1 :TMM-2 : TE-V10 | 26.5 | 5.7 | 0.33/0.63 | 25500 |
| 24 | TMM-1 :TMM-2 : TE-11 | 10.5 | 5.3 | 0.64/0.36 | 20500 |
| | TMM-1 :TMM-2 : TE-V11 | 7.5 | 6.5 | 0.64/0.36 | 11000 |
| 25 | TMM-1 :TMM-2 : TE-12 | 10.0 | 5.2 | 0.64/0.36 | 18500 |
| | TMM-1 :TMM-2 : TE-V12 | 6.5 | 6.4 | 0.64/0.36 | 12000 |
| 26 | TMM-1 :TMM-2 : TE-13 | 28.5 | 4.8 | 0.35/0.68 | 39000 |
| | TMM-1 :TMM-2 : TE-V13 | 23.0 | 5.3 | 0.33/0.63 | 30000 |

## Claims

1. Compound according to Formula (1)
M(L)ₙ(L')ₘ Formula (1)
where the compound of the general Formula (1) comprises a moiety M(L)ₙ of the Formula (2) 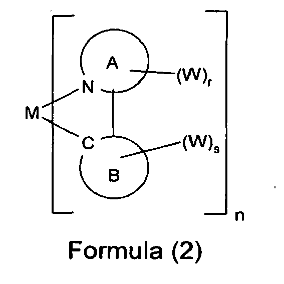 where M is bonded to any desired bidentate ligand L via a nitrogen atom N and via a carbon atom C, and
wherein A and B can be any with one or more R¹ substituted or unsubstituted aromatic or heterearomatic ring or any with one or more R¹ substituted or unsubstituted aromatic or heteroaromatic polycyclic ring system; and
wherein the symbols and indices are defined as follows:
M is a metal selected from the group consisting of iridium, rhodium, platinum and palladium, preferably iridium, platinum and palladium, particularly preferably iridium and platinum and very particularly preferably iridium;
L' is, identically or differently on each occurrence, any desired co-ligand;
W is, identically or differently on each occurrence, equal to Formula (3) 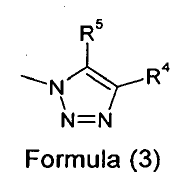
r is 0, 1, 2 or 3;
s is 0, 1, 2 or 3;
wherein the sum of r and s is at least 1, preferably 1, 2 or 3, particularly preferably 1 or 2 and very particularly preferably 1;
n is 1, 2 or 3 for M equal to iridium or rhodium and is 1 or 2 for M equal to platinum or palladium;
m is 0, 1, 2, 3 or 4;
R¹, R⁴ and R⁵ are identically or differently from each other on each occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of two or more of these groups; two or more radicals R¹, R⁴ or R⁵ here may also form a mono- or polycyclic, aliphatic, aromatic and/or benzo-fused ring system with one another;
R² is, identically or differently on each occurrence, H, D, F, Cl, Br, I, N(R³)₂, CN, NO₂, Si(R³)₃, B(OR³)₂, C(=O)R₃, P(=O)(R³)₂, S(=O)R³, S(=O)₂R³, OSO₂R³, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R³, where one or more non-adjacent CH₂ groups may be replaced by R³C=CR³, C=C, Si(R³)₂, Ge(R³)₂, Sn(R³)₂, C=O, C=S, C=Se, C=NR³, P(=O)(R³), SO, SO₂, NR³, O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 60 aromatic ring atoms, which may be substituted by one or more radicals R³, or a diarylamino group, diheteroarylamino group or arylheteroarylamino group having 10 to 40 aromatic ring atoms, which may be substituted by one or more radicals R³, or a combination of two or more of these groups; two or more adjacent radicals R² here may form a mono- or polycyclic, aliphatic or aromatic ring system with one another;
R³ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents R³ here may also form a mono- or polycyclic, aliphatic or aromatic ring system with one another;
the indices n and m here are selected so that the coordination number on the metal corresponds to 6 for M equal to iridium or rhodium and corresponds to 4 for M equal to platinum or palladium; and wherein
a plurality of ligands L here may also be linked to one another or L may be linked to L' via any desired bridge Z and thus form a tridentate, tetradentate, pentadentate or hexadentate ligand system.

2. The compound according to claim 1, **characterized in that** the compound according to Formula (1) comprises a structure M(L)ₙ having the Formula (4) 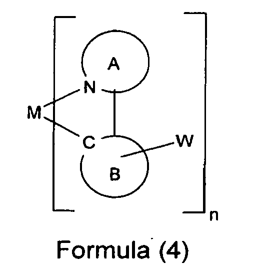 where for the symbols the definitions of claim 1 apply.

3. The compound according to claim 1 or 2, **characterized in that** the compound according to Formula (1) comprises a structure M(L)ₙ having the Formula (5b) 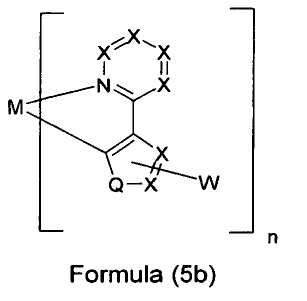 wherein
X is, identically or differently on each occurrence, CR¹ or N;
Q is, identically or differently on each occurrence, R¹C=CR¹, R¹C=N, O, S, Se or NR¹, wherein R¹ is defined as above.

4. The compound according to one or more of claims 1 to 3, **characterized in that** the compound according to Formula (1) comprises a structure M(L)ₙ having the Formula (6) 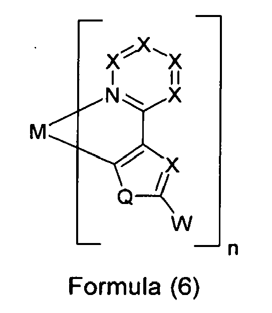 wherein the symbols are defined as above.

5. The compound according to one or more of claims 1 to 4, **characterized in that** the compound according to Formula (1) comprises a structure M(L)ₙ having the Formula selected from Formulae (9) to (19), preferably from Formulae (9), (11), (12), (13), (14), particularly preferably from Formulae (9), (11) and (12) 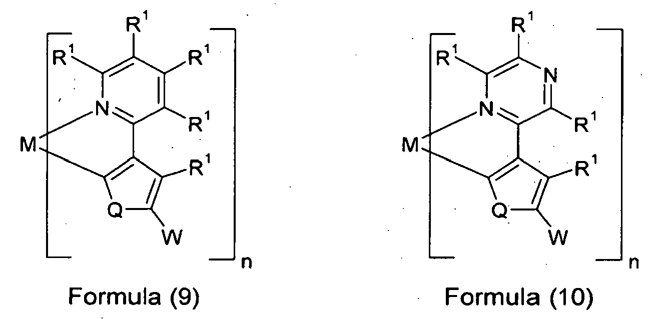 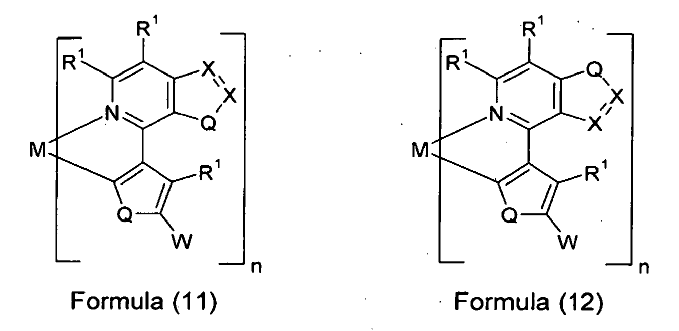 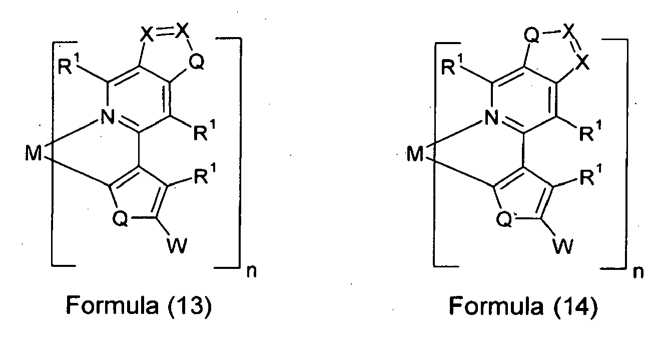 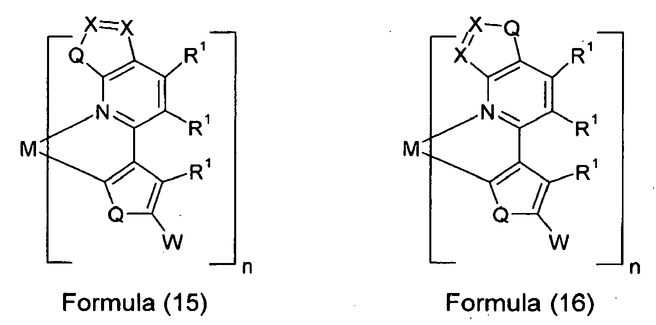 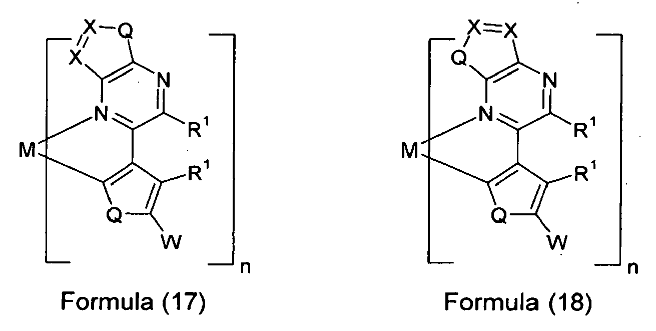 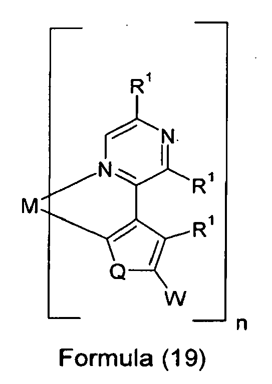 with the definitions of the symbols given above.

6. The compound according to one or more of claims 1 to 5, **characterized in that** the compound according to Formula (1) comprises a structure M(L)ₙ having the Formula selected from the following Formulae (27) to (31), preferably Formulae (27), (29), (30) and (31), particularly preferably the Formulae (27), (29) and (30).

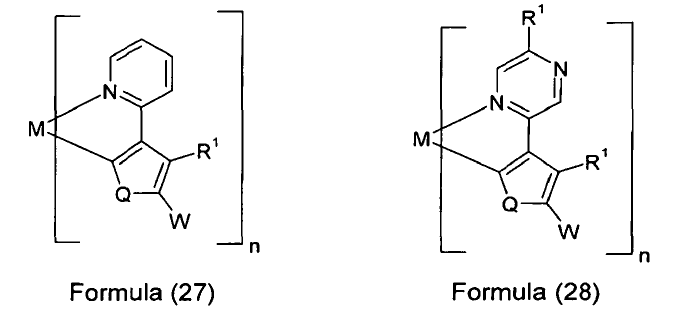

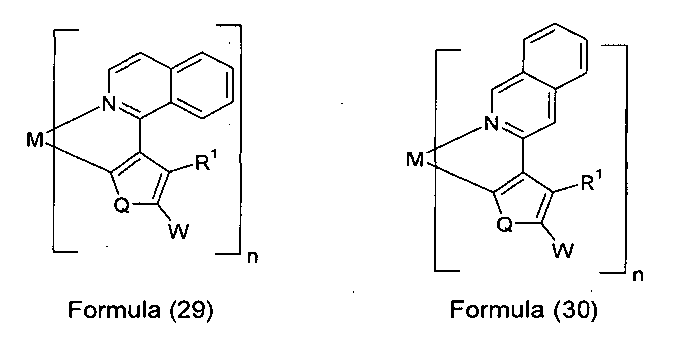

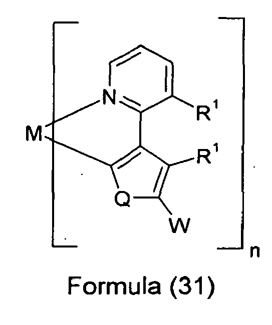

7. The compound according to one or more of claims 3 to 6, **characterized in that** Q is R¹C=CR¹, S or NR¹; particularly preferably R¹C=CR¹ or S and very particularly preferably R¹C=CR¹.

8. The compound according to one or more of claims 1 to 7, **characterized in that** m = 0.

9. The compound according to one or more of claims 1 to 8, **characterized in that** R⁵ is H.

10. The compound according to one or more of claims 1 to 9, **characterized in that** R⁴ is, identically or differently from each other on each occurrence, H, a straight-chain alkyl or alkoxy-group having 1 to 40 C-atoms or a straight-chain alkenyl or aklynyl-group having 2 to 40 C-atoms or a branched or cyclic alkyl-, alkenyl-, alkynyl- or alkoxy-group having 3 to 40 C-atoms, -NH₂, -SR³, -OR³ or an aromatic or heteroaromatic ring system having 5 to 60 aromatic ring atoms, which may in each case be substituted by one or more radicals R².

11. The compound according to one or more of claims 1 to 10, **characterized in that** R⁴ is, identically or differently from each other on each occurrence, H, a straight-chain alkyl or alkoxy-group having 1 to 40 C-atoms or a straight-chain alkenyl or aklynyl-group having 2 to 40 C-atoms or a branched or cyclic alkyl-, alkenyl-, alkynyl- or alkoxy-group having 3 to 40 C-atoms, -NH₂, -SR³, -OR³ or a group selected from the following Formulae (32) to (46). 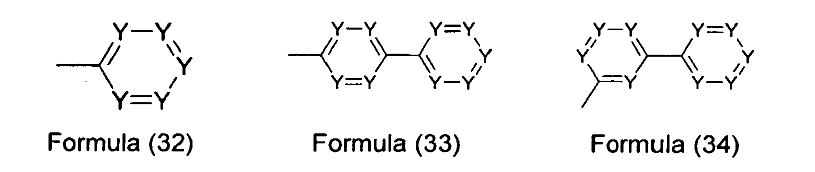 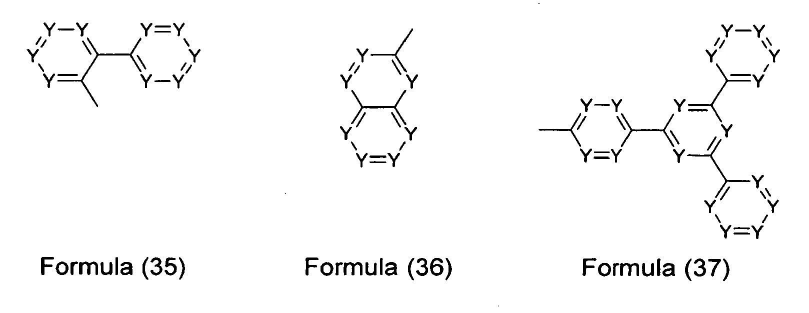 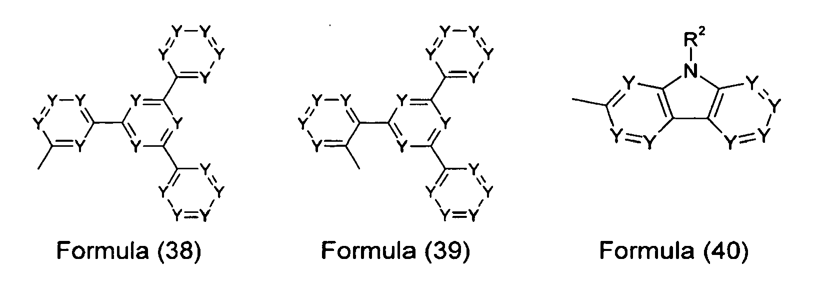 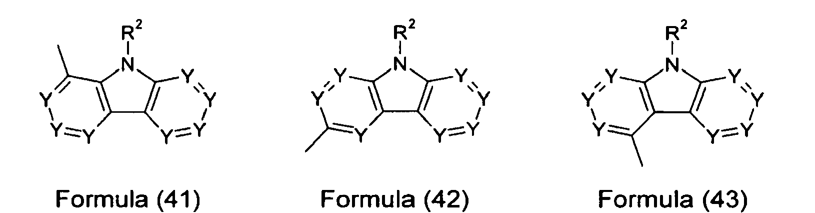 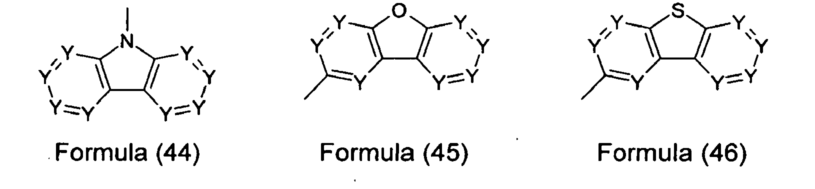 wherein Y is, identically or differently from each other on each occurrence, CR², N, P, or PR²₂, preferably CR² or N, wherein R² and R³ are defined as above.

12. The compound according to one or more of claims 1 to 10, **characterized in that** R⁴ is, identically or differently from each other on each occurrence selected from Formulae (47) to (220)) wherein the rest having the Formulae (47) to (220) can be, identically or differently from each other on each occurrence, further substituted with one or more -CN or R³ and wherein R³ is defined as above

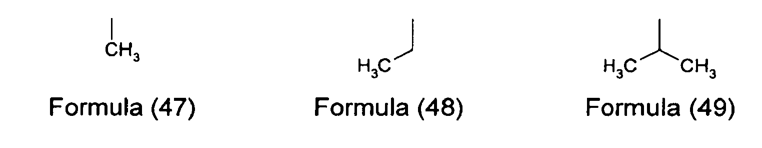

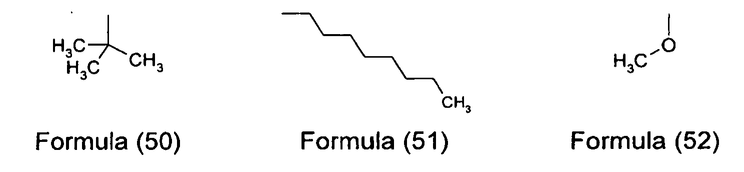

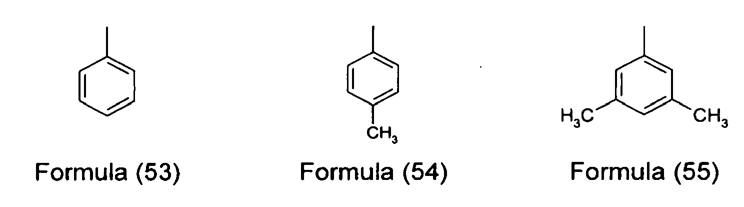

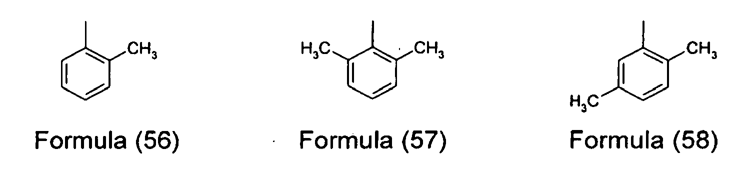

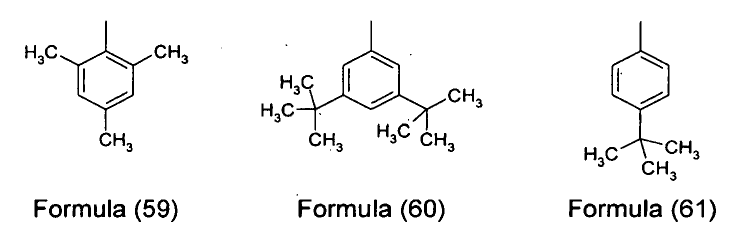

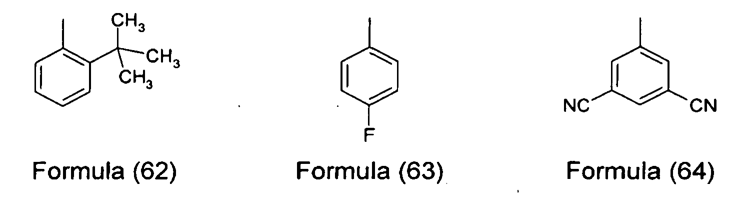

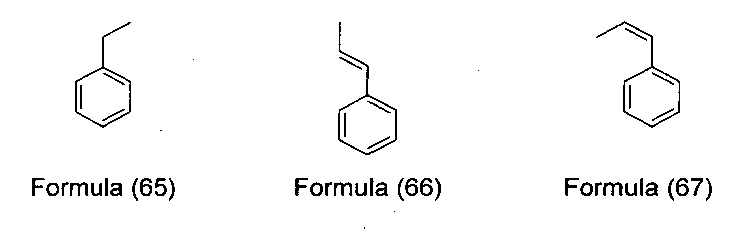

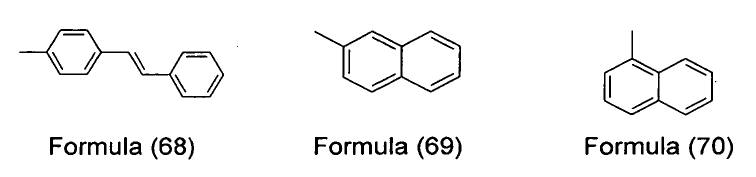

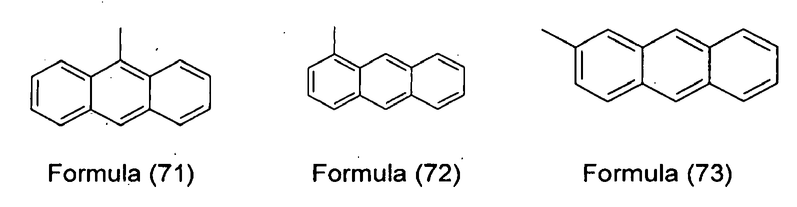

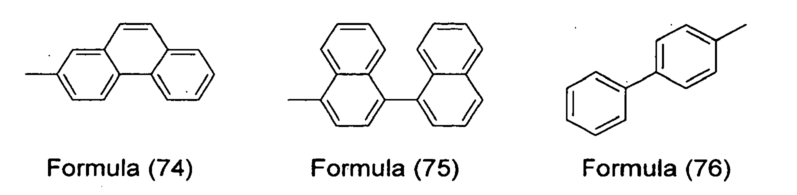

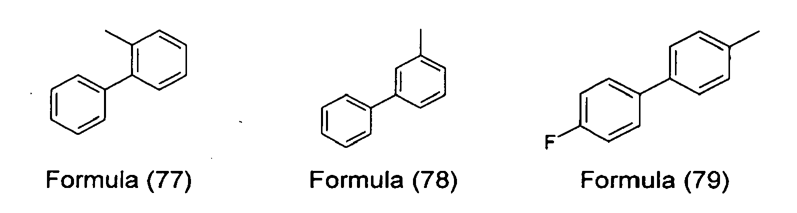

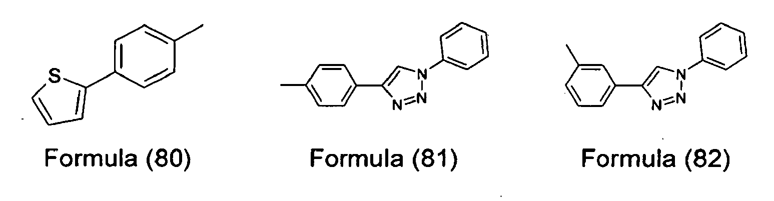

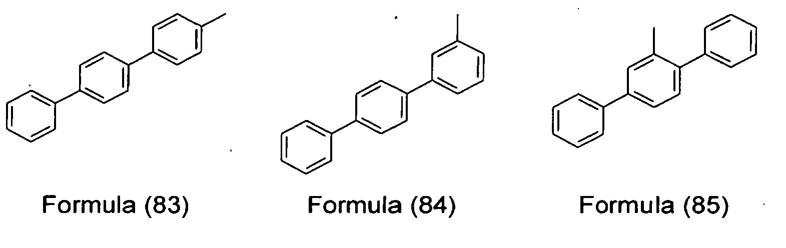

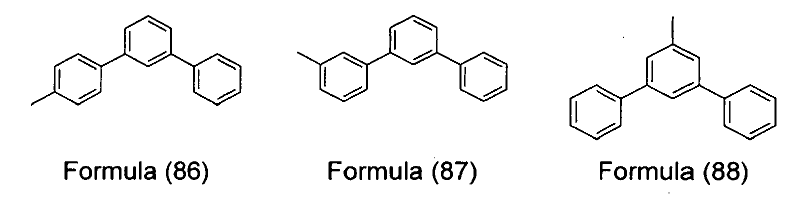

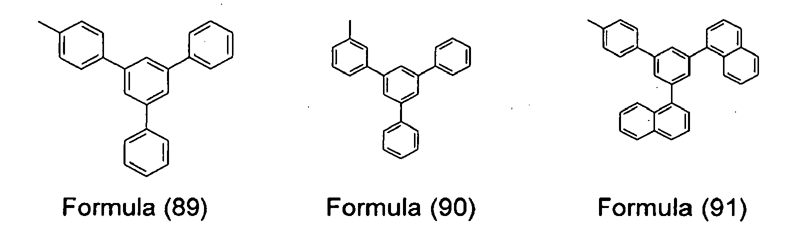

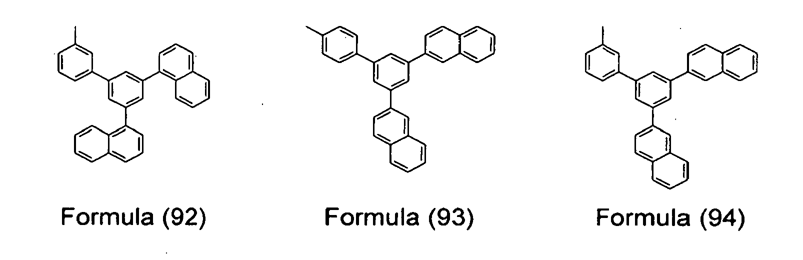

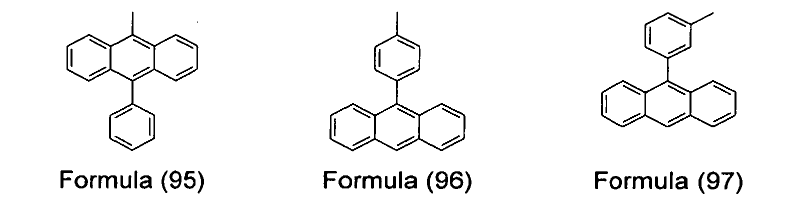

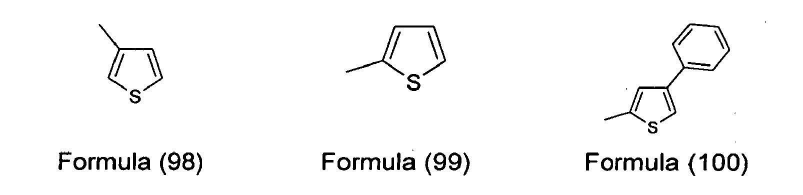

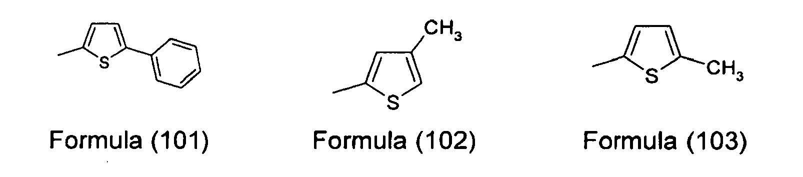

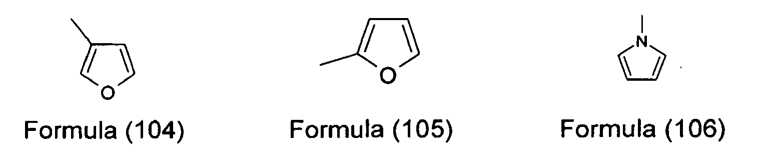

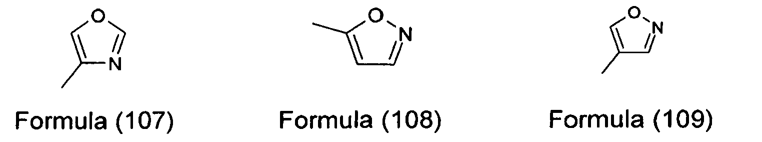

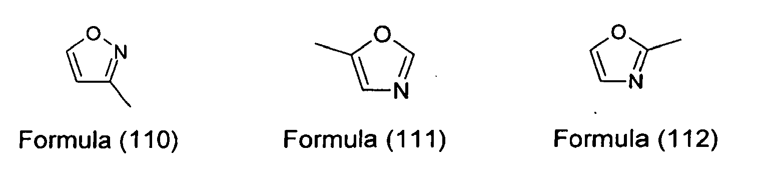

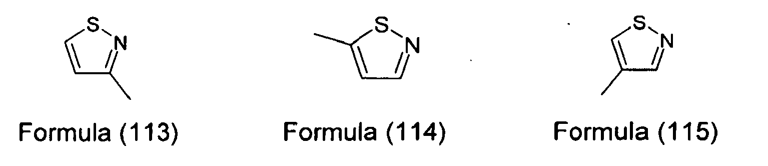

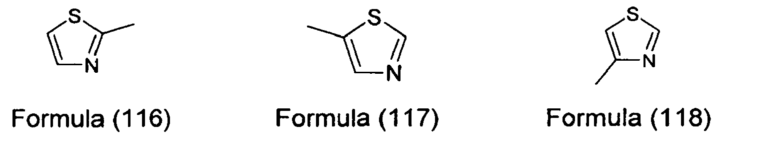

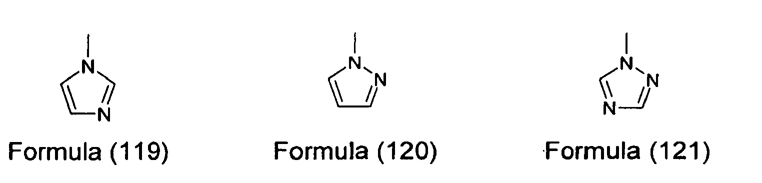

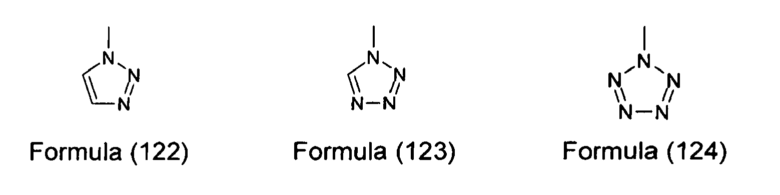

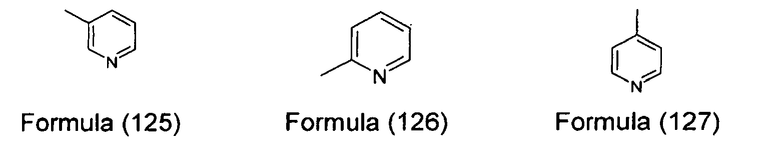

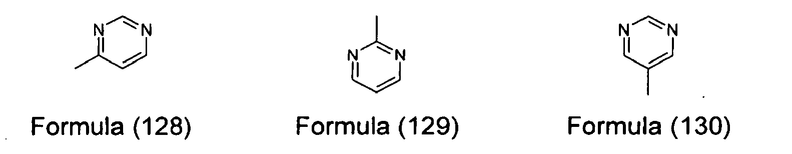

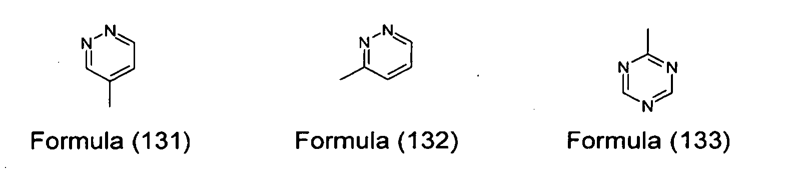

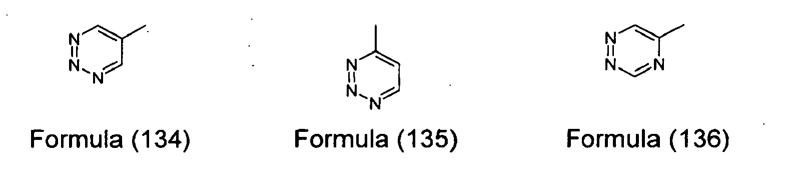

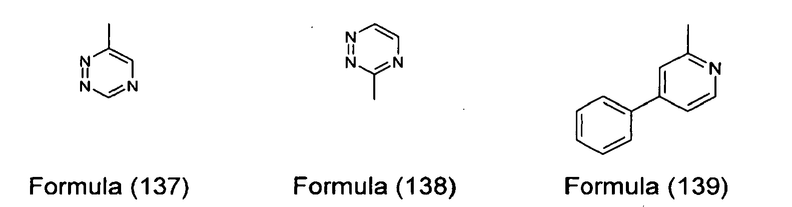

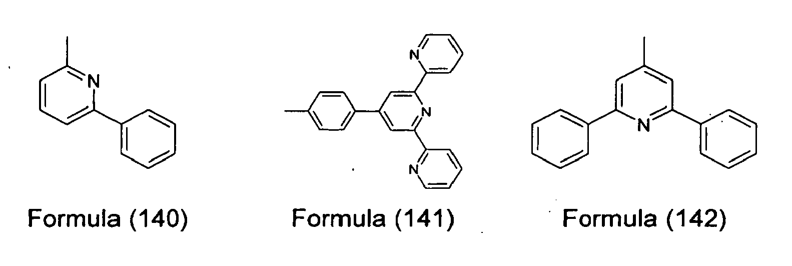

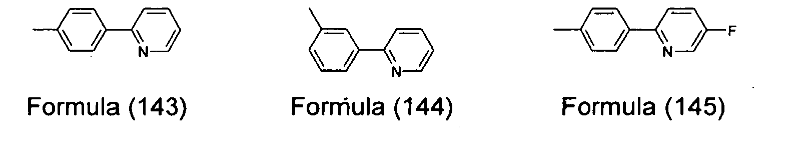

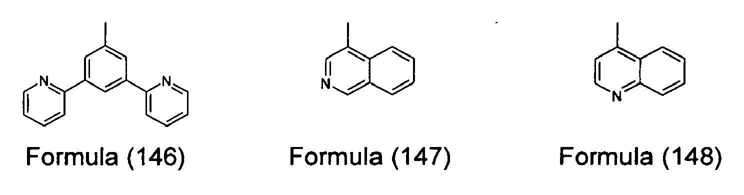

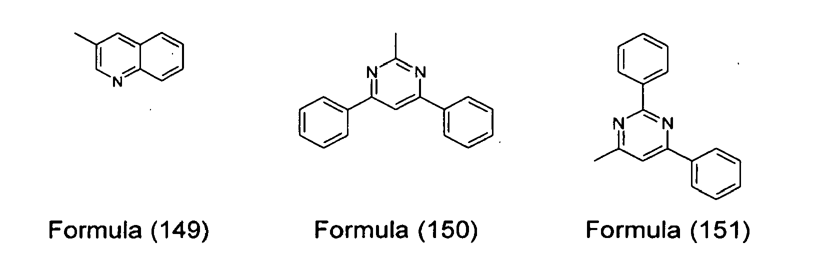

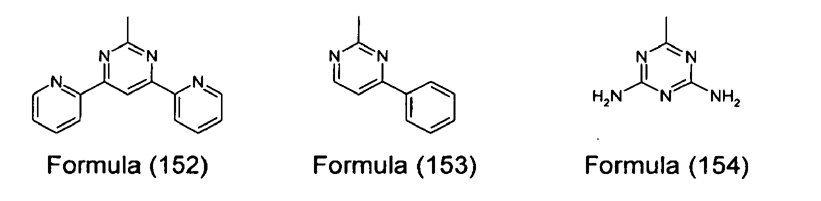

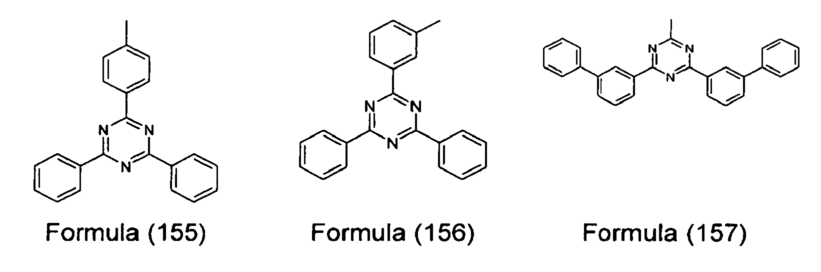

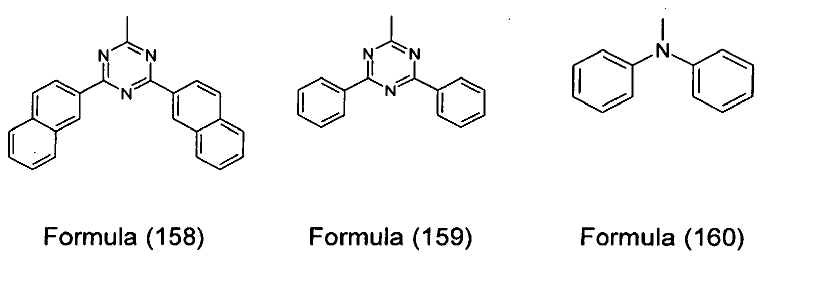

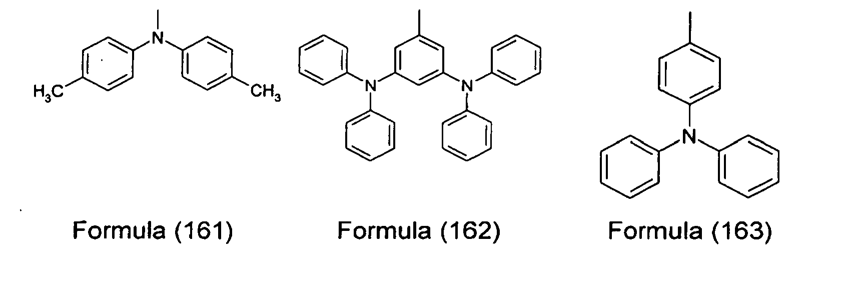

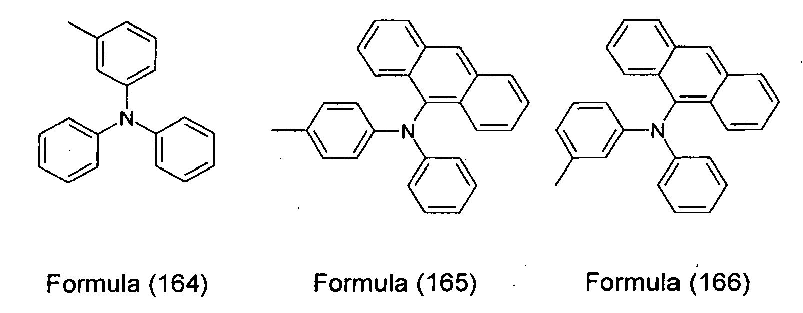

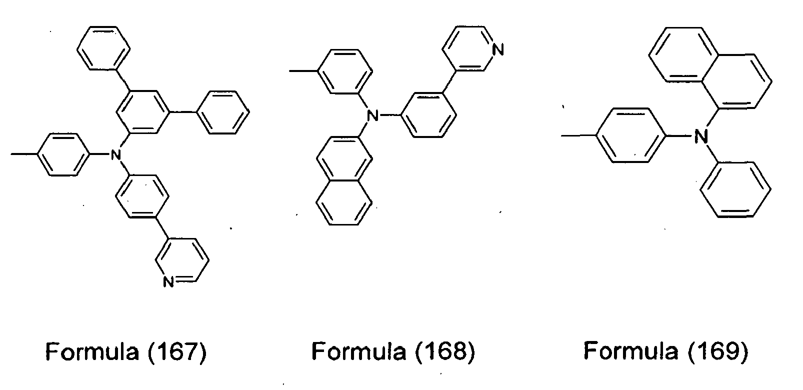

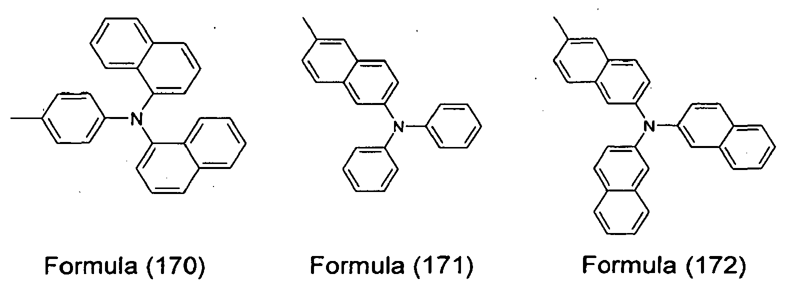

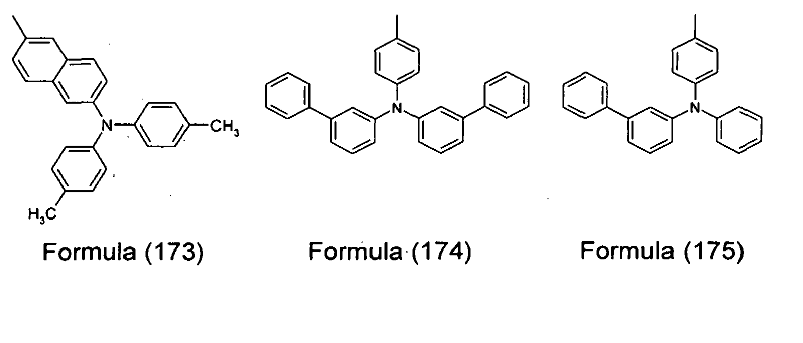

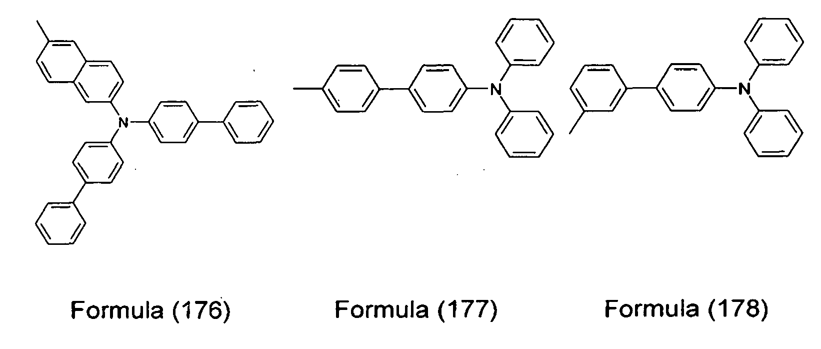

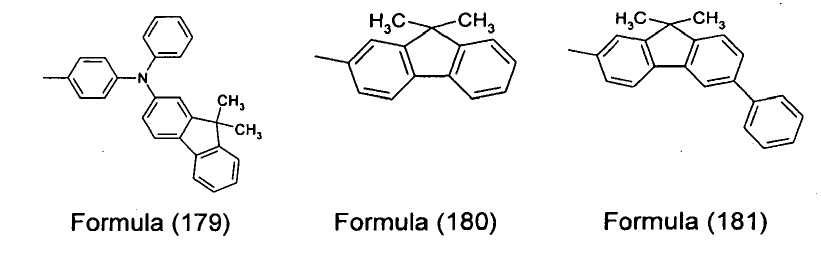

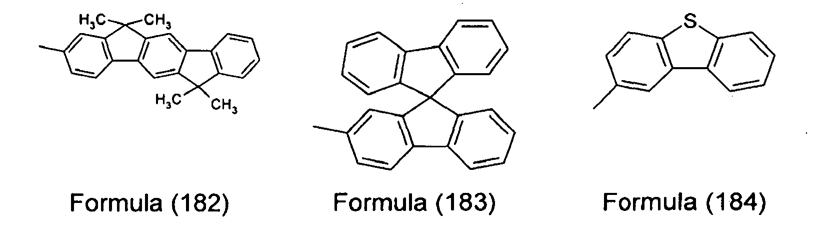

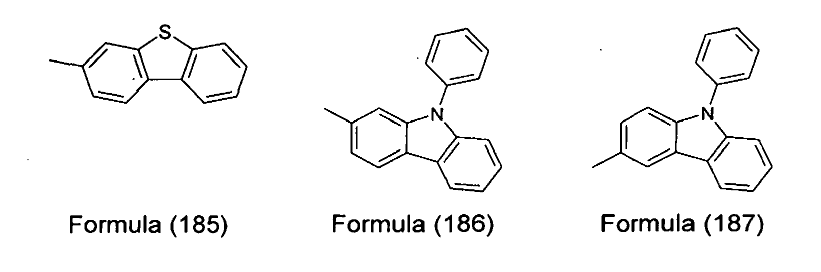

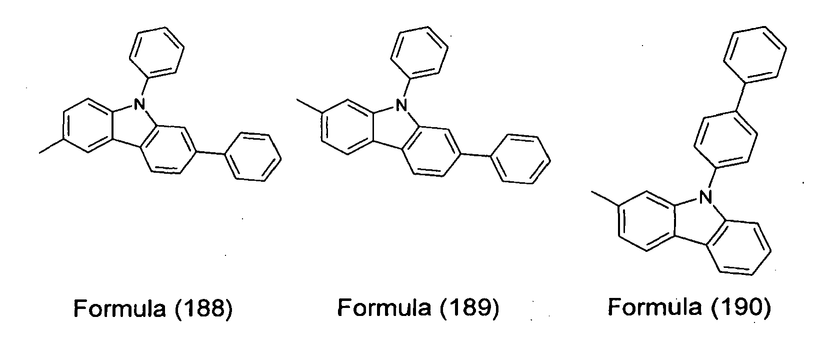

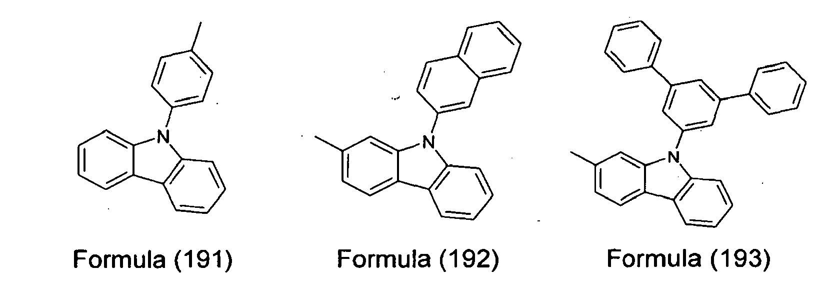

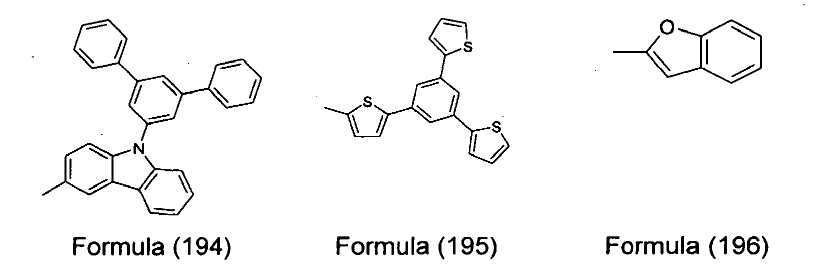

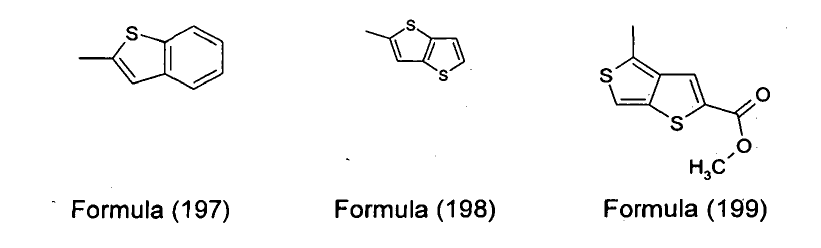

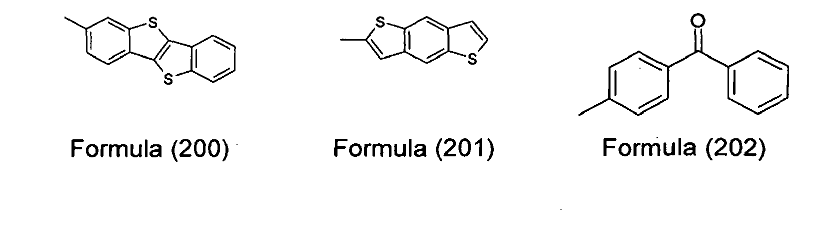

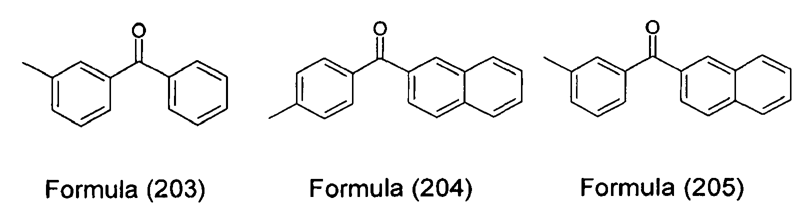

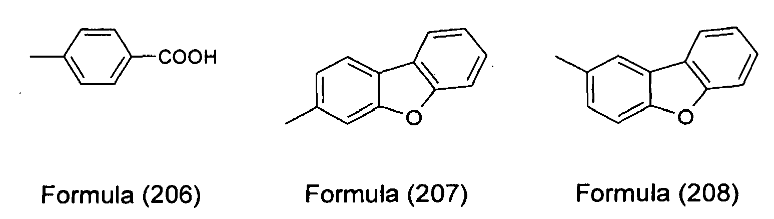

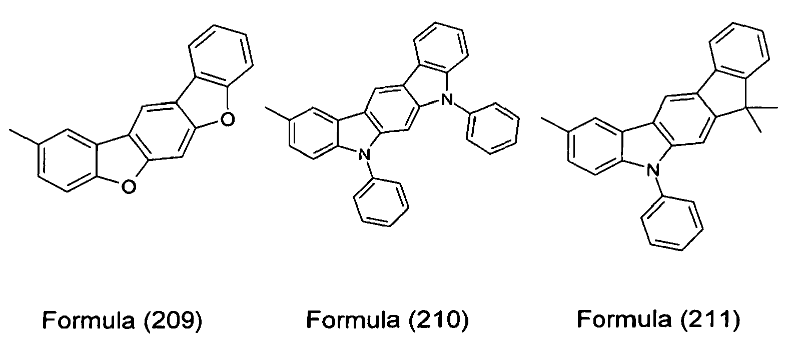

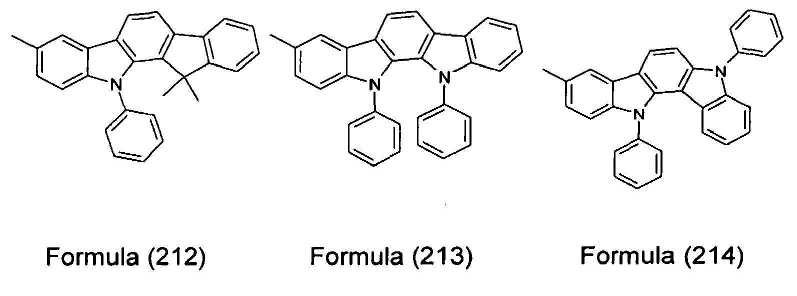

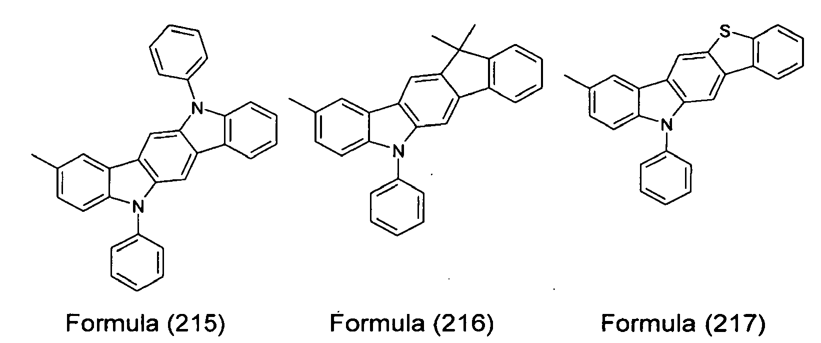

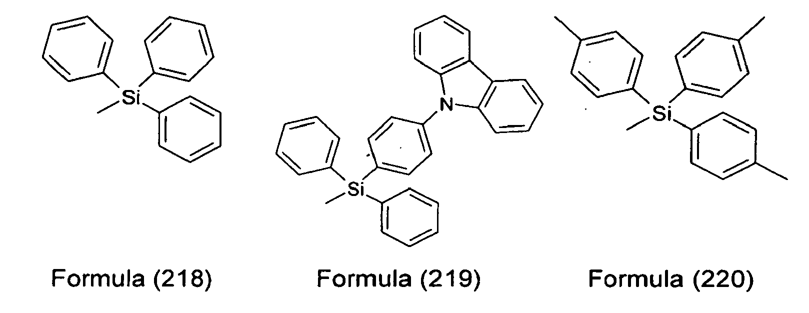

13. Method for the preparation of a compound according to one or more of claims 1 to 12 involving the reaction of A' and B' in order to yield C', wherein the symbols and indices are defined as above

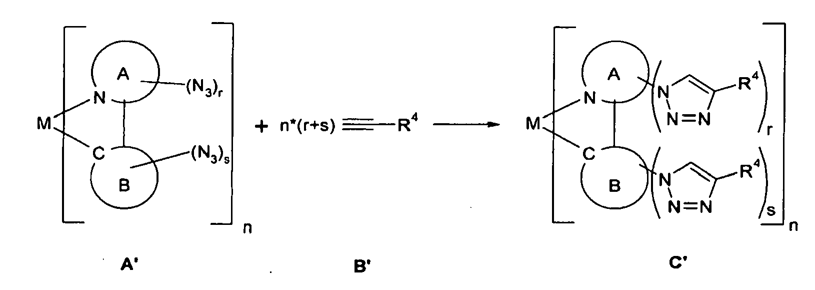

14. Composition comprising at least one compound according to one or more of claims 1 to 12 and at least one organic functional material selected from hole transport materials (HTM), hole injection materials (HIM), electron transport materials (ETM), electron injection materials (EIM), hole blocking materials (HBM), exciton blocking materials (ExBM), host or matrix materials, fluorescent emitter, phosphorescent emitter.

15. Composition according to claim 14, **characterized in that** the at least one organic functional material is a matrix material preferably selected from ketones, phosphinoxides, sulfoxides, sulfones, triarylamines, carbazoles, indolocarbazoles, indenocarbazoles, azacarbazoles, bipolar matrix materials, silanes, azaborolenes, boronesters, triazines, zinc complexes, diaza- or tetraazasiloles or diazaphospholes or mixtures thereof.

16. Formulation comprising at least one compound according to one or more of claim 1 to 12 or a composition according to claim 14 or 15 and at least one solvent.

17. Electronic device comprising at least one compound according to one or more of claims 1 to 12 or a composition according to claim 14 or 15, preferably a device selected from organic electroluminescent devices (OLEDs - organic light emitting diodes, PLEDs - polymer light emitting diodes), organic integrated cicuits (O-ICs), organic field effect transistors (O-FETs), organic thin film transistors (O-TFTs), organic light emitting transistors (O-LETs), organic solar cells (O-SCs), organic optical detectors, organic photoreceptors, organic field quenching devices (O-FQDs), light emitting electrochemical cells (LECs, OLECs, LEECs) or organic laser diodes (O-Laser), particularly preferably an OLED or organic light emitting cell (OLEC).

18. Device according to claim 17, **characterized in that** it comprises the composition according to claim 15 in one or more light emitting layers.

19. Electroluminescent device, which is preferably an OLED or OLEC, comprising at least one compound according to one or more of claims 1 to 12 or a composition according to claim 14 or 15 for use for phototherapy in medicine.

20. Cosmetic use of an electroluminescent device, which is preferably an OLED or OLEC, comprising at least one compound according to one or more of claims 1 to 12 or a composition according to claim 14 or 15.

## Patentansprüche

1. Verbindung gemäß Formel (1)
M(L)ₙ(L')ₘ Formel (1)
wobei die Verbindung der allgemeinen Formel (1) einen Molekülteil M(L)ₙ der Formel (2) 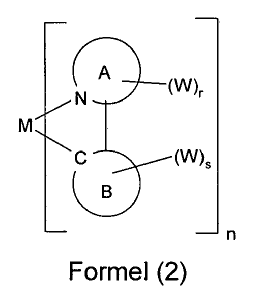 enthält, wobei M über ein Stickstoffatom N sowie über ein Kohlenstoffatom C an einen beliebigen gewünschten zweizähnigen Liganden L gebunden ist, und
in der A und B einen beliebigen mit einem oder mehreren R¹ substituierten oder unsubstituierten aromatischen oder heteroaromatischen Ring oder ein beliebiges mit einem oder mehreren R¹ substituiertes oder unsubstituiertes aromatisches oder heteroaromatisches polycyclisches Ringsystem bedeuten können; und
in der die Symbole und Indizes wie folgt definiert sind:
M bedeutet ein Metall, das aus der Gruppe bestehend aus Iridium, Rhodium, Platin und Palladium, vorzugsweise Iridium, Platin und Palladium, besonders bevorzugt Iridium und Platin und ganz besonders bevorzugt Iridium ausgewählt ist;
L' bedeutet bei jedem Auftreten gleich oder verschieden einen beliebigen gewünschten Coliganden;
W ist bei jedem Auftreten gleich oder verschieden gleich der Formel (3 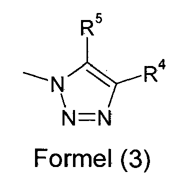
r steht für 0, 1, 2 oder 3;
s steht für 0, 1, 2 oder 3;
in der die Summe von r und s mindestens 1, vorzugsweise 1, 2 oder 3, besonders bevorzugt 1 oder 2 und ganz besonders bevorzugt 1 beträgt;
n steht für 1, 2 oder 3 für M gleich Iridium oder Rhodium und für 1 oder 2 für M gleich Platin oder Palladium;
m steht für 0, 1, 2, 3 oder 4;
R¹ R⁴ und R⁵ bedeuten bei jedem Auftreten gleich oder verschieden voneinander H, D, F, Cl, Br, I, N(R²)₂, CN, NO₂, Si(R²)₃, B(OR²)₂, C(=O)R², P(=O)(R²)₂, S(=_{O})R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils durch einen oder mehrere Reste R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, die durch einen oder mehrere Reste R² substituiert sein kann, oder eine Kombination von zwei oder mehr dieser Gruppen; dabei können zwei oder mehr Reste R¹, R⁴ oder R⁵ auch ein mono- oder polycyclisches, aliphatisches, aromatisches und/oder benzoanelliertes Ringsystem miteinander bilden;
R² bedeutet bei jedem Auftreten gleich oder verschieden H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils durch einen oder mehrere Reste R³ substituiert sein kann, wobei eine oder mehrere nicht benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, die durch einen oder mehrere Reste R³ substituiert sein kann, oder eine Kombination von zwei oder mehr dieser Gruppen; dabei können zwei oder mehr benachbarte Reste R² auch ein mono- oder polycyclisches, aliphatisches oder aromatisches Ringsystem miteinander bilden;
R³ bedeutet bei jedem Auftreten gleich oder verschieden H, D, F oder einen aliphatischen, aromatischen und/oder heteroaromatischen Kohlenwasserstoffrest mit 1 bis 20 C-Atomen, in dem zusätzlich ein oder mehrere H-Atome durch F ersetzt sein können; dabei können zwei oder mehr Substituenten R³ auch ein mono- oder polycyclisches, aliphatisches oder aromatisches Ringsystem miteinander bilden;
dabei sind die Indizes n und m so gewählt, dass die Koordinationszahl am Metall für M gleich Iridium oder Rhodium 6 entspricht und für M gleich Platin oder Palladium 4 entspricht; und in der
dabei auch mehrere Liganden L miteinander verknüpft sein können oder L über eine beliebige gewünschte Brücke Z mit L' verknüpft sein kann und so ein dreizähniges, vierzähniges, fünfzähniges oder sechszähniges Ligandensystem bilden können.

2. Verbindung nach Anspruch 1, **dadurch gekennzeichnet, dass** die Verbindung gemäß Formel (1) eine Struktur M(L)ₙ mit der Formel (4) 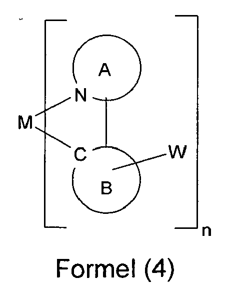 enthält, wobei für die Symbole die Definitionen aus Anspruch 1 gelten.

3. Verbindung nach Anspruch 1 oder 2, **dadurch gekennzeichnet, dass** die Verbindung gemäß Formel (1) eine Struktur M(L)ₙ mit der Formel (5b) 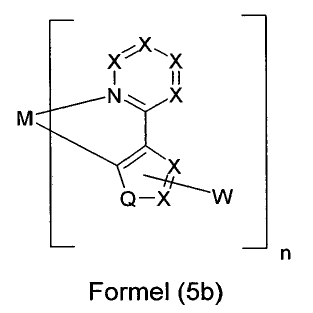 enthält, in der
X bei jedem Auftreten gleich oder verschieden CR¹ oder N bedeutet;
Q bei jedem Auftreten gleich oder verschieden R¹C=CR¹, R¹C=N, O, S, Se oder NR¹ bedeutet, in denen R¹ wie vorstehend definiert ist.

4. Verbindung nach einem oder mehreren der Ansprüche 1 bis 3, **dadurch gekennzeichnet, dass** die Verbindung gemäß Formel (1) eine Struktur M(L)ₙ mit der Formel (6) 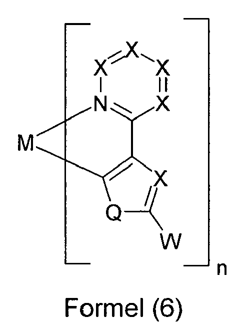 enthält, in der die Symbole wie vorstehend definiert sind.

5. Verbindung nach einem oder mehreren der Ansprüche 1 bis 4, **dadurch gekennzeichnet, dass** die Verbindung gemäß Formel (1) eine Struktur M(L)ₙ mit der Formel enthält, die aus den Formeln (9) bis (19), bevorzugt aus den Formeln (9), (11), (12), (13), (14), besonders bevorzugt aus den Formeln (9), (11) und (12) 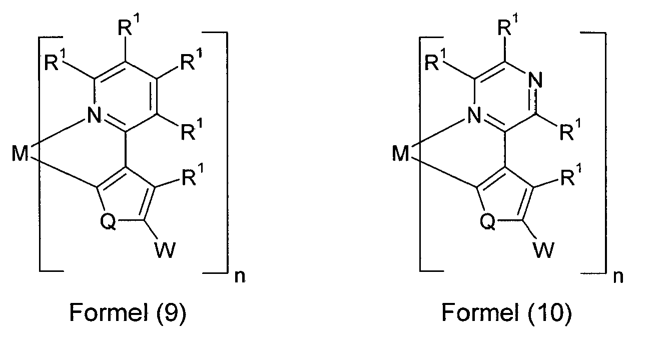 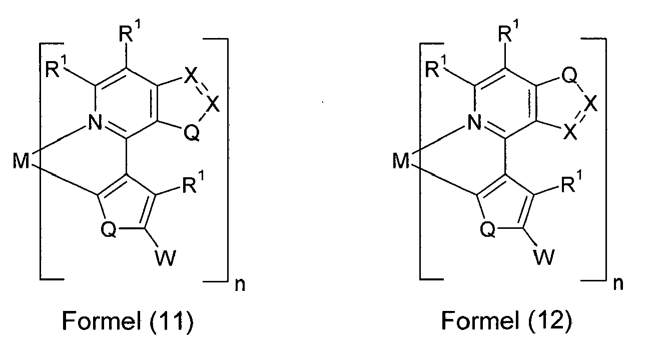 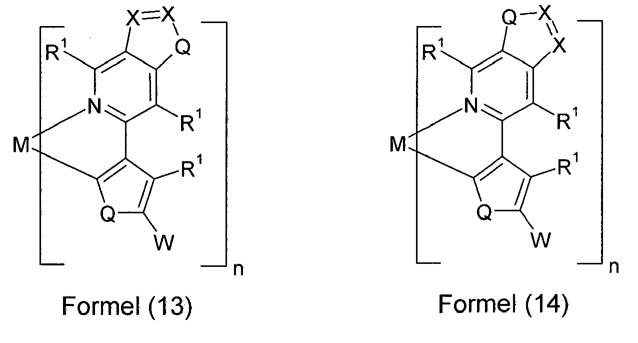 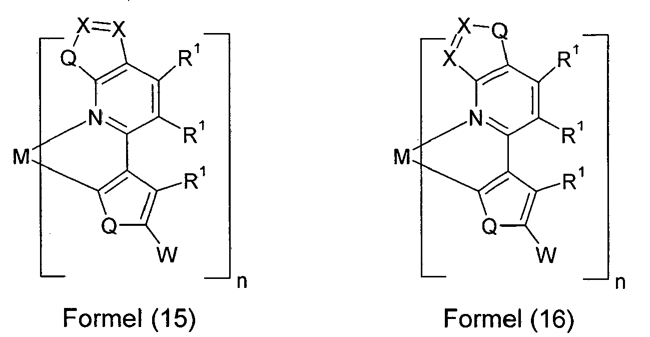 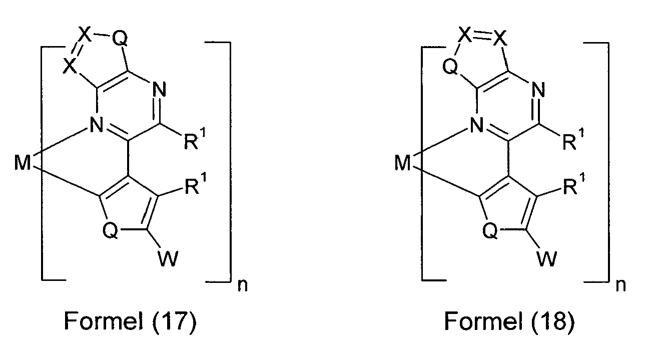 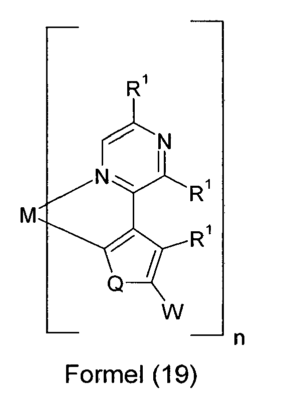 ausgewählt ist, wobei die Definitionen der Symbole vorstehend angegeben sind.

6. Verbindung nach einem oder mehreren der Ansprüche 1 bis 5, **dadurch gekennzeichnet, dass** die Verbindung gemäß Formel (1) eine Struktur M(L)ₙ mit der Formel enthält, die aus den folgenden Formeln (27) bis (31), bevorzugt den Formeln (27), (29), (30) und (31), besonders bevorzugt den Formeln (27), (29) und (30) 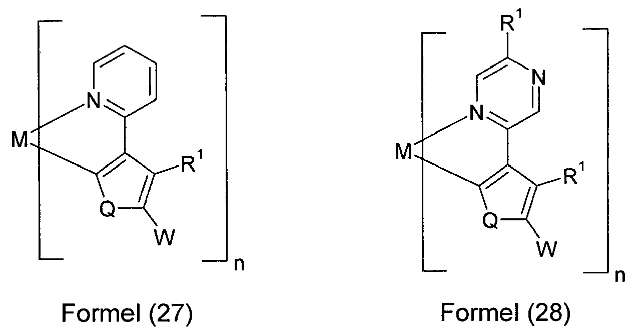 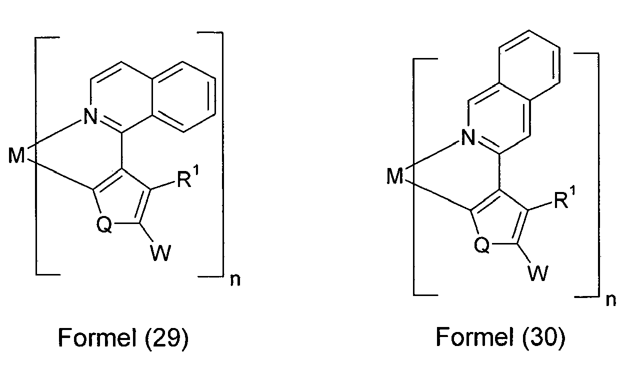 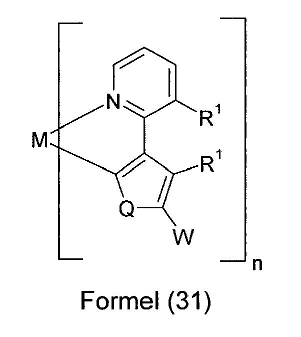 ausgewählt ist.

7. Verbindung nach einem oder mehreren der Ansprüche 3 bis 6, **dadurch gekennzeichnet, dass** Q R¹C=CR¹, S oder NR¹, besonders bevorzugt R¹C=CR¹ oder S und ganz besonders bevorzugt R¹C=CR¹ bedeutet.

8. Verbindung nach einem oder mehreren der Ansprüche 1 bis 7, **dadurch gekennzeichnet, dass** m = 0.

9. Verbindung nach einem oder mehreren der Ansprüche 1 bis 8, **dadurch gekennzeichnet, dass** R⁵ H bedeutet.

10. Verbindung nach einem oder mehreren der Ansprüche 1 bis 9, **dadurch gekennzeichnet, dass** R⁴ bei jedem Auftreten gleich oder verschieden voneinander H, eine geradkettige Alkyl- oder Alkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl- oder Alkoxygruppe mit 3 bis 40 C-Atomen, -NH₂, -SR³, -OR³ oder ein aromatisches oder heteroaromatisches Ringsystem mit 5 bis 60 aromatischen Ringatomen bedeutet, das jeweils durch einen oder mehrere Reste R² substituiert sein kann.

11. Verbindung nach einem oder mehreren der Ansprüche 1 bis 10, **dadurch gekennzeichnet, dass** R⁴ bei jedem Auftreten gleich oder verschieden voneinander H, eine geradkettige Alkyl- oder Alkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl- oder Alkoxygruppe mit 3 bis 40 C-Atomen, -NH₂, -SR³, -OR³ oder eine Gruppe ausgewählt aus den folgenden Formeln (32) bis (46) 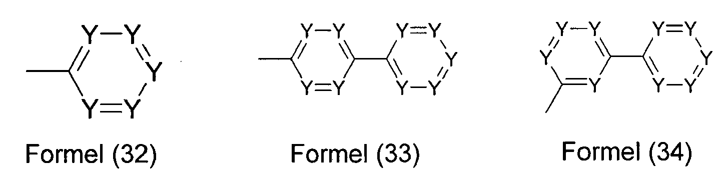 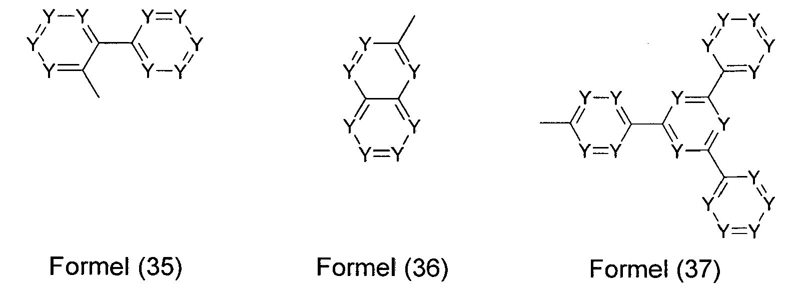 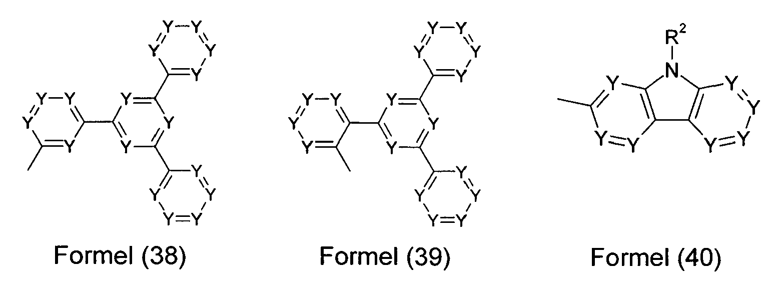 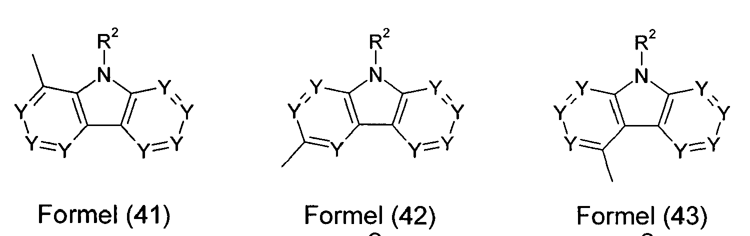 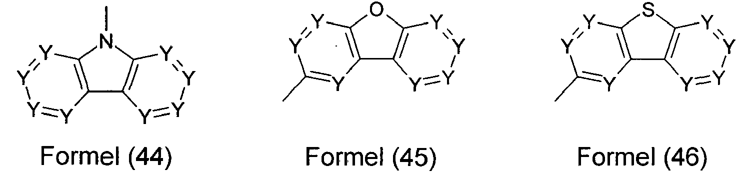 bedeutet, in denen Y bei jedem Auftreten gleich oder verschieden voneinander CR², N, P oder PR²₂, vorzugsweise CR² oder N bedeutet, in denen R² und R³ wie vorstehend definiert sind.

12. Verbindung nach einem oder mehreren der Ansprüche 1 bis 10, **dadurch gekennzeichnet, dass** R⁴ bei jedem Auftreten gleich oder verschieden voneinander aus den Formeln (47) bis (220) ausgewählt ist, in denen der Rest mit den Formeln (47) bis (220) bei jedem Auftreten gleich oder verschieden voneinander weiter mit einem oder mehreren -CN oder R³ substituiert sein kann und in denen R³ wie vorstehend definiert ist

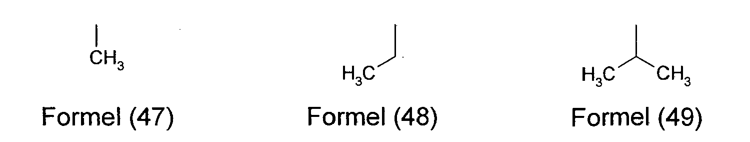

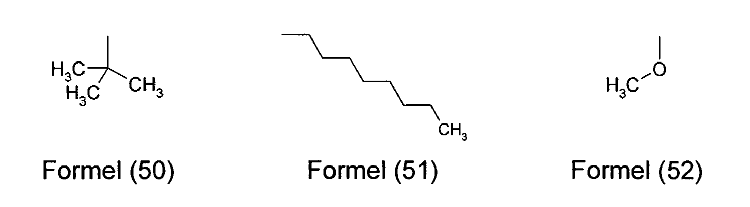

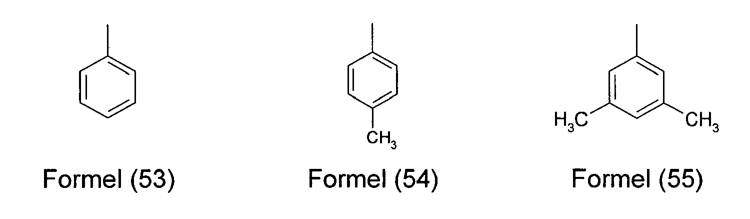

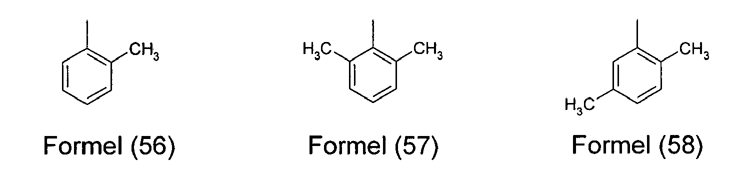

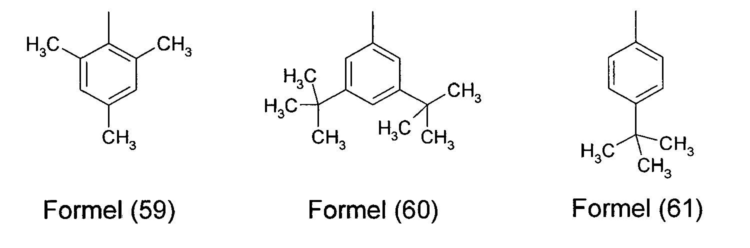

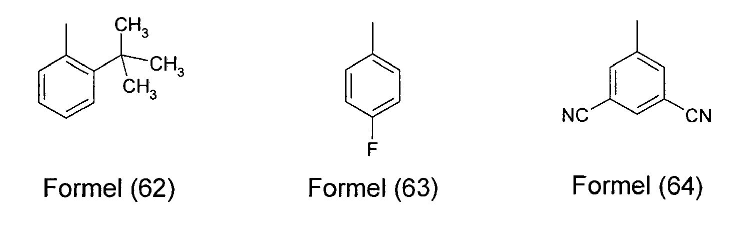

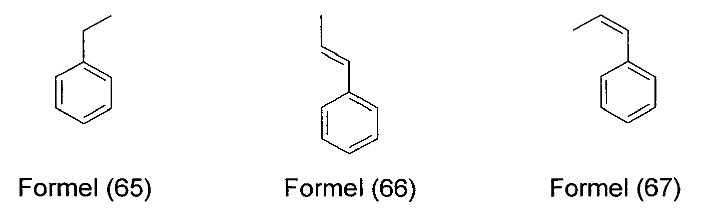

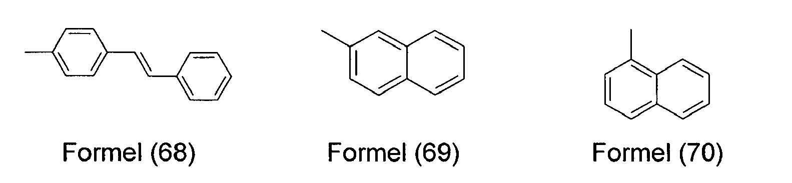

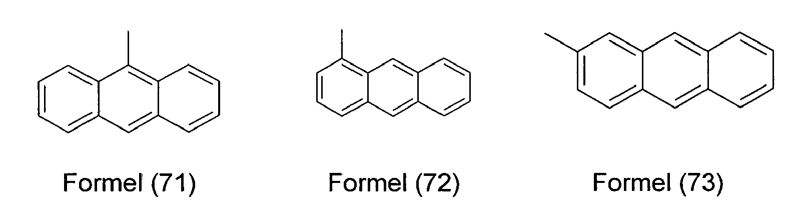

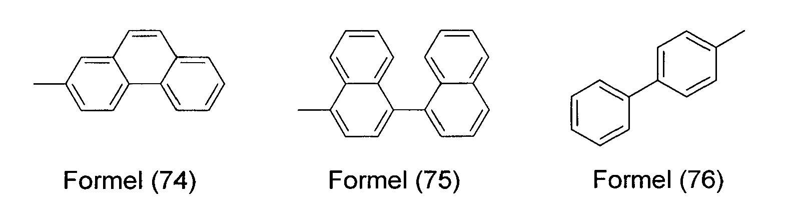

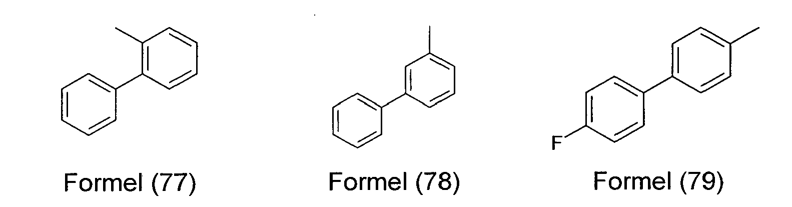

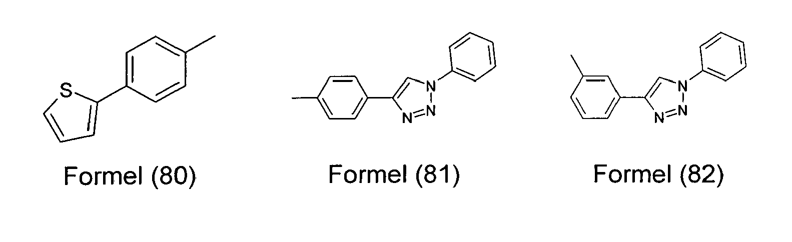

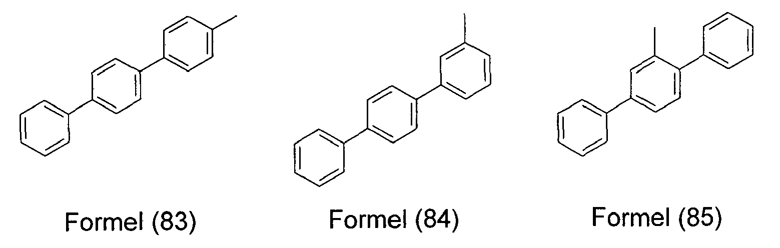

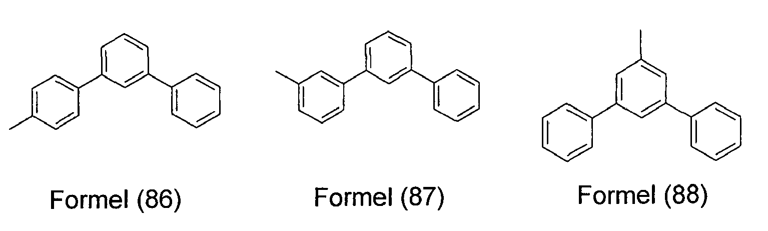

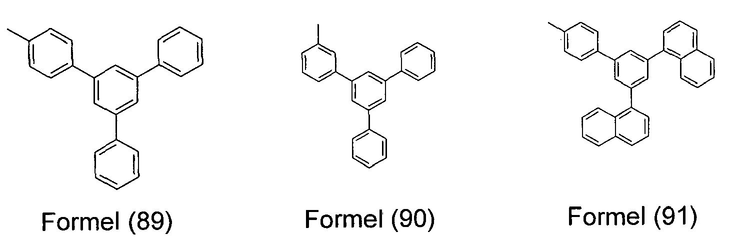

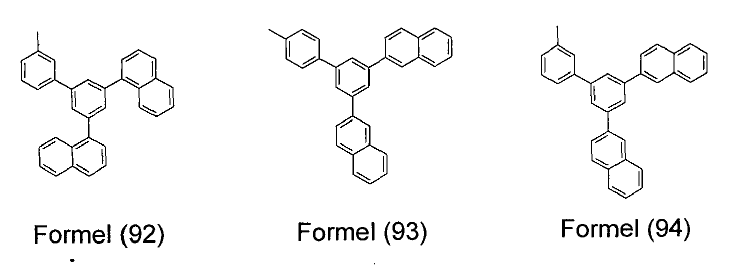

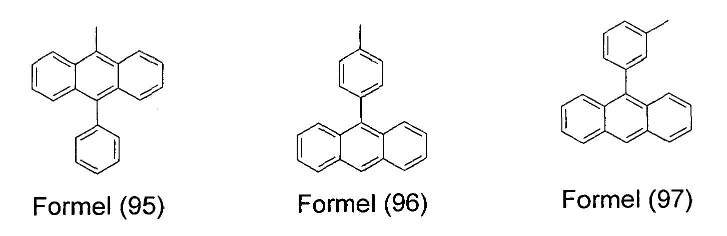

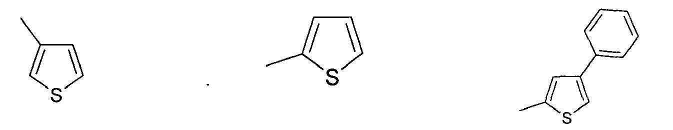

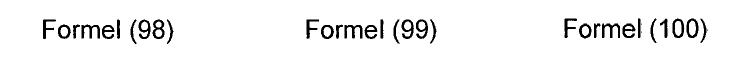

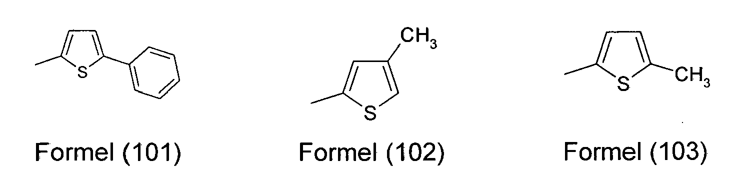

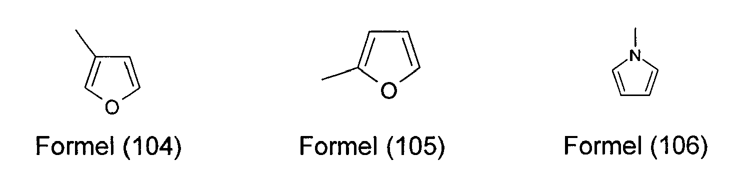

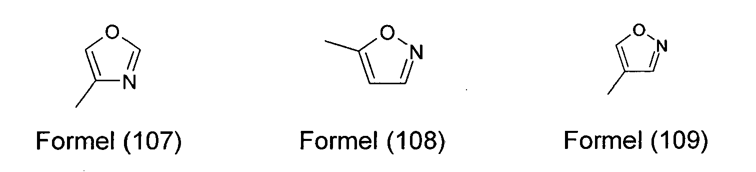

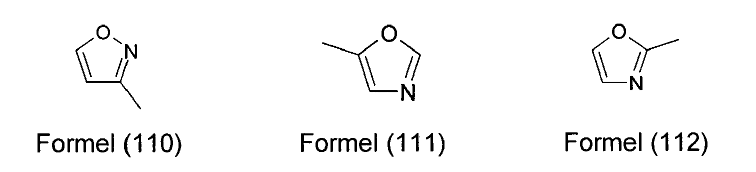

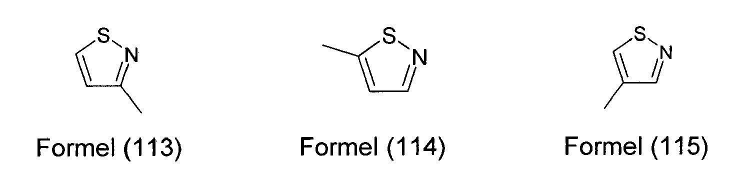

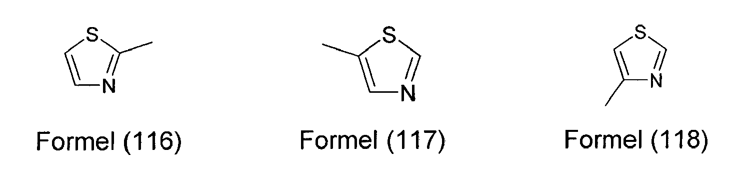

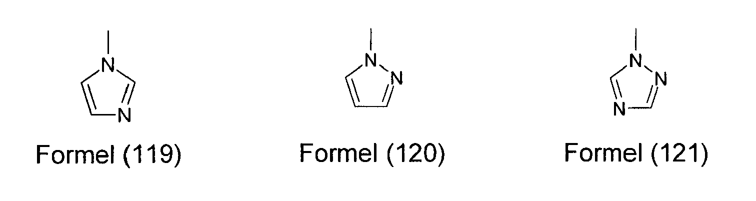

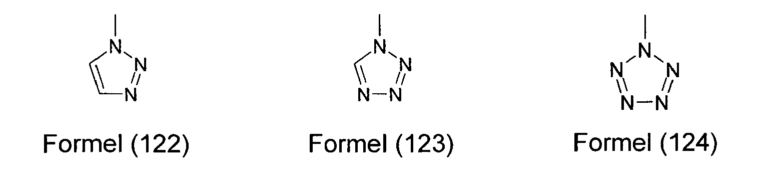

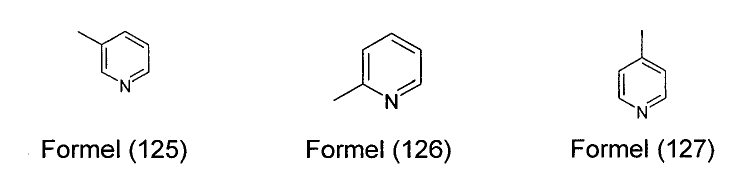

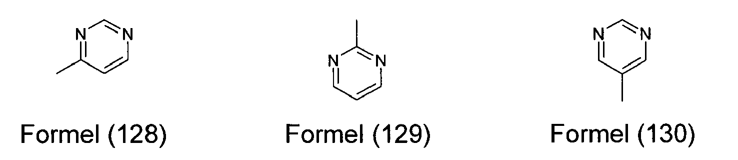

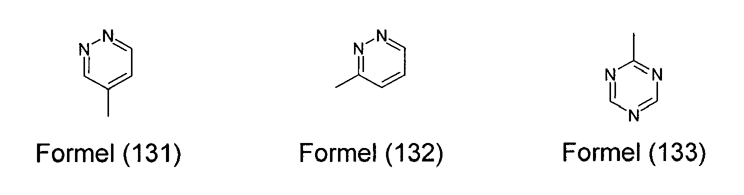

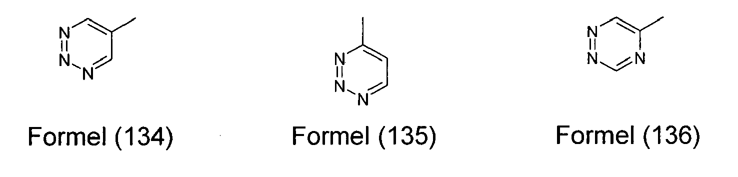

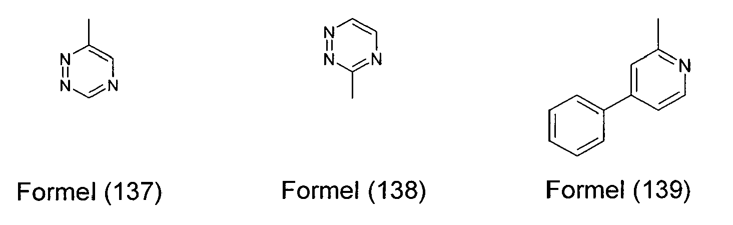

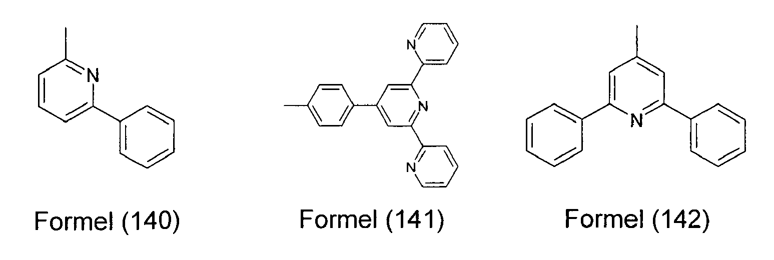

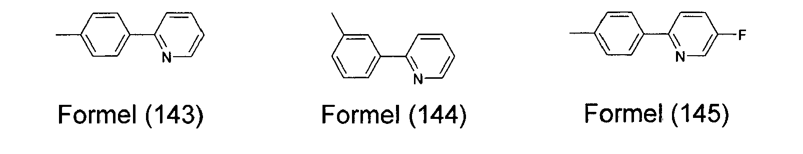

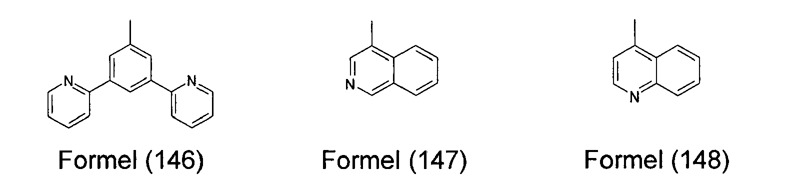

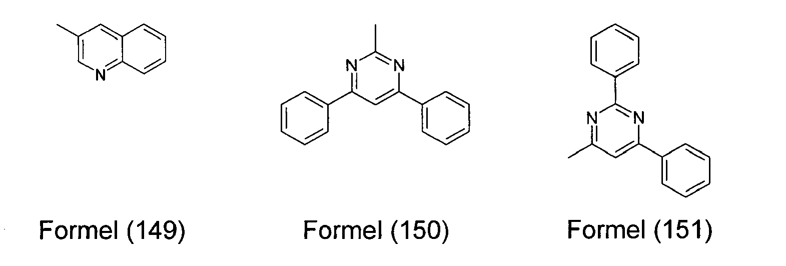

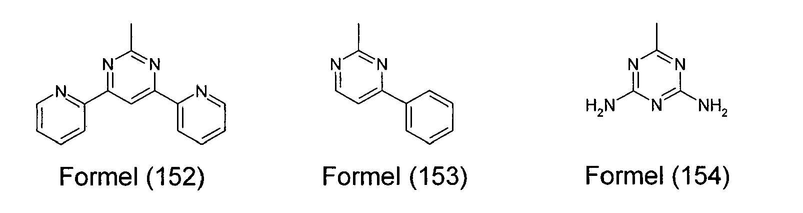

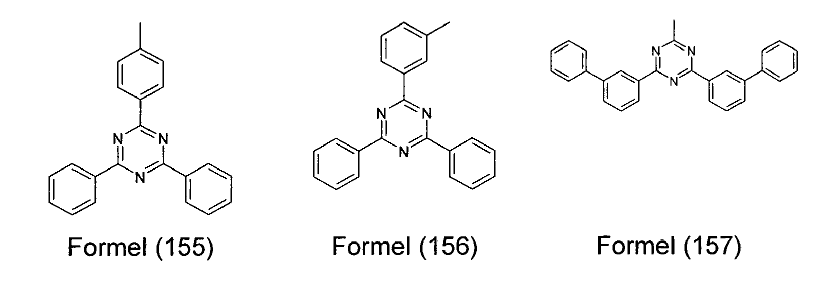

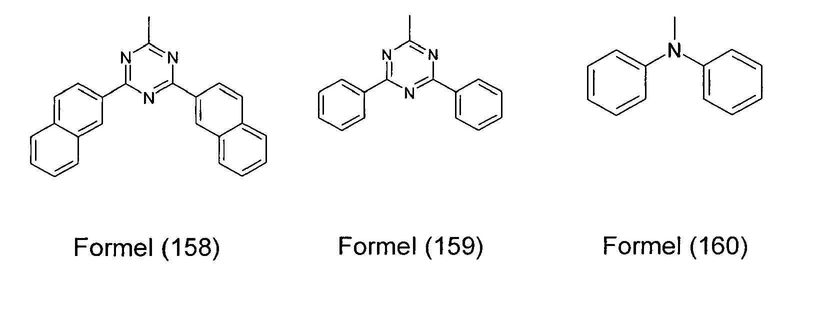

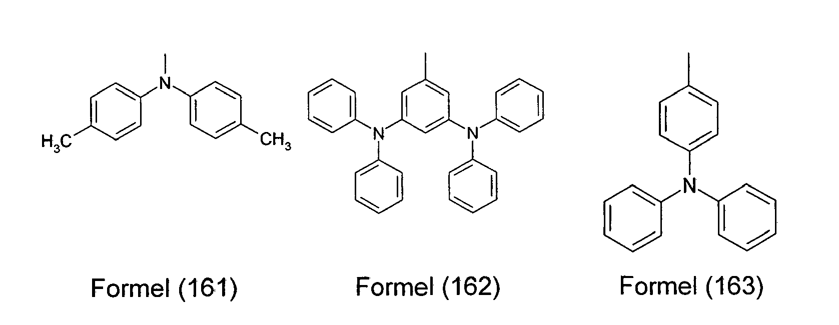

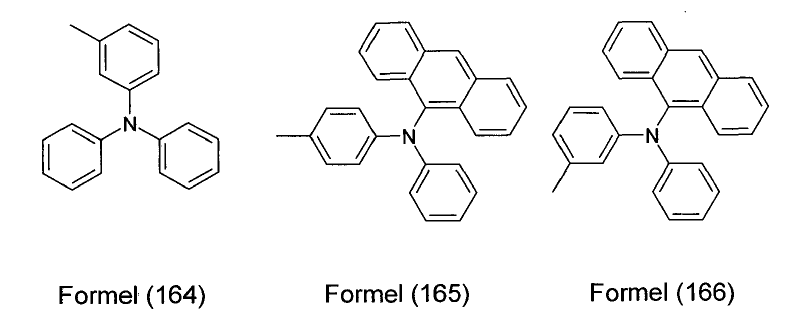

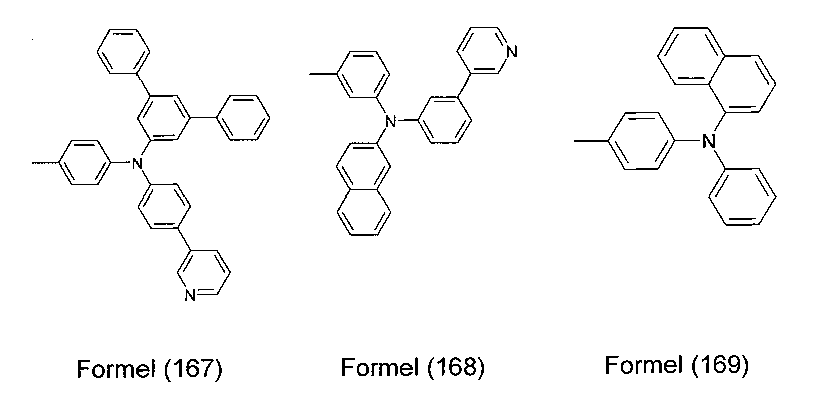

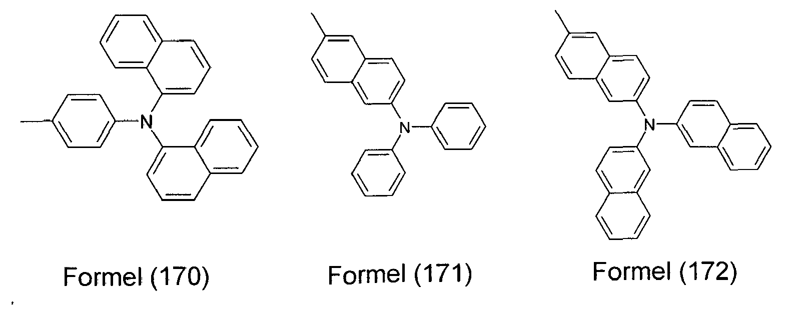

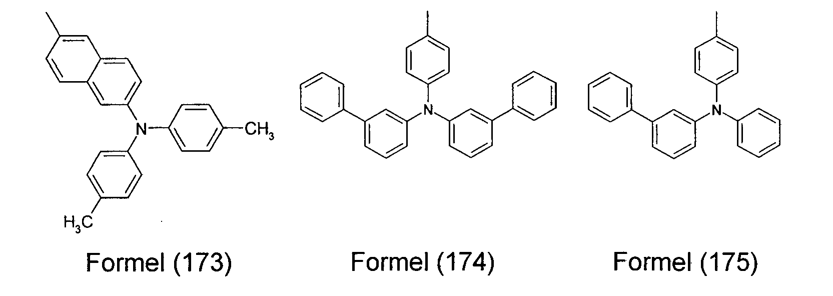

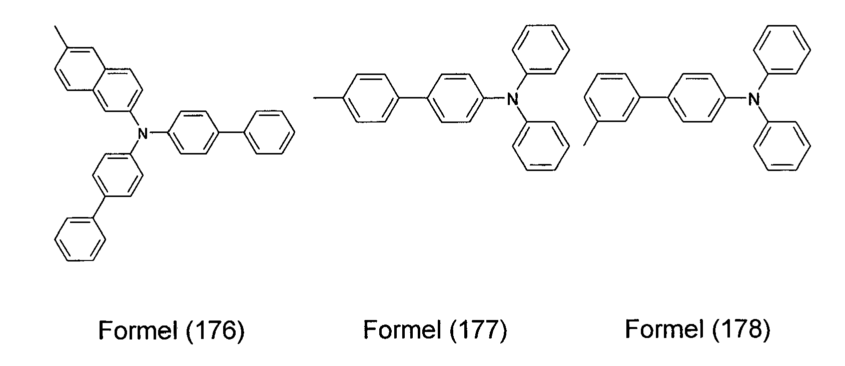

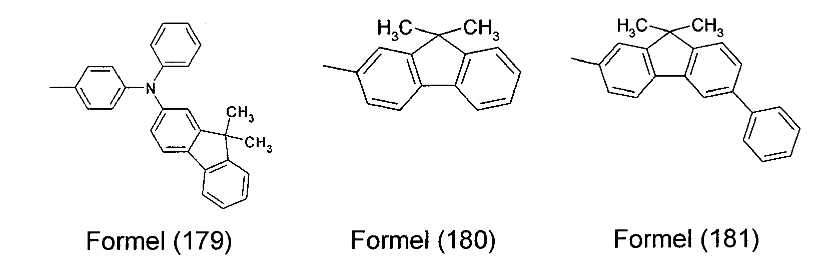

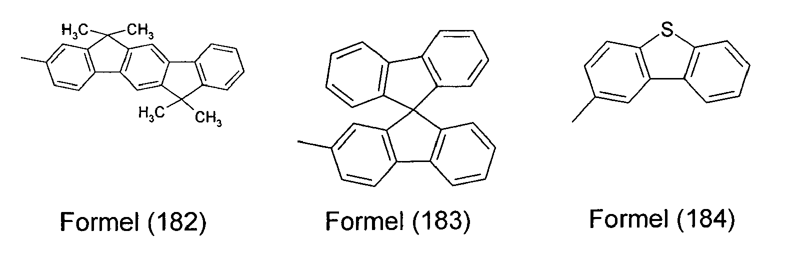

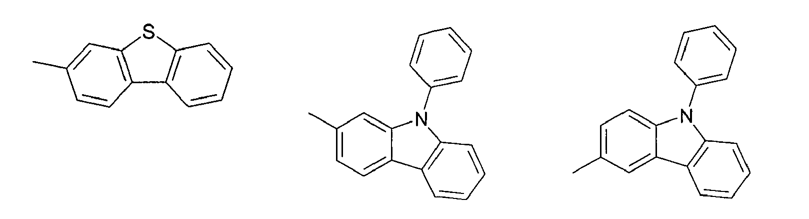

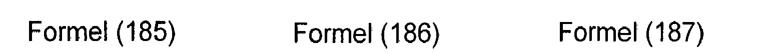

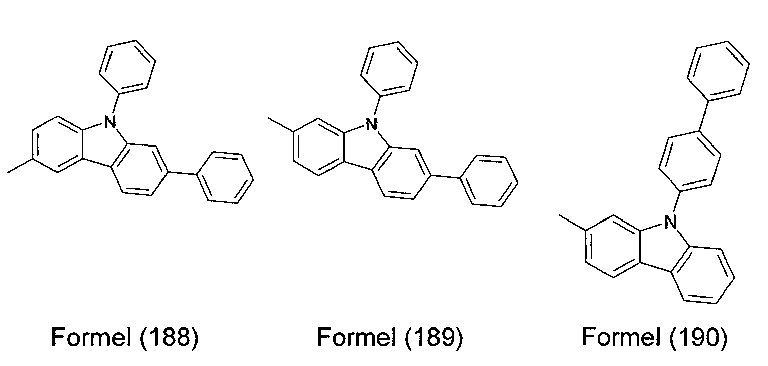

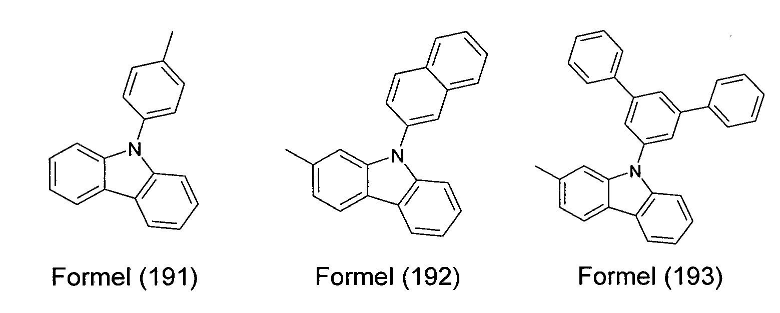

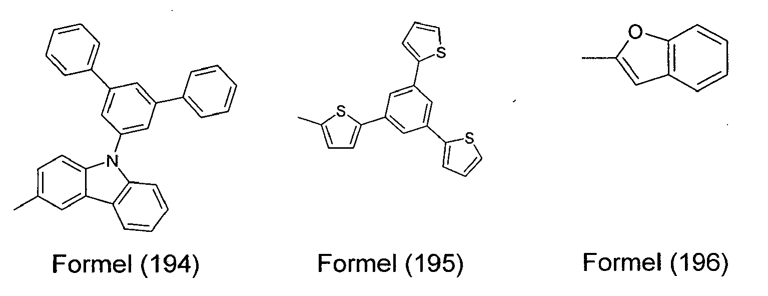

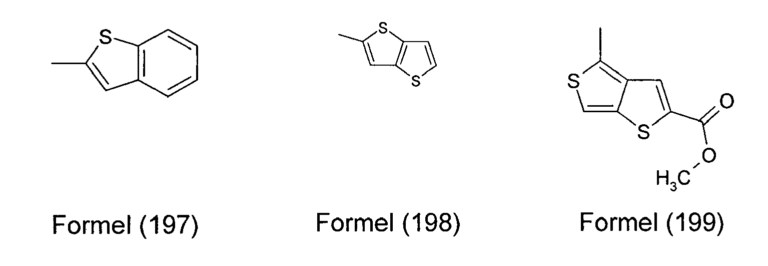

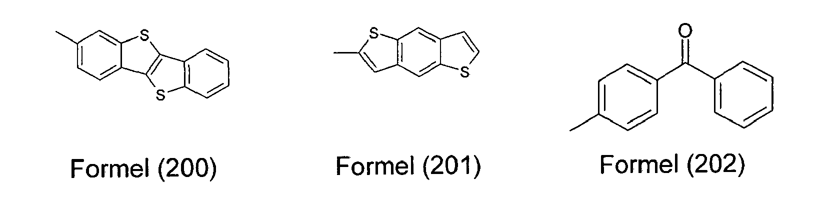

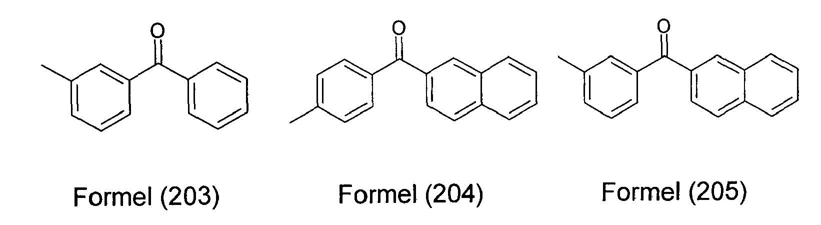

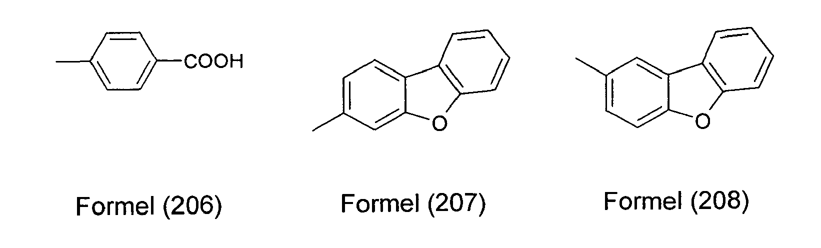

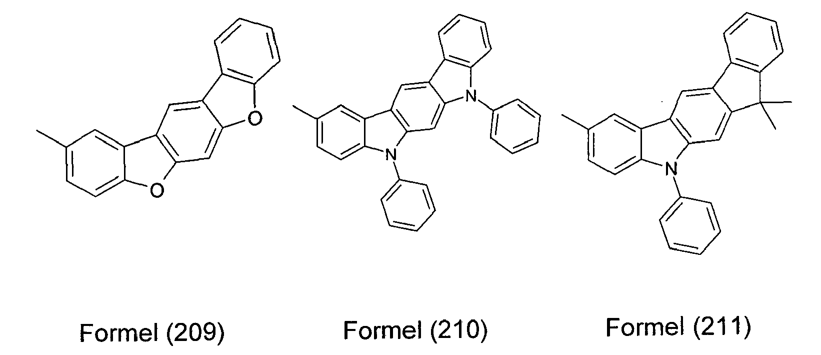

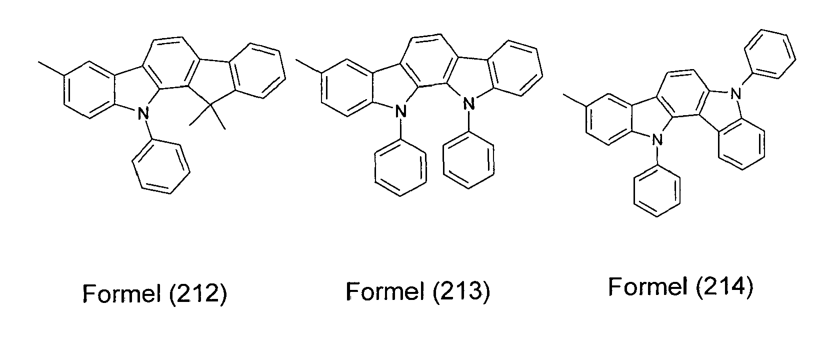

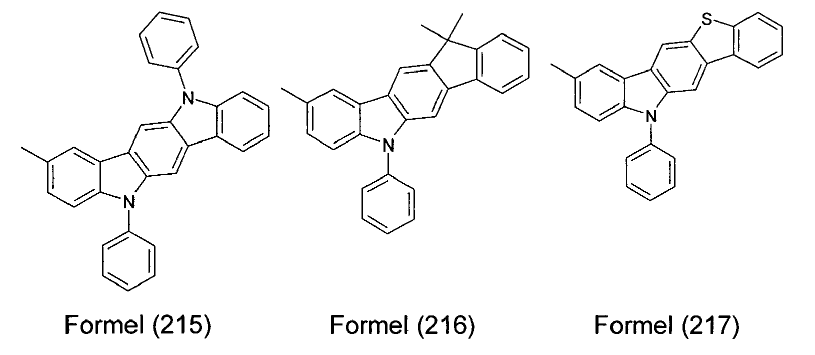

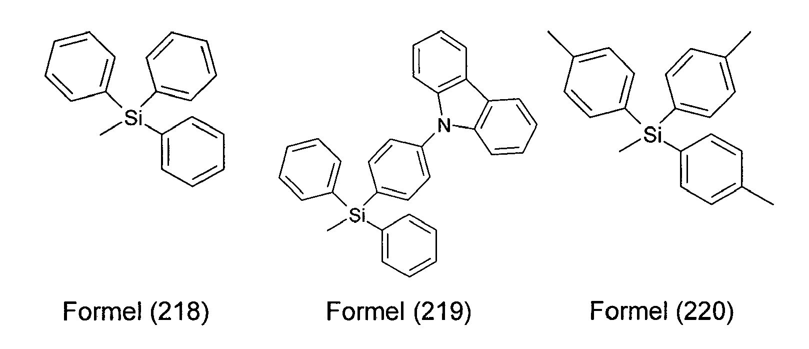

13. Verfahren zur Herstellung einer Verbindung nach einem oder mehreren der Ansprüche 1 bis 12, bei dem A' und B' miteinander zu C' umgesetzt werden, wobei die Symbole und Indizes wie vorstehend definiert sind

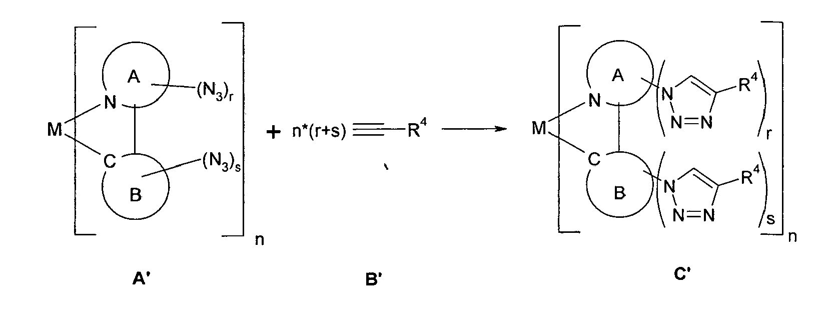

14. Zusammensetzung enthaltend mindestens eine Verbindung nach einem oder mehreren der Ansprüche 1 bis 12 und mindestens ein organisches funktionelles Material, das aus Lochtransportmaterialien (hole transport materials - HTM), Lochinjektionsmaterialien (hole injection materials - HIM), Elektronentransportmaterialien (electron transport materials - ETM), Elektroneninjektionsmaterialien (electron injection materials - EIM), lochblockierenden Materialien (hole blocking materials - HBM), exzitonblockierenden Materialien (exciton blocking materials - ExBM), Host- oder Matrixmaterialien, fluoreszierender Emitter, phosphoreszierender Emitter ausgewählt ist.

15. Zusammensetzung nach Anspruch 14, **dadurch gekennzeichnet, dass** es sich bei dem mindestens einen organischen funktionellen Material um ein Matrixmaterial handelt, das vorzugsweise aus Ketonen, Phosphinoxiden, Sulfoxiden, Sulfonen, Triarylaminen, Carbazolen, Indolocarbazolen, Indenocarbazolen, Azacarbazolen, bipolaren Matrixmaterialien, Silanen, Azaborolenen, Boronestern, Triazinen, Zinkkomplexen, Diaza- oder Tetraazasilolen oder Diazaphospholen oder deren Mischungen ausgewählt ist.

16. Formulierung enthaltend mindestens eine Verbindung nach einem oder mehreren von Anspruch 1 bis 12 oder eine Zusammensetzung nach Anspruch 14 oder 15 und mindestens ein Lösungsmittel.

17. Elektronische Vorrichtung enthaltend mindestens eine Verbindung nach einem oder mehreren der Ansprüche 1 bis 12 oder eine Zusammensetzung nach Anspruch 14 oder 15, vorzugsweise eine Vorrichtung, die aus organischen Elektrolumineszenzvorrichtungen (OLEDs - organischen Leuchtdioden (organic light emitting diodes), PLEDs - Polymer-Leuchtdioden (polymer light emitting diodes)), organischen integrierten Schaltungen (organic integrated cicuits - O-ICs), organischen Feldeffekttransistoren (organic field effect transistors - O-FETs), organischen Dünnschichttransistoren (organic thin film transistors - O-TFTs), organischen lichtemittierenden Transistoren (organic light emitting transistors - O-LETs), organischen Solarzellen (organic solar cells - O-SCs), organischen optischen Detektoren, organischen Photorezeptoren, organischen Feld-Quench-Devices (organic field quenching devices - O-FQDs), lichtemittierenden elektrochemischen Zellen (light emitting electrochemical cells - LECs, OLECs, LEECs) oder organischen Laserdioden (organic laser diodes - O-Laser), besonders bevorzugt einer OLED oder organischen lichtemittierenden Zelle (organic light emitting cell - OLEC) ausgewählt ist.

18. Vorrichtung nach Anspruch 17, **dadurch gekennzeichnet, dass** sie die Zusammensetzung nach Anspruch 15 in einer oder mehreren lichtemittierenden Schichten enthält.

19. Elektrolumineszenzvorrichtung, bei der es sich vorzugsweise um eine OLED oder OLEC handelt, enthaltend mindestens eine Verbindung nach einem oder mehreren der Ansprüche 1 bis 12 oder eine Zusammensetzung nach Anspruch 14 oder 15 zur Verwendung bei der Phototherapie in der Medizin.

20. Kosmetische Verwendung einer Elektrolumineszenzvorrichtung, bei der es sich vorzugsweise um eine OLED oder OLEC handelt, enthaltend mindestens eine Verbindung nach einem oder mehreren der Ansprüche 1 bis 12 oder eine Zusammensetzung nach Anspruch 14 oder 15.

## Revendications

1. Composé selon la Formule (1) :
M(L)ₙ(L')ₘ Formule (1)
dans laquelle le composé de la Formule générale (1) comprend une moitié M(L)ₙ de la Formule (2) : 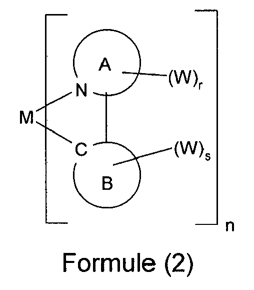 dans laquelle M est lié à un quelconque ligand bidenté souhaité L via un atome d'azote N et via un atome de carbone C, et
dans laquelle A et B peuvent être un quelconque cycle aromatique ou hétéroaromatique avec substitution ou non par un ou plusieurs R¹ ou un quelconque système de cycle aromatique ou hétéroaromatique polycyclique avec substitution ou non par un ou plusieurs R¹ ; et
dans laquelle les symboles et indices sont définis comme suit :
M est un métal choisi parmi le groupe constitué par iridium, rhodium, platine et palladium, de façon préférable iridium, platine et palladium, de façon particulièrement préférable iridium et platine et de façon très particulièrement préférable iridium ;
L' est, de manière identique ou différente pour chaque occurrence, un quelconque co-ligand souhaité ;
W est, de manière identique ou différente pour chaque occurrence, égal à la Formule (3) : 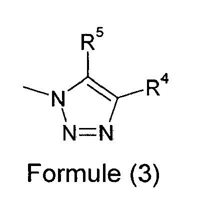
r est 0, 1, 2 ou 3 ;
s est 0, 1, 2 ou 3 ;
où la somme de r et s est d'au moins 1, de façon préférable de 1, 2 ou 3, de façon particulièrement préférable de 1 ou 2 et de façon très particulièrement préférable de 1 ;
n est 1, 2 ou 3 pour M égal à iridium ou rhodium et est 1 ou 2 pour M égal à platine ou palladium ;
m est 0, 1, 2, 3 ou 4 ;
R¹, R⁴ et R⁵ sont, de manière identique ou différente les uns par rapport aux autres pour chaque occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite comportant 1 à 40 atome(s) de C ou un groupe alkényle ou alkynyle en chaîne droite comportant 2 à 40 atomes de C ou un groupe alkyle, alkényle, alkynyle, alcoxy ou thioalcoxy ramifié ou cyclique comportant 3 à 40 atomes de C, dont chacun peut être substitué par un radical ou plusieurs radicaux R², où un ou plusieurs groupe(s) CH₂ non adjacents peut/peuvent être remplacé(s) par R²C=CR², C≡C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R², ou un groupe aryloxy ou hétéroaryloxy comportant 5 à 60 atomes de cycle aromatique, lequel peut être substitué par un radical ou plusieurs radicaux R², ou un groupe diarylamino, ou un groupe dihétéroarylamino ou un groupe arylhétéroarylamino comportant 10 à 40 atomes de cycle aromatique, lequel peut être substitué par un radical ou plusieurs radicaux R², ou une combinaison de deux de ces groupes ou plus ; deux radicaux R¹, R⁴ ou R⁵ ou plus peuvent ici également former un système de cycle aliphatique, aromatique et/ou benzo-fusionné monocyclique ou polycyclique l'un avec l'autre ou les uns avec les autres ;
R² est, de manière identique ou différente pour chaque occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, un groupe alkyle, alcoxy ou thioalcoxy en chaîne droite comportant 1 à 40 atome(s) de C ou un groupe alkényle ou alkynyle en chaîne droite comportant 2 à 40 atomes de C ou un groupe alkyle, alkényle, alkynyle, alcoxy ou thioalcoxy ramifié ou cyclique comportant 3 à 40 atomes de C, dont chacun peut être substitué par un radical ou plusieurs radicaux R³, où un ou plusieurs groupe(s) CH₂ non adjacents peut/peuvent être remplacé(s) par R³C=CR³, C=C, Si(R³)₂, Ge(R³)₂, Sn(R³)₂, C=O, C=S, C=Se, C=NR³, P(=O)(R³), SO, SO₂, NR³, O, S ou CONR³ et où un ou plusieurs atome(s) de H peut/ peuvent être remplacé(s) par D, F, Cl, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R³, ou un groupe aryloxy ou hétéroaryloxy comportant 5 à 60 atomes de cycle aromatique, lequel peut être substitué par un radical ou plusieurs radicaux R³, ou un groupe diarylamino, un groupe dihétéroarylamino ou un groupe arylhétéroarylamino comportant 10 à 40 atomes de cycle aromatique, lequel peut être substitué par un radical ou plusieurs radicaux R³, ou une combinaison de deux ou plus de ces groupes ; deux radicaux R² adjacents ou plus peuvent ici également former un système de cycle aliphatique ou aromatique monocyclique ou polycyclique l'un avec l'autre ou les uns avec les autres ;
R³ est, de manière identique ou différente pour chaque occurrence, H, D, F ou un radical hydrocarbone aliphatique, aromatique et/ou hétéroaromatique comportant 1 à 20 atome(s) de C, où, en outre, un ou plusieurs atome(s) de H peut/ peuvent être remplacé(s) par F ; deux substituants R³ ou plus peuvent ici également former un système de cycle aliphatique ou aromatique monocyclique ou polycyclique l'un avec l'autre ou les uns avec les autres ;
les indices n et m sont ici choisis de telle sorte que le nombre de coordination sur le métal corresponde à 6 pour M égal à iridium ou rhodium et corresponde à 4 pour M égal à platine ou palladium ; et dans lequel :
les ligands d'une pluralité de ligands L peuvent ici également être liés les uns aux autres ou L peut être lié à L' via un quelconque pont souhaité Z et ainsi former un système de ligand tridenté, tétradenté, pentadenté ou hexadenté.

2. Composé selon la revendication 1, **caractérisé en ce que** le composé selon la Formule (1) comprend une structure M(L)ₙ présentant la Formule (4) : 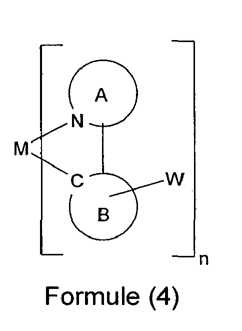 dans laquelle pour les symboles, les définitions de la revendication 1 s'appliquent.

3. Composé selon la revendication 1 ou 2, **caractérisé en ce que** le composé selon la Formule (1) comprend une structure M(L)ₙ présentant la Formule (5b) : 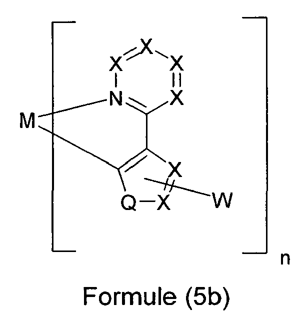 dans laquelle :
X est, de manière identique ou différente pour chaque occurrence, CR¹ ou N ;
Q est, de manière identique ou différente pour chaque occurrence, R¹C=CR¹, R¹C=N, O, S, Se ou NR¹, ou R¹ est défini comme ci avant.

4. Composé selon une ou plusieurs des revendications 1 à 3, **caractérisé en ce que** le composé selon la Formule (1) comprend une structure M(L)ₙ présentant la Formule (6) : 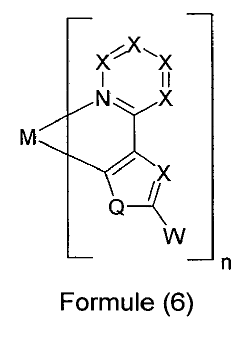 dans laquelle les symboles sont définis comme ci avant.

5. Composé selon une ou plusieurs des revendications 1 à 4, **caractérisé en ce que** le composé selon la Formule (1) comprend une structure M(L)ₙ présentant la Formule choisie parmi les Formules (9) à (19), de façon préférable parmi les Formules (9), (11), (12), (13), (14), de façon particulièrement préférable parmi les Formules (9), (11) et (12): 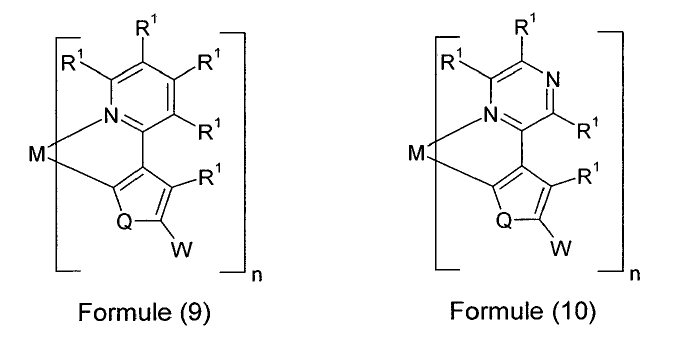 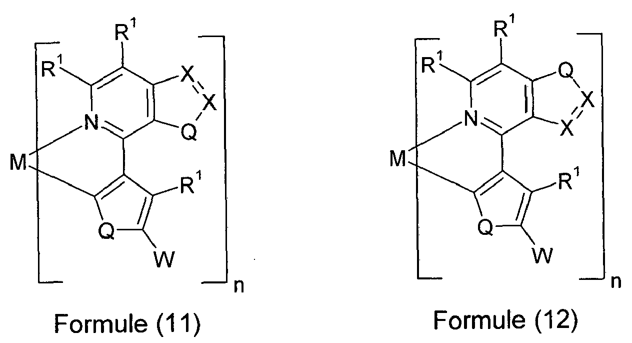 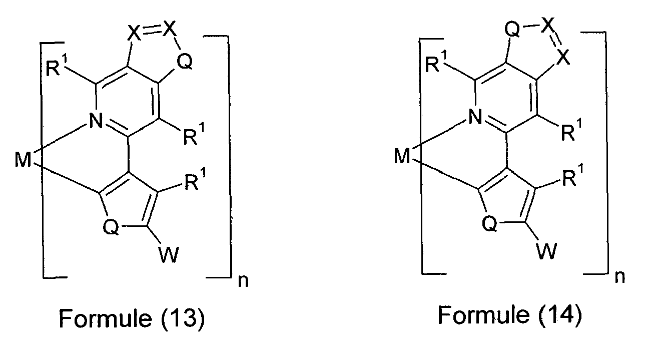 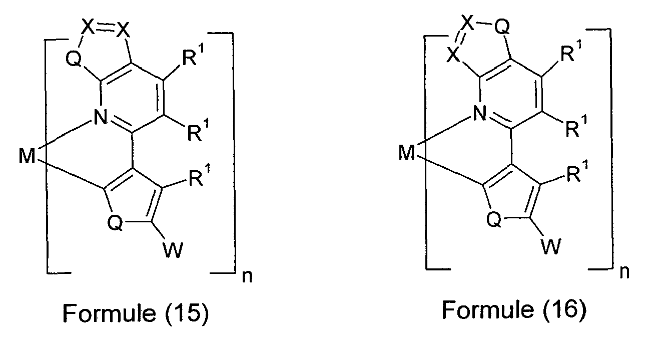 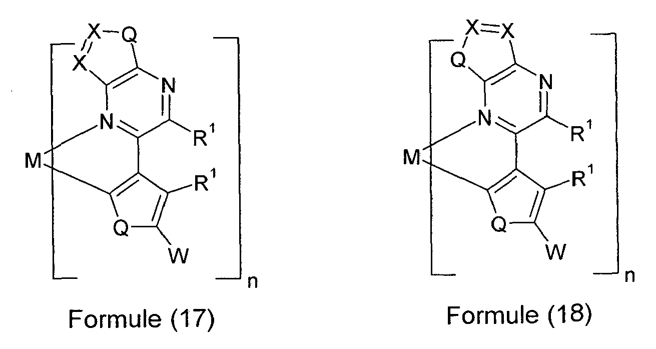 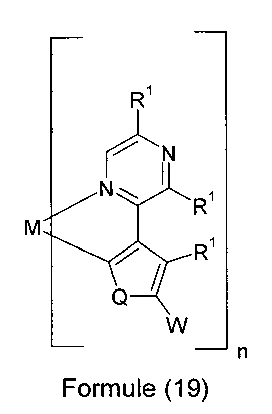 avec les définitions des symboles données ci avant.

6. Composé selon une ou plusieurs des revendications 1 à 5, **caractérisé en ce que** le composé selon la Formule (1) comprend une structure M(L)ₙ présentant la Formule choisie parmi les Formules qui suivent (27) à (31), de façon préférable les Formules (27), (29), (30) et (31), de façon particulièrement préférable les Formules (27), (29) et (30) :

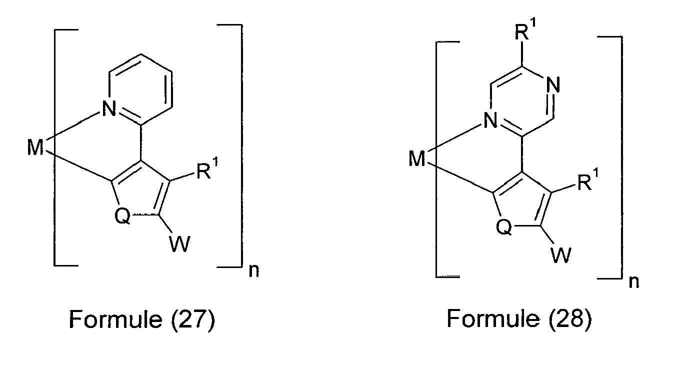

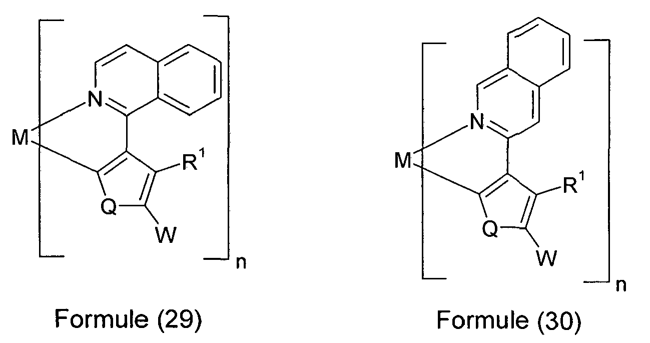

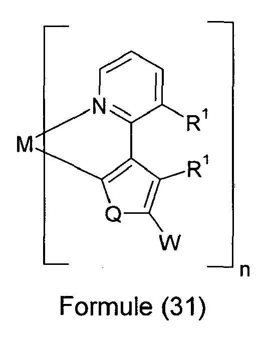

7. Composé selon une ou plusieurs des revendications 3 à 6, **caractérisé en ce que** Q est R¹C=CR¹, S ou NR¹ ; de façon particulièrement préférable R¹C=CR¹ ou S et de façon très particulièrement préférable R¹C=CR¹.

8. Composé selon une ou plusieurs des revendications 1 à 7, **caractérisé en ce que** m = 0.

9. Composé selon une ou plusieurs des revendications 1 à 8, **caractérisé en ce que** R⁵ est H.

10. Composé selon une ou plusieurs des revendications 1 à 9, **caractérisé en ce que** R⁴ est, de manière identique ou différente les uns par rapport aux autres pour chaque occurrence, H, un groupe alkyle ou alcoxy en chaîne droite comportant 1 à 40 atome(s) de C ou un groupe alkényle ou alkynyle en chaîne droite comportant 2 à 40 atomes de C ou un groupe alkyle, alkényle, alkynyle ou alcoxy ramifié ou cyclique comportant 3 à 40 atomes de C, -NH₂, -SR³, -OR³ ou un système de cycle aromatique ou hétéroaromatique comportant 5 à 60 atomes de cycle aromatique, lequel peut dans chaque cas être substitué par un radical ou plusieurs radicaux R².

11. Composé selon une ou plusieurs des revendications 1 à 10, **caractérisé en ce que** R⁴ est, de manière identique ou différente les uns par rapport aux autres pour chaque occurrence, H, un groupe alkyle ou alcoxy en chaîne droite comportant 1 à 40 atome(s) de C ou un groupe alkényle ou alkynyle en chaîne droite comportant 2 à 40 atomes de C ou un groupe alkyle, alkényle, alkynyle ou alcoxy ramifié ou cyclique comportant 3 à 40 atomes de C, -NH₂, -SR³, -OR³ ou un groupe choisi parmi les Formules qui suivent (32) à (46) : 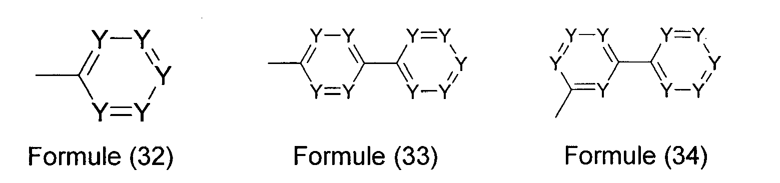 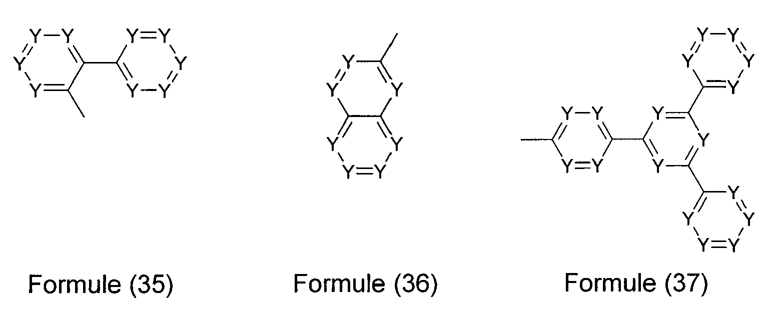 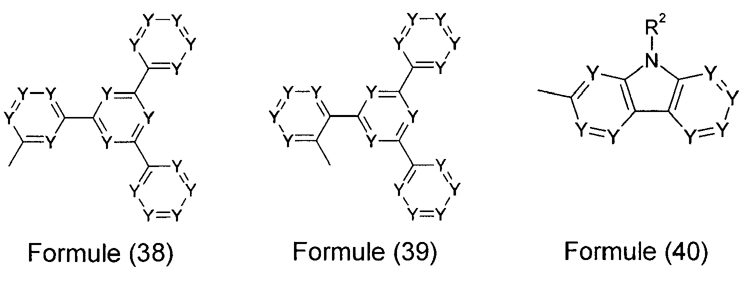 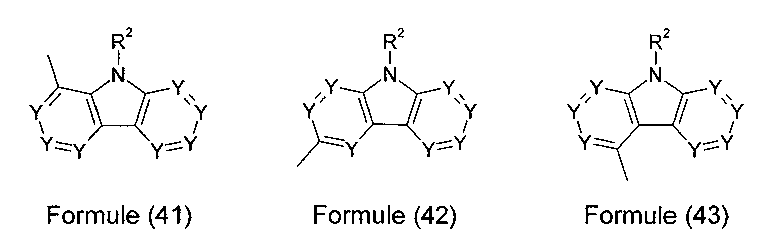 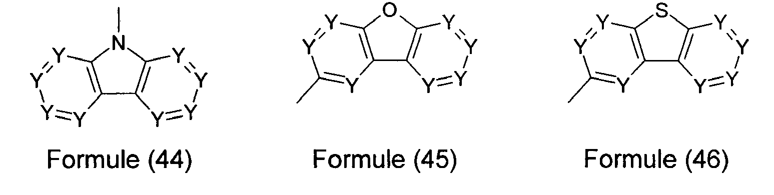 dans lesquelles Y est, de manière identique ou différente les uns par rapport aux autres pour chaque occurrence, CR², N, P, ou PR²₂, de façon préférable CR² ou N, où R² et R³ sont définis comme ci avant.

12. Composé selon une ou plusieurs des revendications 1 à 10, **caractérisé en ce que** R⁴ est, de manière identique ou différente les uns par rapport aux autres pour chaque occurrence, choisi parmi les Formules (47) à (220) où le reste présentant les Formules (47) à (220) peut être, de manière identique ou différente les uns par rapport aux autres pour chaque occurrence, en outre substitué par un ou plusieurs -CN ou R³ et où R³ est défini comme ci avant:

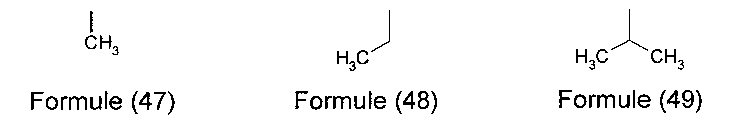

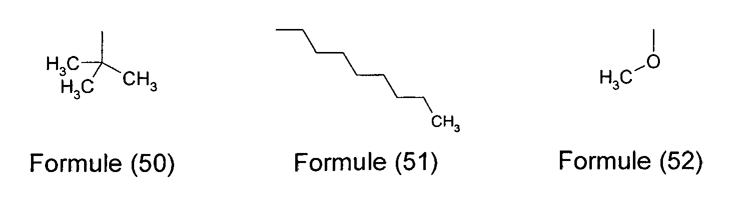

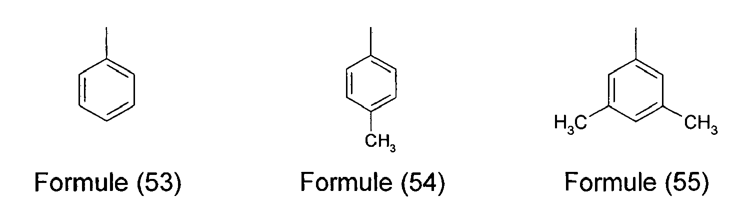

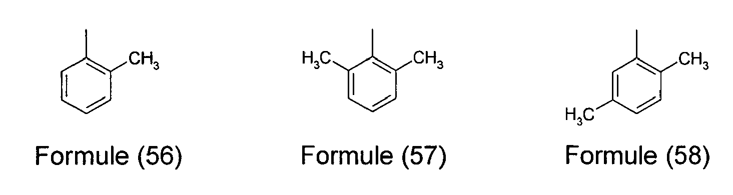

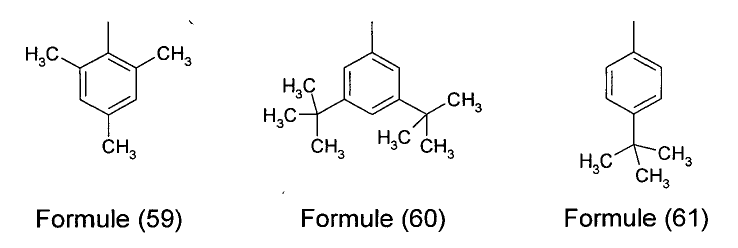

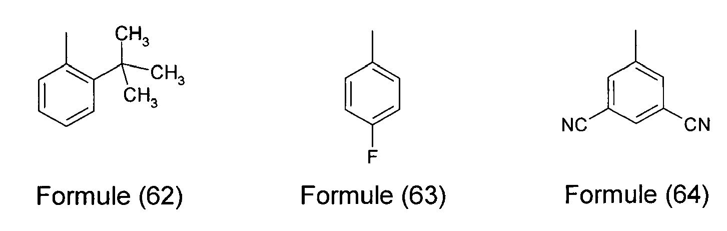

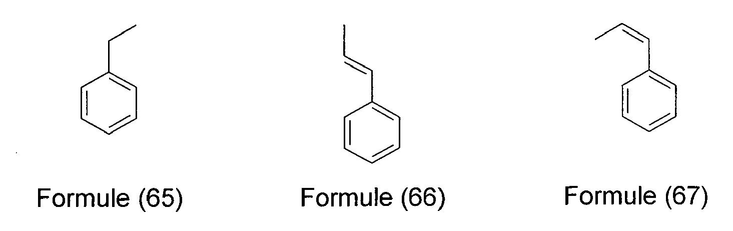

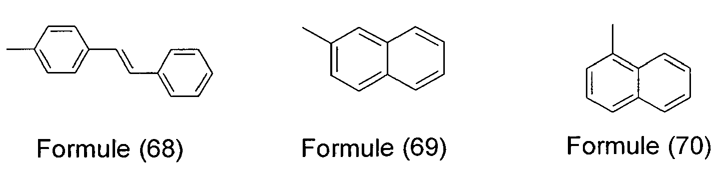

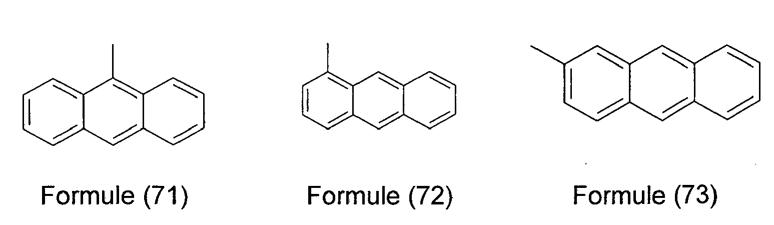

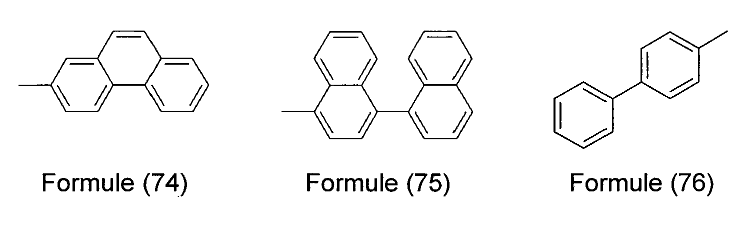

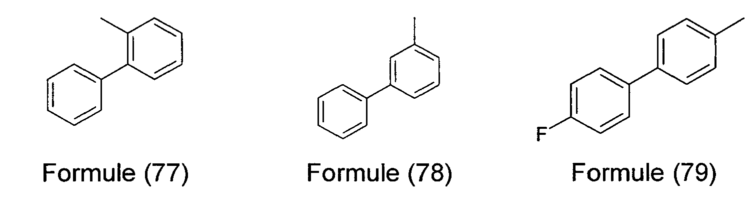

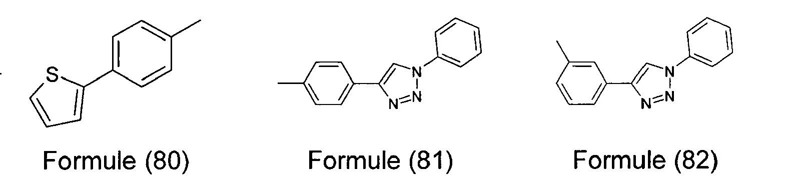

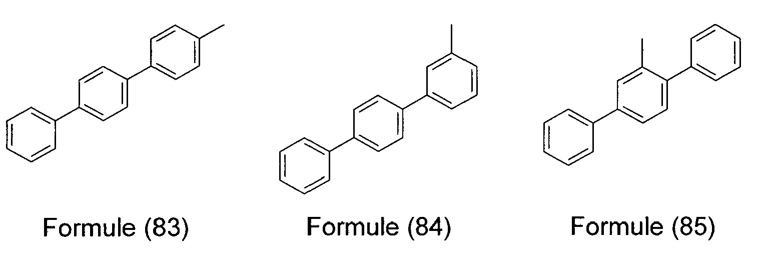

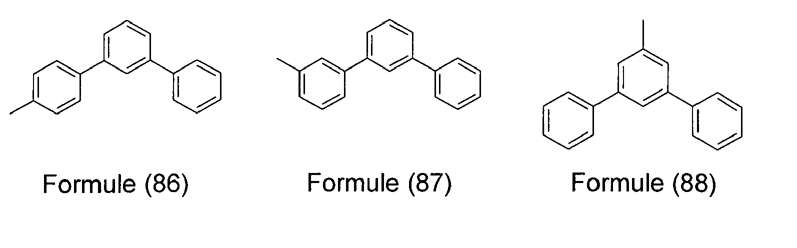

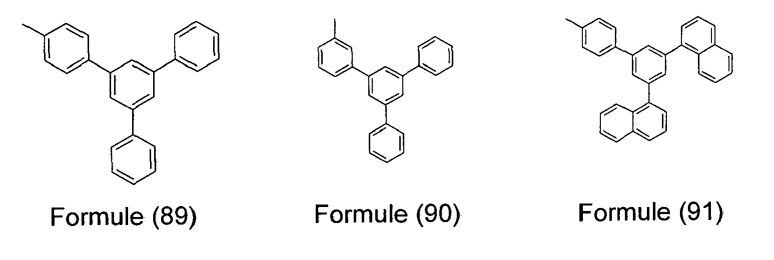

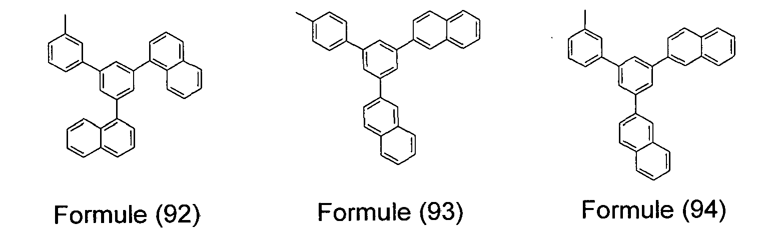

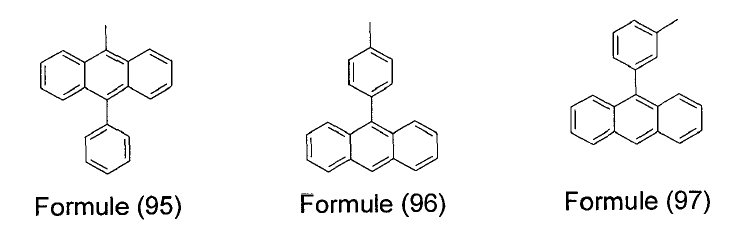

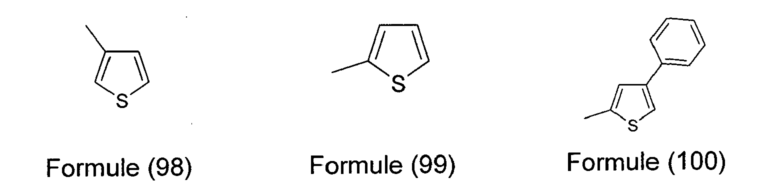

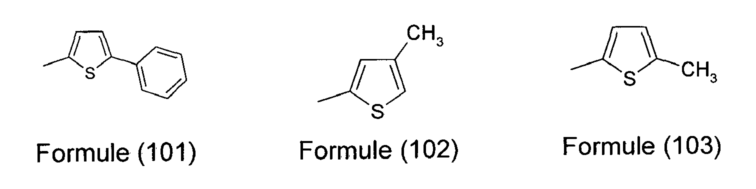

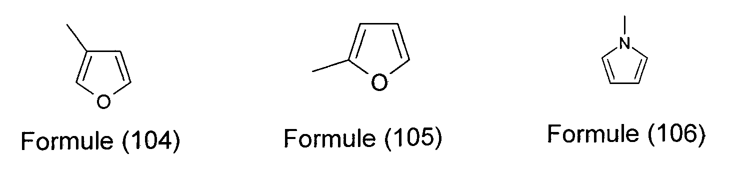

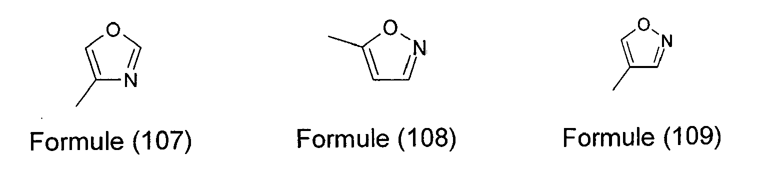

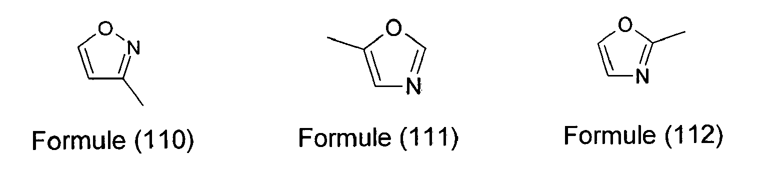

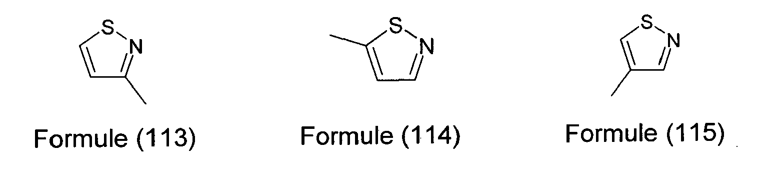

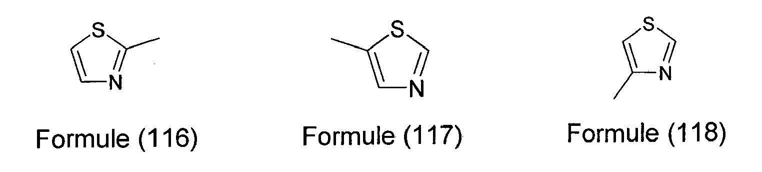

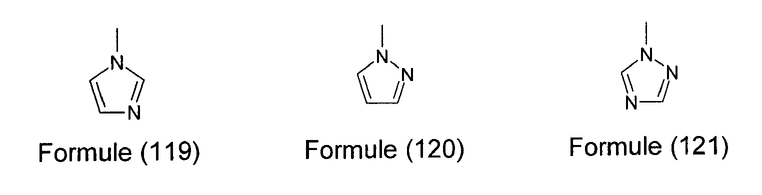

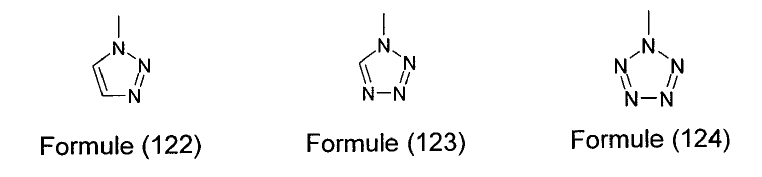

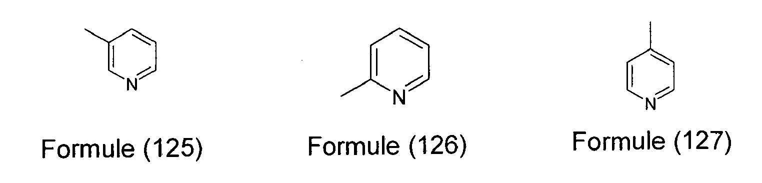

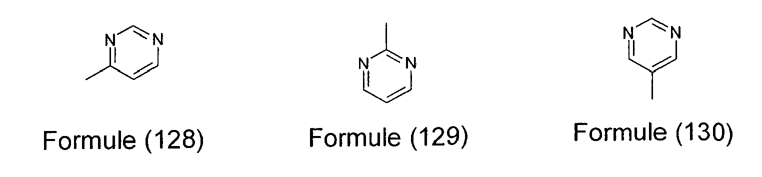

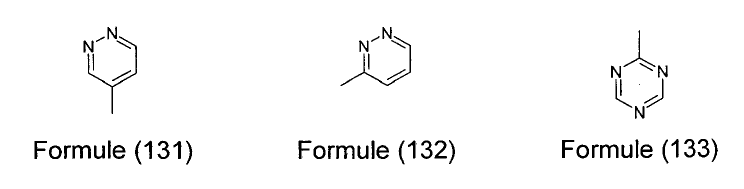

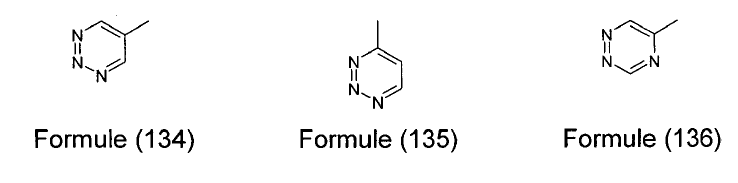

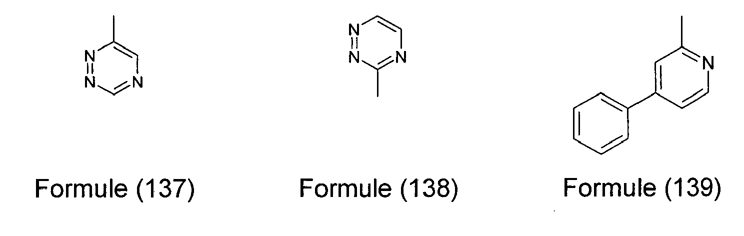

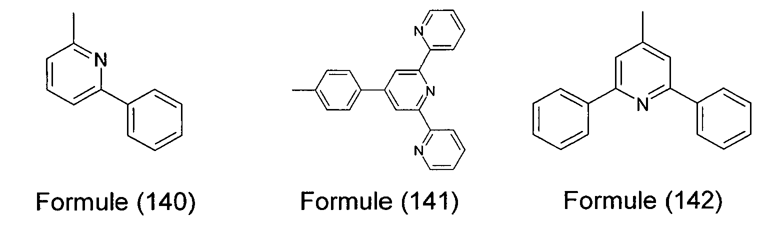

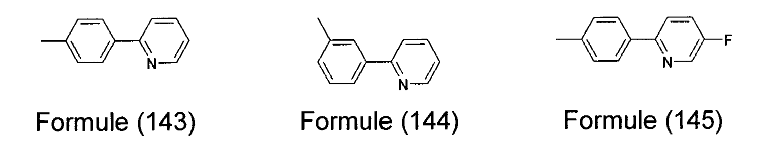

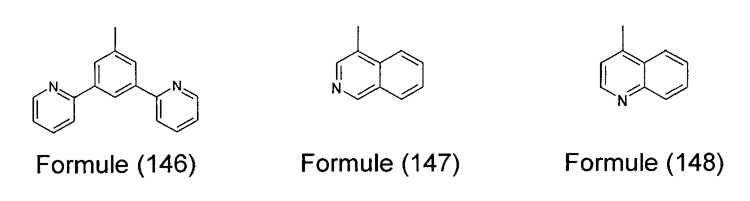

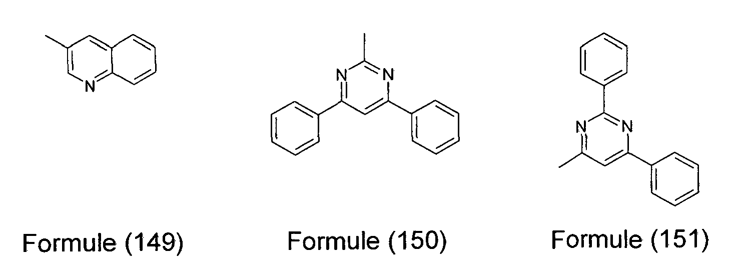

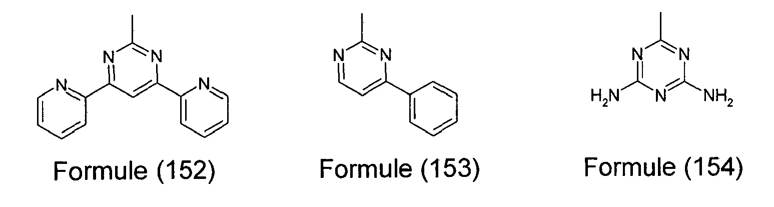

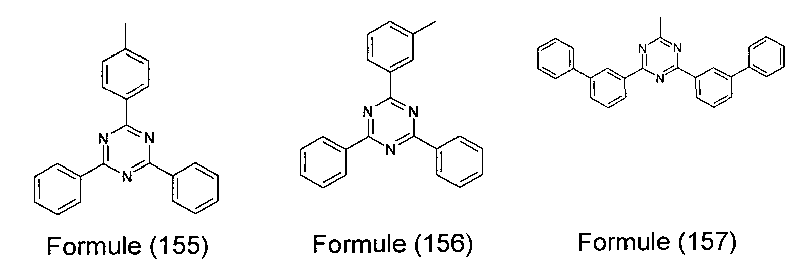

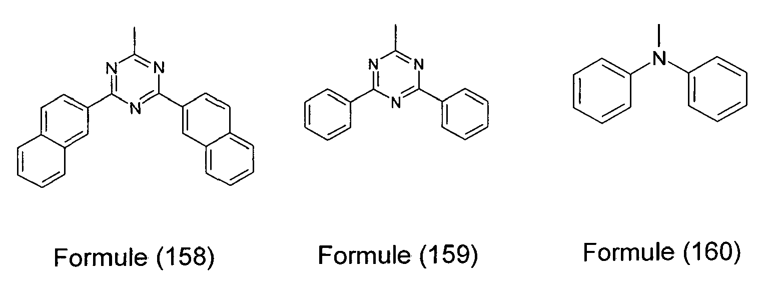

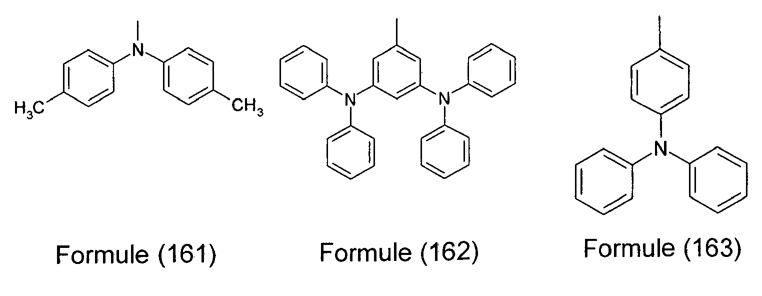

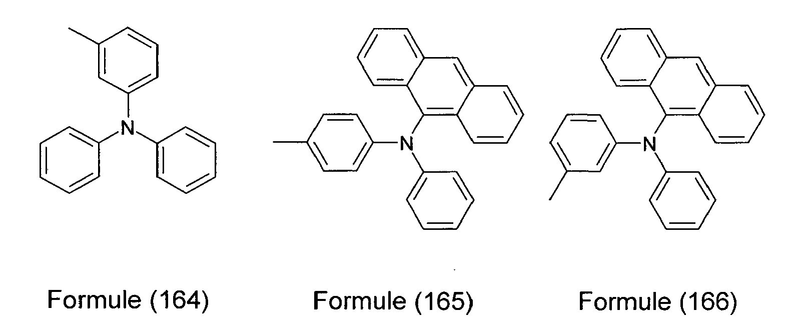

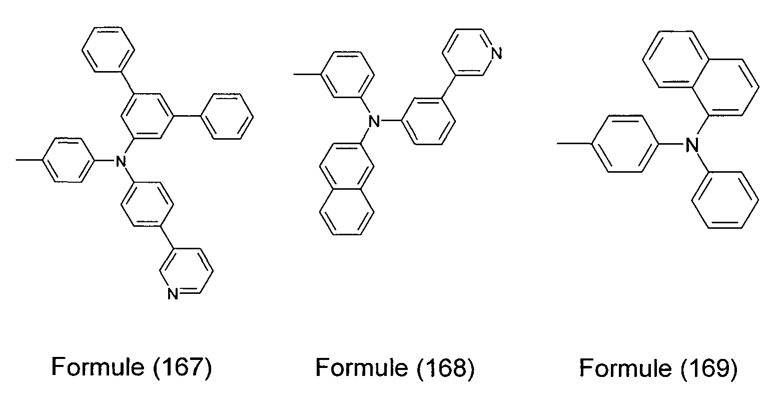

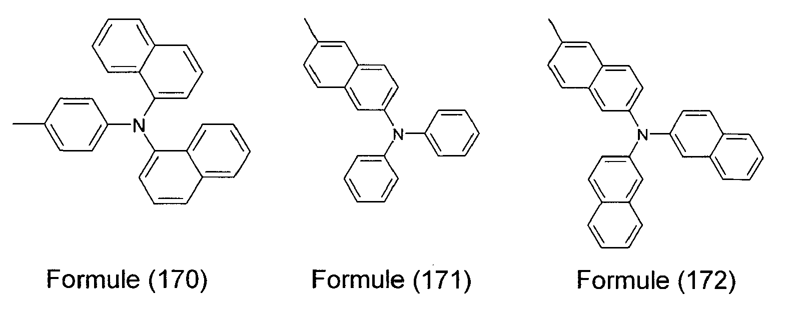

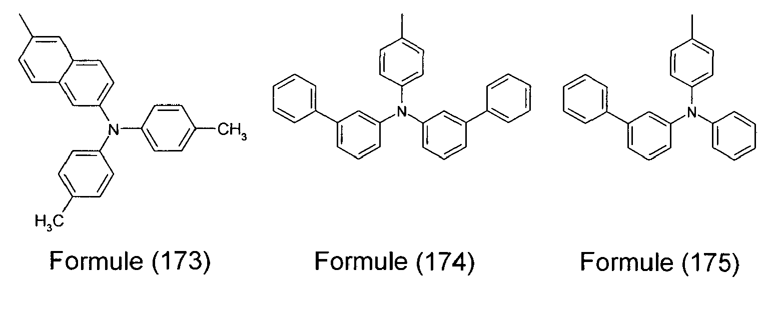

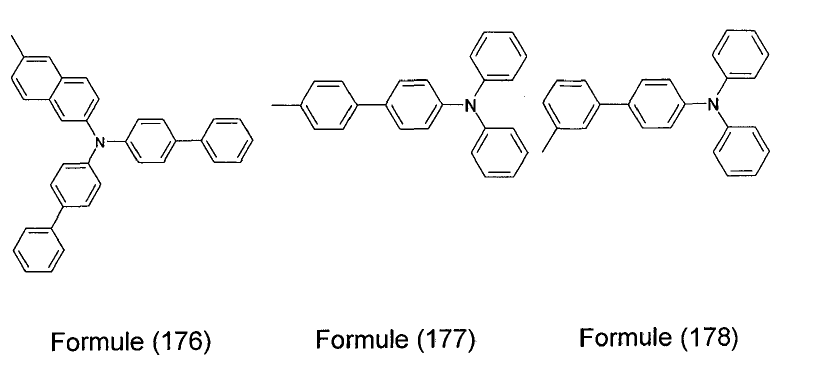

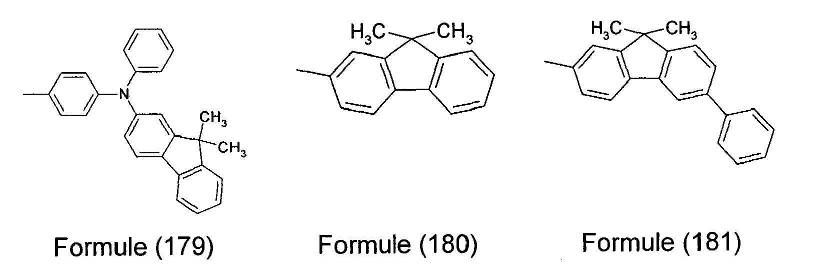

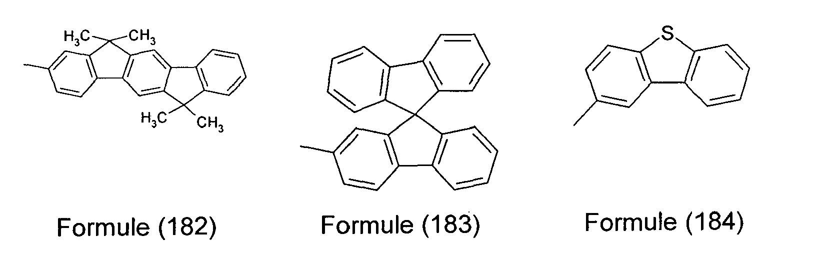

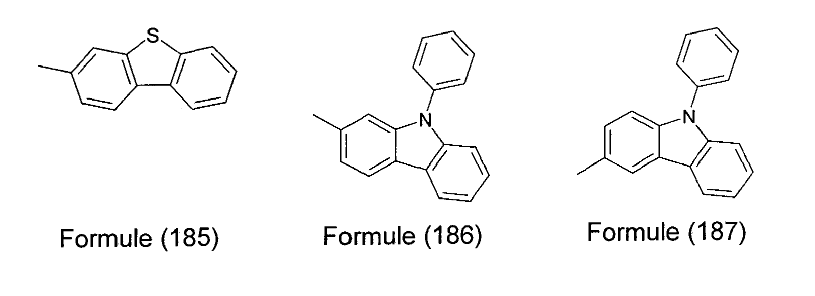

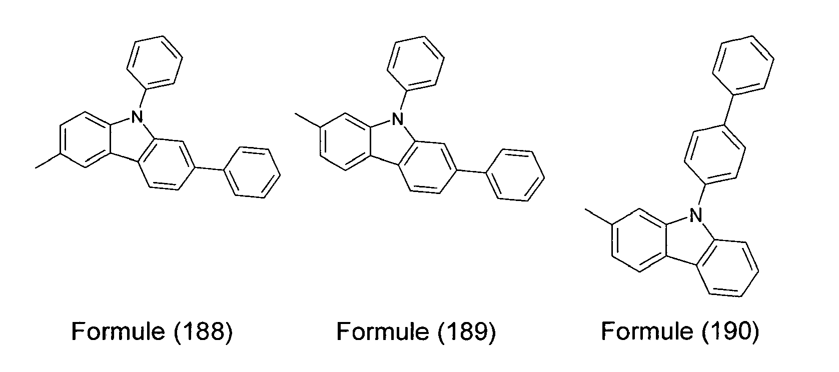

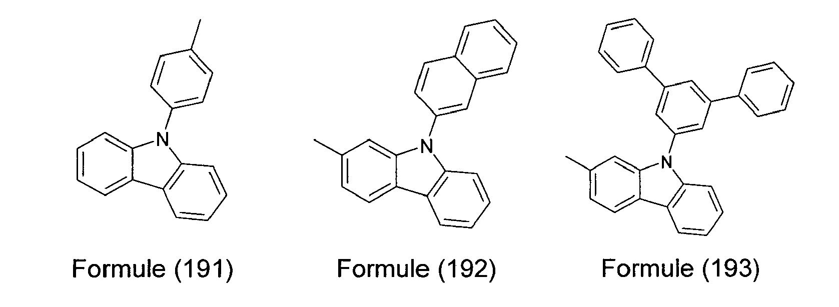

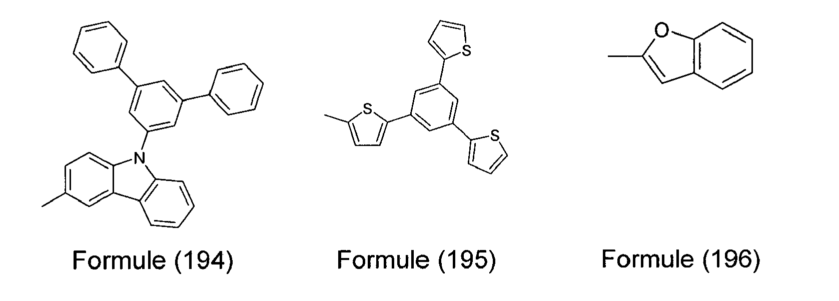

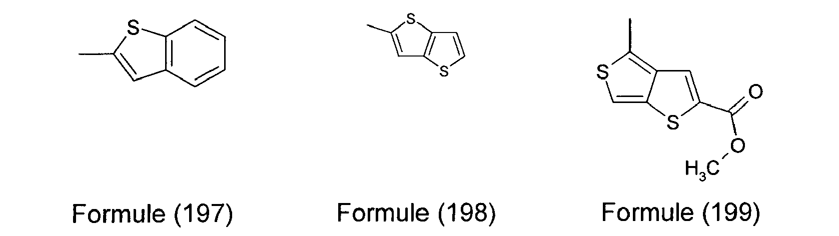

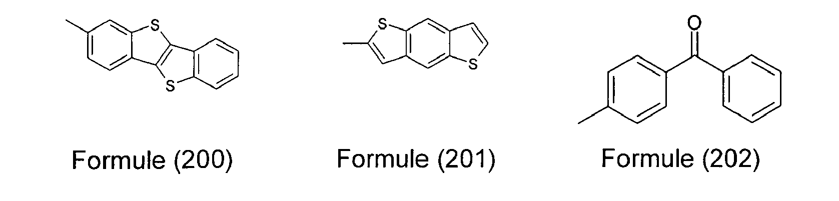

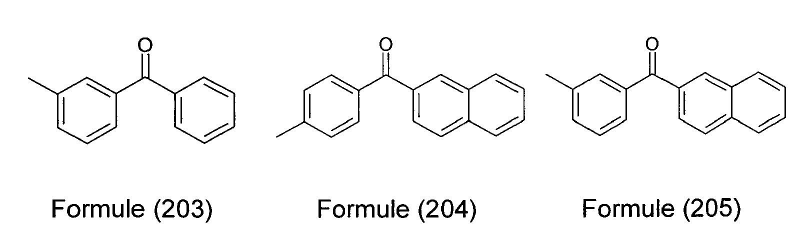

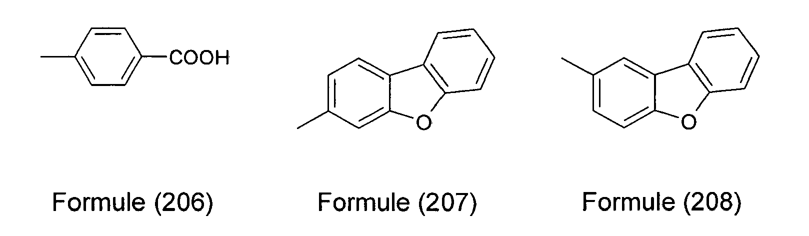

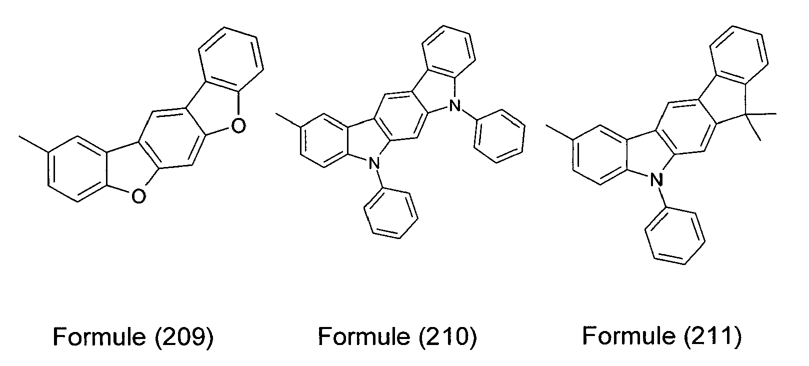

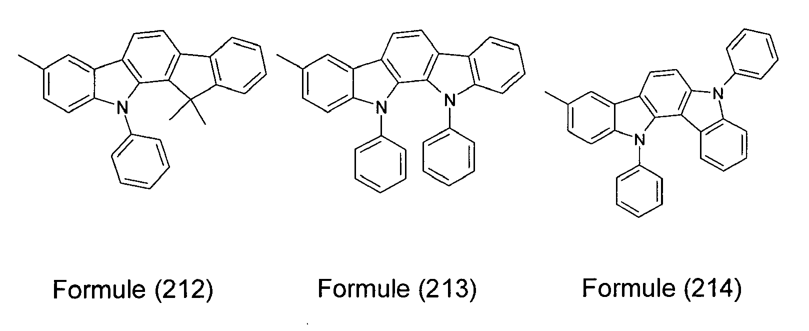

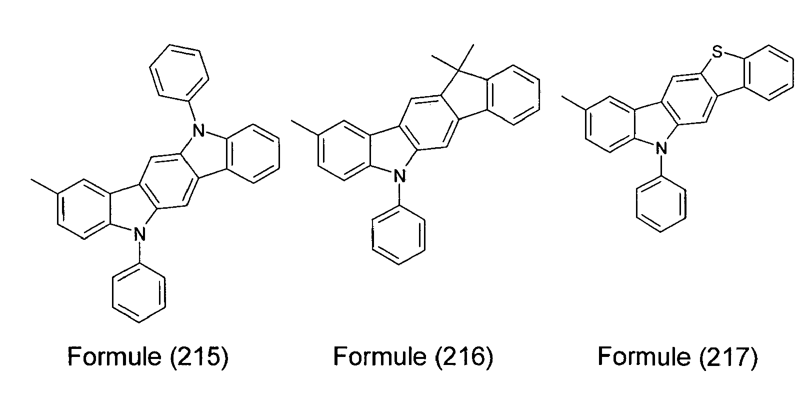

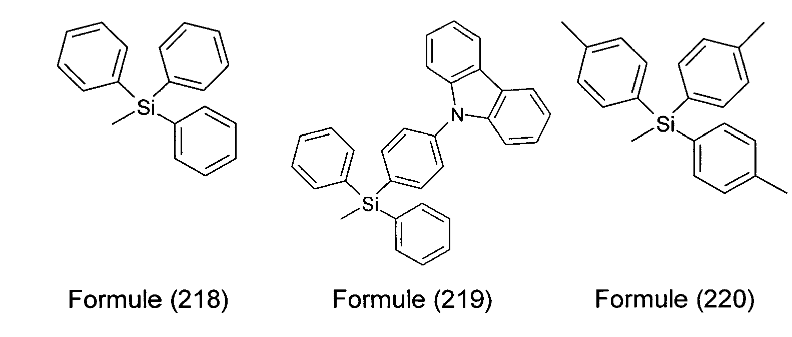

13. Procédé pour la préparation d'un composé selon une ou plusieurs des revendications 1 à 12 mettant en jeu la réaction de A' et B' afin d'obtenir C', dans lequel les symboles et indices sont défini comme ci avant :

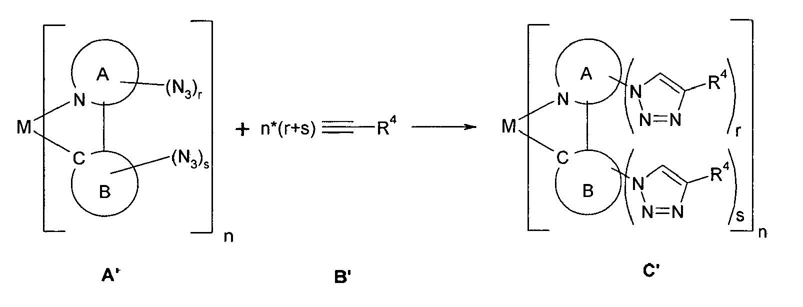

14. Composition comprenant au moins un composé selon une ou plusieurs des revendications 1 à 12 et au moins un matériau fonctionnel organique choisi parmi des matériaux de transport de trous (HTM), des matériaux d'injection de trous (HIM), des matériaux de transport d'électrons (ETM), des matériaux d'injection d'électrons (EIM), des matériaux de blocage de trous (HBM), des matériaux de blocage d'excitons (ExBM), des matériaux hôtes ou de matrice, un émetteur fluorescent, un émetteur phosphorescent.

15. Composition selon la revendication 14, **caractérisée en ce que** l'au moins un matériau fonctionnel organique est un matériau de matrice de façon préférable choisi parmi des cétones, des phosphinoxydes, des sulfoxydes, des sulfones, des triarylamines, des carbazoles, des indolocarbazoles, des indénocarbazoles, des azacarbazoles, des matériaux de matrice bipolaires, des silanes, des azaborolènes, des boronesters, des triazines, des complexes de zinc, des diaza- ou tétraazasiloles ou des diazaphospholes ou des mélanges afférents.

16. Formulation comprenant au moins un composé selon une ou plusieurs des revendications 1 à 12 ou une composition selon la revendication 14 ou 15 et au moins un solvant.

17. Dispositif électronique comprenant au moins un composé selon une ou plusieurs des revendications 1 à 12 ou une composition selon la revendication 14 ou 15, de façon préférable un dispositif choisi parmi des dispositifs électroluminescents organiques (des OLED - des diodes émettrices de lumière organiques, des PLED - des diodes émettrices de lumière en polymère), des circuits intégrés organiques (O-IC), des transistors à effet de champ organiques (O-FET), des transistors à film mince organiques (O-TFT), des transistors à émission de lumière organiques (O-LET), des cellules solaires organiques (O-SC), des détecteurs optiques organiques, des photorécepteurs organiques, des dispositifs à extinction de champ organiques (O-FQD), des cellules électrochimiques à émission de lumière (LEC, OLEC, LEEC) ou des diodes laser organiques (O-Laser), de façon particulièrement préférable un OLED ou une cellule à émission de lumière organique (OLEC).

18. Dispositif selon la revendication 17, **caractérisé en ce qu'**il comprend la composition selon la revendication 15 dans une ou plusieurs couche(s) d'émission de lumière.

19. Dispositif électroluminescent, lequel est de façon préférable un OLED ou une OLEC, comprenant au moins un composé selon une ou plusieurs des revendications 1 à 12 ou une composition selon la revendication 14 ou 15 pour une utilisation pour la photothérapie en médecine.

20. Utilisation cosmétique d'un dispositif électroluminescent, lequel est de façon préférable un OLED ou une OLEC, comprenant au moins un composé selon une ou plusieurs des revendications 1 à 12 ou une composition selon la revendication 14 ou 15.
